# FASCICULE DE BREVET EUROPEEN

(11) **EP 2 419 410 B1**
(45) Date de publication et mention de la délivrance du brevet: **06.03.2013**
(21) Numéro de dépôt: 10717193.6
(22) Date de dépôt: 16.04.2010
(51) Int. Cl.: C07D 233/72, C07D 233/76, A61K 31/4166, A61P 35/00

(54) **DERIVES D'IMIDAZOLIDINE-2,4-DIONE ET LEUR UTILISATION COMME MEDICAMENT CONTRE LE CANCER**
IMIDAZOLIDIN-2,4-DIONDERIVATE UND DEREN VERWENDUNG ALS KREBSMITTEL
IMIDAZOLIDINE-2,4-DIONE DERIVATIVES, AND USE THEREOF AS A CANCER DRUG

(30) Priorité: 17.04.2009 FR 0901865
(43) Date de publication de la demande: 22.02.2012
(73) Titulaire: IPSEN PHARMA S.A.S., 92100 Boulogne-Billancourt (FR)
(72) Inventeur: PREVOST, Grégoire, F-92160 Antony (FR); AUVIN, Serge, F-91120 Palaiseau (FR); LANCO, Christophe, F-91410 Dourdan (FR); LIBERATORE, Anne-Marie, F-78610 Auffargis (FR); LAVERGNE, Olivier, F-91120 Palaiseau (FR)
(74) Mandataire: Audonnet, Nathalie
(86) Numéro de dépôt international: PCT/FR2010/000315
(87) Numéro de publication internationale: WO 2010/119193

(56) Documents cités:
- US-A1- 2007 249 697

## Description

### DOMAINE DE L'INVENTION

La présente demande a pour objet de nouveaux dérivés d'imidazolidine-2,4-dione. Ces produits ont une activité anti-proliférative. Ils sont particulièrement intéressants pour traiter les états pathologiques et les maladies liés à une prolifération cellulaire anormale tels que les cancers. L'invention concerne également des compositions pharmaceutiques contenant lesdits produits et leur utilisation pour la préparation d'un médicament.

### ETAT DE LA TECHNIQUE

De nos jours, le cancer constitue encore une des causes majeures de décès et ce malgré de nombreuses molécules sur le marché des médicaments.

II est donc nécessaire d'identifier de nouvelles molécules plus puissantes permettant une meilleure réponse antitumorale et ce par une bonne activité inhibitrice de la prolifération de colonies cellulaires tumorales.

De telles molécules sont donc particulièrement intéressantes pour traiter les états pathologiques liés à une prolifération cellulaire anormale. Elles peuvent ainsi être utilisées pour le traitement de tumeurs ou de cancers, par exemple ceux de l'oesophage, de l'estomac, des intestins, du rectum, de la cavité orale, du pharynx, du larynx, du poumon, du colon, du sein, du cervix uteri, du corpus endométrium, des ovaires, de la prostate, des testicules, de la vessie, des reins, du foie, du pancréas, des os, des tissus conjonctifs, de la peau tels que les mélanomes, des yeux, du cerveau et du système nerveux central, ainsi que le cancer de la thyroïde, la leucémie, la maladie de Hodgkin, les lymphomes autres que ceux de Hodgkin, les mélanomes multiples et autres cancers.

Il est d'intérêt particulier de trouver des thérapies aux cancers hormonodépendants, aux tumeurs exprimant des récepteurs aux androgènes, aux cancers du sein et de la prostate.

US2007/0249697 décrit des composés ayant la structure phényl-imidazolidine-2,4-dione et utilisés comme antagonistes du récepteur androgène pour traiter le cancer.

L'utilisation des anti-androgènes dans le cancer de la prostate est basée sur leur propriété à entrer en compétition avec les agonistes naturels du récepteur des androgènes. Cependant l'efficacité de ces anti-androgènes semble limitée dans le temps, les patients finissant par échapper aux traitements. Plusieurs hypothèses à cet échappement ont été développées montrant une activité agoniste en lieu et place d'une activité antagoniste de ces molécules (Veldscholte J, Berrevoets CA, Brinkmann AO, Grootegoed JA, Mulder E.Biochemistry 1992 Mar 3;31(8):2393-9). Par exemple, le nilutamide est capable de stimuler la croissance de cellules humaines de cancer de prostate en culture. En complément de ces indications expérimentales, des données cliniques supportent aussi ce rôle délétère des anti-androgènes (Akimoto S. ;Antiandrogen withdrawal syndrome Nippon Rinsho. 1998 Aug;56(8):2135-9. Paul R, Breul J. Antiandrogen withdrawal syndrome associated with prostate cancer therapies: incidence and clinical significance Drug Saf. 2000 Nov;23(5):381-90).

De plus, l'obtention de composés anti androgènes purs ne semblent pas aisé, ainsi les travaux de K. Tachibana et collaborateurs dans Bioorg. Med. Chem. 15 (2007) 174-185 ainsi que ceux de Cantin et collaborateurs dans The Journal of Biological Chemistry, 282, 42, (2007) 30910-30919, montrent que des composés de structures chimiques très voisines, possédant un pharmacophore ligand des récepteurs aux androgènes peuvent conduire à des activités biologiques agonistes comme antagonistes.

Ici la demanderesse a identifié des composés montrant une activité anti-proliférative de la tumeur prostatique qui de manière surprenante ne montrent pas d'activité agoniste à des concentrations où le nilutamide se comporte comme un agoniste. Cette différence de comportement sur la prolifération des nouveaux composés par rapport au nilutamide est supportée par leur capacité à induire la disparition des récepteurs androgènes sous leur forme protéique. Le nilutamide n'a lui aucun effet sur ce taux de récepteur.

Les propriétés de ces nouvelles molécules doivent permettre une meilleure prise en charge du cancer de la prostate évitant l'échappement aux anti-androgènes actuels.

De plus, les composés de la présente invention peuvent également être utilisés pour traiter les pathologies liées à la présence de récepteurs aux androgènes comme par exemple l'hyperplasie bénigne de la prostate, la prostamégalie, l'acné, l'alopécie androgénique, l'hirsutisme etc...

### RESUME DE L'INVENTION

L'invention a donc pour objet des composés de formule générale (I) 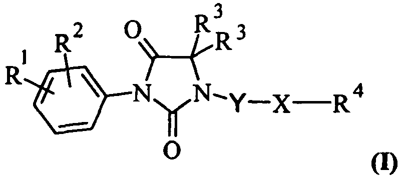 sous forme racémique, d'énantiomère ou toutes combinaisons de ces formes et dans laquelle,
R¹ et R² représentent indépendamment un atome d'halogène, ou bien un radical alkyle, haloalkyle, alkoxy, cyano, nitro, amino, alkylamino, dialkylamino, -NR⁸-CO-R⁵, -NR⁸-SO₂-R⁵, -NR⁸-CO-(CH₂)ₙ-NR⁶R⁷, -NR⁸-SO₂-(CH₂)ₙ-NR⁶R⁷ ou -CO-NH₂ ;
n représente un entier choisi parmi 0, 1, 2, 3, 4, 5 et 6 ;
R⁵ représente un radical alkyle, aryle, ou hétéroaryle ;
R⁶ et R⁷ représentent indépendamment un atome d'hydrogène, un radical alkyle ou alkyloxycarbonyl ;
R⁸ représente un atome d'hydrogène ou un radical alkyle ;
R³ représente un radical alkyle ou un atome d'hydrogène ; ou bien les deux radicaux R³ forment ensemble avec l'atome de carbone auquel ils se rattachent un radical cycloalkyle comportant de 3 à 6 chaînons ;
R⁴ représente un radical haloalkyle de 2 à 10 atomes de carbone ;
Y représente une chaîne alkylène de 2 à 14 atomes de carbone, linéaire ou ramifiée, cette chaîne pouvant être saturée ou insaturée, et pouvant contenir un ou plusieurs chaînons -O- supplémentaires ;
X représente -S-, -SO-, -SO₂-, -S=N(R⁹)- ou -S(O)=N(R⁹)-,
R⁹ représente un atome d'hydrogène ou un radical haloalkylcarbonyle
ou un sel pharmaceutiquement acceptable de ce dernier.

De préférence, Y représente une chaîne alkylène de 2 à 14 atomes de carbone linéaire saturée.

De préférence, R¹ représente un atome d'halogène, ou bien un radical alkyle, alkoxy, cyano, nitro, amino, alkylamino, dialkylamino, -NR⁸-CO-R⁵, -NR⁸-SO₂-R⁵, -NR⁸-CO-(CH₂)ₙ-NR⁶ R⁷, -NR⁸-SO₂-(CH₂)ₙ-NR⁶ R⁷, ou -CO-NH₂; R² représente un atome d'halogène, un radical alkyle ou haloalkyle.

De préférence, R² représente un atome d'hydrogène.

De préférence, R⁸ représente un atome d' hydrogène.

De préférence, R⁵ représente un radical alkyle.

De préférence, R³ représente un radical alkyle.

De préférence, R⁴ représente un radical haloalkyle comportant de 4 à 6 atomes de carbone et 3 à 9 atomes de fluor ; et Y représente une chaîne alkylène de 5 à 10 atomes de carbone.

De préférence, n représente un entier choisi parmi 0, 1, 2, 3 et 4. De préférence, n vaut 0 ou 1. De préférence, n vaut 0. De préférence, n vaut 1. Selon une alternative, n vaut 2. Selon une alternative, n vaut 3.

De préférence, R¹ est en position para.

De préférence, R² est en position meta.

De préférence, R¹ représente un radical cyano, nitro, amino, -NR⁸-CO-R⁵, -NR⁸-SO₂-R⁵, -NR⁸-CO-(CH₂)ₙ-NR⁶R⁷, -NR⁸-SO₂-(CH₂)ₙ-NR⁶ R⁷, ou -CO-NH₂ ;

R² représente un radical alkyle ou haloalkyle ;

R⁵ représente un radical alkyle ;

R⁶ et R⁷ représentent indépendamment un atome d'hydrogène, un radical alkyle ou alkyloxycarbonyle ;

R³ représente un radical alkyle ou bien les deux radicaux R³ forment ensemble avec l'atome de carbone auquel ils se rattachent un radical cycloalkyle comportant de 3 à 6 chaînons ;

R⁴ représente un radical haloalkyle comportant de 4 à 6 atomes de carbone et 3 à 9 atomes de fluor ;

n vaut 0 ou 1 ; et

R⁹ représente un atome d'hydrogène ou -COCF₃.

De préférence, R¹ représente un radical cyano, nitro, amino, -NH-CO-R⁵, -CO-NH₂ , - NH-CO-NHR⁶, -NH-SO₂-NHR⁶ ;

R² représente un radical alkyle ou haloalkyle ;

R⁵ représente un radical alkyle ;

R⁶ représente un atome d'hydrogène, un radical alkyle ou alkyloxycarbonyl ;

R³ représente un radical alkyle ;

R⁴ représente un radical haloalkyle comportant de 4 à 6 atomes de carbone et 3 à 9 atomes de fluor ;

Et n représente un entier compris entre 5 et 11.

De préférence, R¹ représente un radical cyano, nitro, -NR⁸-CO-R⁵, -NR⁸-SO₂-R⁵, -NR⁸-CO-(CH₂)ₙNR⁶ R⁷, -NR⁸-SO₂-(CH₂)ₙ-NR⁶ R⁷, ou -CO-NH₂ ; n vaut 0 ou 1 ; R⁵ représente un radical alkyle, R⁶ et R⁷ représentent indépendamment un atome d'hydrogène ou un radical alkyle, et R² représente un radical alkyle ou haloalkyle.

De préférence, R¹ représente un radical cyano, nitro, -NH-CO-R⁵, -CO-NH₂, -NH-CO-NHR⁶, -NH-SO₂-NHR⁶ ; R⁵ représente un radical alkyle, R⁶ représente un atome d'hydrogène ou un radical alkyle, et R² représente un radical alkyle ou haloalkyle.

De préférence, R¹ représente un radical nitro ou -NR⁸-CO-R⁵ dans lequel R⁵ représente un radical alkyle.

De préférence, R¹ représente un radical nitro ou -NH-CO-R⁵ dans lequel R⁵ représente un radical alkyle.

De préférence, le radical alkyle représente un groupement méthyle et/ou le radical haloalkyle représente un groupement triflurométhyle, ou un radical de formule brute C₅H₆F₅, C₅H₄F₇, C₆H₈F₅, C₆H₆F₇ ou C₆H₄F₉.

De préférence, Y représente une chaîne alkylène de 9 à 10 atomes de carbones.

De manière préférentielle, la présente invention a pour objet un composé de formule générale (I) choisi parmi :
- 5,5-diméthyl-3-[4-nitro-3-(trifluorométhyl)phényl]-1-{9-[(4,4,5,5,5-pentafluoropentyl)thio]nonyl}imidazolidine-2,4-dione
- 5,5-diméthyl-3-[4-nitro-3-(trifluorométhyl)phényl]-1-{9-[(4,4,5,5,5-pentafluoropentyl)sulfinyl]nonyl}imidazolidine-2,4-dione
- 5,5-diméthyl-3-[4-nitro-3-(trifluorométhyl)phényl]-1-{9-[(4,4,5,5,5-pentafluoropentyl)sulfonyl]nonyl}imidazolidine-2,4-dione
- 5,5-diméthyl-3-[4-nitro-3-(trifluorométhyl)phényl]-1-{5-[(4,4,5,5,5-pentafluoropentyl)thio]pentyl}imidazolidine-2,4-dione
- 5,5-diméthyl-3-[4-nitro-3-(trifluorométhyl)phényl]-1-{5-[(4,4,5,5,5-pentafluoropentyl)sulfinyl]pentyl}imidazolidine-2,4,-dione
- 5,5-diméthyl-3-[4-nitro-3-(trifluorométhyl)phényl]-1-{8-[(4,4,5,5,5-pentafluoropentyl)thio]octyl}imidazolidine-2,4-dione
- 5,5-diméthyl-3-[4-nitro-3-(trifluorométhyl)phényl]-1-{8-[(4,4,5,5,5-pentafluoropentyl)sulfinyl]octyl}imidazolidine-2,4-dione
- 5,5-diméthyl-3-[4-nitro-3-(trifluorométhyl)phényl]-1-{10-[(4,4,5,5,5-pentafluoropentyl)thio]decyl}imidazolidine-2,4-dione
- 5,5-diméthyl-3-[4-nitro-3-(trifluorométhyl)phényl]-1-{10-[(4,4,5,5,5-pentafluoropentyl)sulfinyl]décyl}imidazolidine-2,4-dione
- 5,5-diméthyl-3-[4-nitro-3-(trifluorométhyl)phényl]-1-{11-[(4,4,5,5,5-pentafluoropentyl)thio]undecyl}imidazolidine-2,4-dione
- 5,5-diméthyl-3-[4-nitro-3-(trifluorométhyl)phényl]-1-{11-[(4,4,5,5,5-pentafluoropentyl)sulfinyl]undécyl}imidazolidine-2,4-dione
- 5,5-diméthyl-3-[4-nitro-3-(trifluorométhyl)phényl]-1-{9-[(4,4,4-trifluorobutyl)thio]nonyl}imidazolidine-2,4-dione
- 5,5-diméthyl-3-[4-nitro-3-(trifluorométhyl)phényl]-1-{9-[(4,4,4-trifluorobutyl)sulfinyl]nonyl}imidazolidine-2,4-dione
- 5,5-diméthyl-3-[4-nitro-3-(trifluorométhyl)phényl]-1-{9-[(3,3,4,4,5,5,6,6,6-nonafluorohexyl)thio]nonyl}imidazolidine-2,4-dione
- 5,5-diméthyl-3-[4-nitro-3-(trifluorométhyl)phényl]-1-{9-[(3,3,4,4,5,5,6,6,6-nonafluorohexyl)sulfinyl]nonyl}imidazolidine-2,4-dione
- 3-[4-amino-3-(trifluorométhyl)phényl]-5,5-diméthyl-1-{9-[(4,4,5,5,5-pentafluoropentyl)thio]nonyl}imidazolidine-2,4-dione
- N-[4-(4,4-diméthyl-2,5-dioxo-3-{9-[(4,4,5,5,5-pentafluoropentyl)thio]-nonyl}imidazolidin-1-yl)-2-(trifluorométhyl)phényl]acetamide
- N-[4-(4,4-diméthyl-2,5-dioxo-3-{9-[(4,4,5,5,5-pentafluoropentyl)sulfinyl]-nonyl}imidazolidin-1-yl)-2-(trifluorométhyl)phényl]acetamide
- 4-(4,4-dimethyl-2,5-dioxo-3-{9-[(4,4,5,5,5-pentafluoropentylsulfanyl]nonyl} imidazolidin-1-yl)-2-(trifluoromethyl)benzonitrile
- 4-(4,4-dimethyl-2,5-dioxo-3-{9-[(4,4,5,5,5-pentafluoropentyl)sulfinyl] nonyl}imidazolidin-1-yl)-2-(trifluoromethyl)benzonitrile
- 4-(4,4-dimethyl-2,5-dioxo-3-{9-[(4,4,5,5,5-pentafluoropentyl)sulfanyl] nonyl}imidazolidin-1-yl)-2-(trifluoromethyl)benzamide
- 4-(4,4-dimethyl-2,5-dioxo-3-{9-[(4,4,5,5,5-pentafluoropentyl)sulfinyl] nonyl}imidazolidin-1-yl)-2-(trifluoromethyl)benzamide
- 5,5-dimethyl-3-(3-methyl-4-nitrophenyl)-1-{9-[(4,4,5,5,5-pentafluoro pentyl)sulfanyl]nonyl}imidazolidine-2,4-dione
- 5,5-dimethyl-3-(3-methyl-4-nitrophenyl)-1-{9-[(4,4,5,5,5-pentafluoro pentyl)sulfinyl]nonyl}imidazolidine-2,4-dione
- 3-(4-amino-3-methylphenyl)-5,5-dimethyl-1-{9-[(4,4,5,5,5-pentafluoro pentyl)sulfanyl]nonyl}imidazolidine-2,4-dione
- 3-(4-amino-3-methylphenyl)-5,5-dimethyl-1-{9-[(4,4,5,5,5-pentafluoro pentyl)sulfinyl]nonyl}imidazolidine-2,4-dione
- 1-[4-(4,4-dimethyl-2,5-dioxo-3-{9-[(4,4,5,5,5-pentafluoropentyl)sulfanyl] nonyl}imidazolidin-1-yl)-2-methylphenyl]-3-(1-methylpropyl)urea
- 1-[4-(4,4-dimethyl-2,5-dioxo-3-{9-[(4,4,5,5,5-pentafluoropentyl)sulfinyl} nonyl}imidazolidin-1-yl)-2-methylphenyl]-3-(1-methylpropyl)urea
- tert-butyl {[4-(4,4-dimethyl-2,5-dioxo-3-{9-[(4,4,5,5,5-pentafluoropentyl)sulfanyl]nonyl}imidazolidin-1-yl)-2-methylphenyl]sulfamoyl}carbamate
- N-[4-(4,4-dimethyl-2,5-dioxo-3-{9-[(4,4,5,5,5-pentafluoropentyl)sulfanyl] nonyl}imidazolidin-1-yl)-2-methylphenyl]sulfamide
- N-[4-(4,4-dimethyl-2,5-dioxo-3-{9-[(4,4,5,5,5-pentafluoropentyl)sulfinyl] nonyl}imidazolidin-1-yl)-2-methylphenyl]sulfamide
- N-[4-(4,4-dimethyl-2,5-dioxo-3-{9-[(4,4,5,5,5-pentafluoropentyl)sulfonyl] nonyl}imidazolidin-1-yl)-2-methylphenyl]sulfamide
- 3-[4-amino-3-(trifluorométhyl)phényl]-5,5-diméthyl-1-{9-[(4,4,5,5,5-pentafluoropentyl)sulfinyl]nonyl}imidazolidine-2,4-dione.
- 7-[4-nitro-3-(trifluoromethyl)phenyl]-5-{9-[(4,4,5,5,5-pentafluoropentyl)sulfinyl]nonyl}-5,7-diazaspiro[3.4]octane-6,8-dione
- 5,5-dimethyl-3-[4-nitro-2-(trifluoromethyl)phenyl]-1-{9-[(4,4,5,5,5-pentafluoropentyl)sulfinyl]nonyl}imidazolidine-2,4-dione
- 5,5-dimethyl-3-[4-nitro-3-(trifluoromethyl)phenyl]-1-[2-(2-{2-[(4,4,5,5,5-pentafluoropentyl)sulfanyl]ethoxy}ethoxy)ethyl]imidazolidine-2,4-dione
- 5,5-dimethyl-3-[4-nitro-3-(trifluoromethyl)phenyl]-1-[2-(2-{2-[(4,4,5,5,5-pentafluoropentyl)sulfinyl]ethoxy}ethoxy)ethyl]imidazolidine-2,4-dione
- N-[4-{4,4-dimethyl-2,5-dioxo-3-[2-(2-{2-[(4,4,5,5,5-pentafluoropentyl)sulfanyl]ethoxy}ethoxy)ethyl]imidazolidin-1-yl}-2-(trifluoromethyl)phenyl]acetamide
- N-[4-{4,4-dimethyl-2,5-dioxo-3-[2-(2-{2-[(4,4,5,5,5-pentafluoropentyl)sulfinyl]ethoxy}ethoxy)ethyl]imidazolidin- 1-yl}-2-(trifluoromethyl)phenyl]acetamide
- N-[4-(4,4-dimethyl-2,5-dioxo-3-{9-[(4,4,5,5,5-pentafluoropentyl)sulfinyl]nonyl}imidazolidin-1-yl)-2-(trifluoromethyl)phenyl]-N-methylacetamide
- N-[4-(4,4-dimethyl-2,5-dioxo-3-{9-[(4,4,5,5,5-pentafluoropentyl)sulfinyl]nonyl}imidazolidin-1-yl)-2-(trifluoromethyl)phenyl]methanesulfonamide
- N-[4-(4,4-dimethyl-2,5-dioxo-3-{9-[(4,4,5,5,5-pentafluoropentyl)sulfanyl]nonyl}imidazolidin-1-yl)-2-(trifluoromethyl)phenyl]-N2,N2-dimethylglycinamide
- N-[4-(4,4-dimethyl-2,5-dioxo-3-{9-[(4,4,5,5,5-pentafluoropentyl)sulfinyl]nonyl}imidazolidin-1-yl)-2-(trifluoromethyl)phenyl]-N2,N2-dimethylglycinamide
- Chlorhydrate de N-[4-(4,4-dimethyl-2,5-dioxo-3-{9-[(4,4,5,5,5-pentafluoropentyl)sulfinyl]nonyl}imidazolidin-1-yl)-2-(trifluoromethyl)phenyl]-N2,N2-dimethylglycinamide
- N-[(1Z)-(9-{5,5-dimethyl-3-[4-nitro-3-(trifluoromethyl)phenyl]-2,4-dioxoimidazolidin-1-yl}nonyl)(4,4,5,5,5-pentafluoropentyl)-□4-sulfanylidene]-2,2,2-trifluoroacetamide
- N-[(9-{5,5-dimethyl-3-[4-nitro-3-(trifluoromethyl)phenyl]-2,4-dioxoimidazolidin-1-yl}nonyl)(oxido)(4,4,5,5,5-pentafluoropentyl)-□4-sulfanylidene]-2,2,2-trifluoroacetamide
- 5,5-dimethyl-3-[4-nitro-3-(trifluoromethyl)phenyl]-1-{9-[S-(4,4,5,5,5-pentafluoropentyl)sulfonimidoyl]nonyl}imidazolidine-2,4-dione.

De préférence, le composé de formule (I) est choisi parmi :
- 5,5-diméthyl-3-[4-nitro-3-(trifluorométhyl)phényl]-1-{9-[(4,4,5,5,5-pentafluoropentyl)thio]nonyl}imidazolidine-2,4-dione
- 5,5-diméthyl-3-[4-nitro-3-(trifluorométhyl)phényl]-1-{9-[(4,4,5,5,5-pentafluoropentyl)sulfinyl]nonyl}imidazolidine-2,4-dione
- 5,5-diméthyl-3-[4-nitro-3-(trifluorométhyl)phényl]-1-{9-[(4,4,5,5,5-pentafluoropentyl)sulfonyl]nonyl}imidazolidine-2,4-dione
- 5,5-diméthyl-3-[4-nitro-3-(trifluorométhyl)phényl]-1-{5-[(4,4,5,5,5-pentafluoropentyl)thio]pentyl}imidazolidine-2,4-dione
- 5,5-diméthyl-3-[4-nitro-3-(trifluorométhyl)phényl]-1-{5-[(4,4,5,5,5-pentafluoropentyl)sulfinyl]pentyl}imidazolidine-2,4-dione
- 5,5-diméthyl-3-[4-nitro-3-(trifluorométhyl)phényl]-1-{8-[(4,4,5,5,5-pentafluoropentyl)thio]octyl}imidazolidine-2,4-dione
- 5,5-diméthyl-3-[4-nitro-3-(trifluorométhyl)phényl]-1-{8-[(4,4,5,5,5-pentafluoropentyl)sulfinyl]octyl}imidazolidine-2,4-dione
- 5,5-diméthyl-3-[4-nitro-3-(trifluorométhyl)phényl]-1-{10-[(4,4,5,5,5-pentafluoropentyl)thio]decyl}imidazolidine-2,4-dione
- 5,5-diméthyl-3-[4-nitro-3-(trifluorométhyl)phényl]-1-{10-[(4,4,5,5,5-pentafluoropentyl)sulfinyl]décyl}imidazolidine-2,4-dione
- 5,5-diméthyl-3-[4-nitro-3-(trifluorométhyl)phényl]-1-{11-[(4,4,5,5,5-pentafluoropentyl)thio]undecyl}imidazolidine-2,4-dione
- 5,5-diméthyl-3-[4-nitro-3-(trifluorométhyl)phényl]-1-{11-[(4,4,5,5,5-pentafluoropentyl)sulfinyl]undécyl}imidazolidine-2,4-dione
- 5,5-diméthyl-3-[4-nitro-3-(trifluorométhyl)phényl]-1-{9-[(4,4,4-trifluorobutyl)thio]nonyl}imidazolidine-2,4-dione
- 5,5-diméthyl-3-[4-nitro-3-(trifluorométhyl)phényl]-1-{9-[(4,4,4-trifluorobutyl)sulfinyl]nonyl}imidazolidine-2,4-dione
- 5,5-diméthyl-3-[4-nitro-3-(trifluorométhyl)phényl]-1-{9-[(3,3,4,4,5,5,6,6,6-nonafluorohexyl)thio]nonyl}imidazolidine-2,4-dione
- 5,5-diméthyl-3-[4-nitro-3-(trifluorométhyl)phényl]-1-{9-[(3,3,4,4,5,5,6,6,6-nonafluorohexyl)sulfinyl]nonyl}imidazolidine-2,4-dione
- 3-[4-amino-3-(trifluorométhyl)phényl]-5,5-diméthyl-1-{9-[(4,4,5,5,5-pentafluoropentyl)thio]nonyl}imidazolidine-2,4-dione
- N-[4-(4,4-diméthy!-2,5-dioxo-3-{9-[(4,4,5,5,5-pentafluoropentyl)thio]-nonyl}imidazolidin-1-yl)-2-(trifluorométhyl)phényl]acetamide
- N-[4-(4,4-diméthyl-2,5-dioxo-3-{9-[(4,4,5,5,5-pentafluoropentyl)sulfinyl]-nonyl}imidazolidin-1-yl)-2-(trifluorométhyl)phényl]acetamide
- 4-(4,4-dimethyl-2,5-dioxo-3-{9-[(4,4,5,5,5-pentafluoropentylsulfanyl]nonyl} imidazolidin-1-yl)-2-(trifluoromethyl)benzonitrile
- 4-(4,4-dimethyl-2,5-dioxo-3-{9-[(4,4,5,5,5-pentafluoropentyl)sulfinyl] nonyl}imidazolidin-1-yl)-2-(trifluoromethyl)benzonitrile
- 4-(4,4-dimethyl-2,5-dioxo-3-{9-[(4,4,5,5,5-pentafluoropentyl)sulfanyl] nonyl}imidazolidin-1-yl)-2-(trifluoromethyl)benzamide
- 4-(4,4-dimethyl-2,5-dioxo-3-{9-[(4,4,5,5,5-pentafluoropentyl)sulfinyl] nonyl}imidazolidin-1-yl)-2-(trifluoromethyl)benzamide
- 5,5-dimethyl-3-(3-methyl-4-nitrophenyl)-1-{9-[(4,4,5,5,5-pentafluoro pentyl)sulfanyl]nonyl}imidazolidine-2,4-dione
- 5,5-dimethyl-3-(3-methyl-4-nitrophenyl)-1-{9-[(4,4,5,5,5-pentafluoro pentyl)sulfinyl]nonyl}imidazolidine-2,4-dione
- 3-(4-amino-3-methylphenyl)-5,5-dimethyl-1-{9-[(4,4,5,5,5-pentafluoro pentyl)sulfanyl]nonyl}imidazolidine-2,4-dione
- 3-(4-amino-3-methylphenyl)-5,5-dimethyl-1-{9-[(4,4,5,5,5-pentafluoro pentyl)sulfinyl]nonyl}imidazolidine-2,4-dione
- 1-[4-(4,4-dimethyl-2,5-dioxo-3-{9-[(4,4,5,5,5-pentafluoropentyl)sulfanyl] nonyl}imidazolidin-1-yl)-2-methylphenyl]-3-(1-methylpropyl)urea
- 1-[4-(4,4-dimethyl-2,5-dioxo-3-{9-[(4,4,5,5,5-pentafluoropentyl)sulfinyl] nonyl}imidazolidin-1-yl)-2-methylphenyl]-3-(1-methylpropyl)urea
- tert-butyl {[4-(4,4-dimethyl-2,5-dioxo-3-{9-[(4,4,5,5,5-pentafluoropentyl)sulfanyl] nonyl}imidazolidin-1-yl)-2-methylphenyl]sulfamoyl} carbamate
- N-[4-(4,4-dimethyl-2,5-dioxo-3-{9-[(4,4,5,5,5-pentafluoropentyl)sulfanyl] nonyl}imidazolidin-1-yl)-2-methylphenyl]sulfamide
- N-[4-(4,4-dimethyl-2,5-dioxo-3-{9-[(4,4,5,5,5-pentafluoropentyl)sulfinyl] nonyl}imidazolidin-1-yl)-2-methylphenyl]sulfamide
- N-[4-(4,4-dimethyl-2,5-dioxo-3-{9-[(4,4,5,5,5-pentafluoropentyl)sulfonyl] nonyl}imidazolidin-1-yl)-2-methylphenyl]sulfamide
- 3-[4-amino-3-(trifluorométhyl)phényl]-5,5-diméthyl-1-{9-[(4,4,5,5,5-pentafluoropentyl)sulfinyl]nonyl}imidazolidine-2,4-dione.

De manière encore plus préférentielle, la présente invention a pour objet un composé de formule générale (I) choisi parmi :
- 5,5-diméthyl-3-[4-nitro-3-(trifluorométhyl)phényl]-1-{9-[(4,4,5,5,5-pentafluoropentyl)sulfinyl]nonyl}imidazolidine-2,4-dione
- 5,5-diméthyl-3-[4-nitro-3-(trifluorométhyl)phényl]-1-{10-[(4,4,5,5,5-pentafluoropentyl)sulfinyl]décyl}imidazolidine-2,4-dione
- 5,5-diméthyl-3-[4-nitro-3-(trifluorométhyl)phényl]-1-{9-[(4,4,4-trifluorobutyl)thio]nonyl}imidazolidine-2,4-dione
- 5,5-diméthyl-3-[4-nitro-3-(trifluorométhyl)phényl]-1-{9-[(4,4,4-trifluorobutyl)sulfinyl]nonyl}imidazolidine-2,4-dione
- N-[4-(4,4-diméthyl-2,5-dioxo-3-{9-[(4,4,5,5,5-pentafluoropentyl)sulfinyl]-nonyl}imidazolidin-1-yl)-2-(trifluorométhyl)phényl]acetamide
- N-[4-(4,4-dimethyl-2,5-dioxo-3-{9-[(4,4,5,5,5-pentafluoropentyl)sulfinyl]nonyl}imidazolidin-1-yl)-2-(trifluoromethyl)phenyl]-N2,N2-dimethylglycinamide
- Chlorhydrate de N-[4-(4,4-dimethyl-2,5-dioxo-3-{9-[(4,4,5,5,5-pentafluoropentyl)sulfinyl]nonyl}imidazolidin-1-yl)-2-(trifluoromethyl)phenyl]-N2,N2-dimethylglycinamide.

De préférence, le composé est choisi parmi
- 5,5-diméthyl-3-[4-nitro-3-(trifluorométhyl)phényl]-1-{9-[(4,4,5,5,5-pentafluoropentyl)sulfinyl]nonyl}imidazolidine-2,4-dione ;
- 5,5-diméthyl-3-[4-nitro-3-(trifluorométhyl)phényl]-1-{10-[(4,4,5,5,5-pentafluoropentyl)sulfinyl]décyl}imidazolidine-2,4-dione ;
- 5,5-diméthyl-3-[4-nitro-3-(trifluorométhyl)phényl]-1-{9-[(4,4,4-trifluorobutyl)thio]nonyl}imidazolidine-2,4-dione ;
- 5,5-diméthyl-3-[4-nitro-3-(trifluorométhyl)phényl]-1-{9-[(4,4,4-trifluorobutyl)sulfinyl]nonyl}imidazolidine-2,4-dione ;
- N-[4-(4,4-diméthyl-2,5-dioxo-3-{9-[(4,4,5,5,5-pentafluoropentyl)sulfinyl]-nonyl}imidazolidin-1-yl)-2-(trifluorométhyl)phényl]acetamide.

De préférence, le composé est le N-[4-(4,4-diméthyl-2,5-dioxo-3-{9-[(4,4,5,5,5-pentafluoropentyl)sulfinyl]-nonyl}imidazolidin-l-yl)-2-(trifluorométhyl)phényl]acetamide.

De préférence, le composé est le N-[4-(4,4-dimethyl-2,5-dioxo-3-{9-[(4,4,5,5,5-pentafluoropentyl)sulfinyl]nonyl}imidazolidin-1-yl)-2-(trifluoromethyl)phenyl]-N2,N2-dimethylglycinamide.

De préférence, le composé est le chlorhydrate de N-[4-(4,4-dimethyl-2,5-dioxo-3-{9-[(4,4,5,5,5-pentafluoropentyl)sulfinyl]nonyl}imidazolidin-1-yl)-2-(trifluoromethyl)phenyl]-N2,N2-dimethylglycinamide.

L'invention a également pour objet un procédé de préparation d'un composé de formule (**I**) tel que défini précédemment, caractérisé en ce qu'il comprend l'obtention de composés de formule générale (**I.1**) (composé de formule générale (**I**) dans laquelle X représente l'atome de soufre), 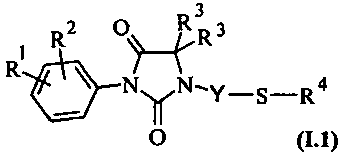 dans laquelle R¹, R², R³, R⁴ et Y sont tels que définis précédemment,
- soit par condensation des dérivés d'hydantoine de formule générale (**II**) 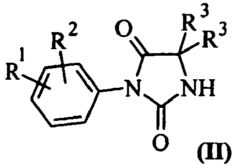
dans laquelle R¹, R², et R³ sont tels que définis précédemment, en présence d'une base forte à une température comprise entre 25 et 60° C, dans un solvant polaire anhydre, sur les dérivés mésylates de formule générale **(III)**, 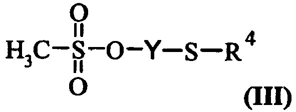 dans laquelle R⁴ et Y sont tels que définis précédemment,
- soit par le traitement de dérivés thiobenzoyles de formule générale **(V)**, 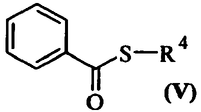 dans laquelle R⁴ est tel que défini précédemment, par un alcoolate dans un solvant protique polaire suivi de
- l'addition du dérivé halogéné de formule générale (**IV**), 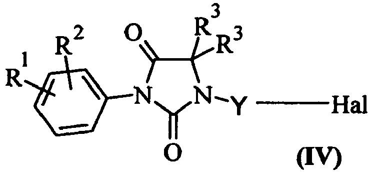 dans laquelle R¹, R², R³ et Y sont tels que définis précédemment, en solution dans un solvant polaire.

De préférence, le procédé comprend en outre une étape d'oxydation des composés de formule générale **(I.1)** en sulfoxyde de formule générale **(I.2)** (composé de formule générale **(I)** dans laquelle X représente le radical -SO-), 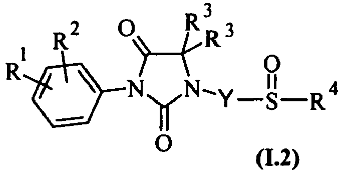 dans laquelle R¹, R², R³, R⁴ et Y sont tels que définis précédemment.

De préférence, le procédé de préparation d'un composé de formule **(I),** comprend en outre une étape d'oxydation des dérivés sulfoxydes de formule générale **(I.2)** en sulfones de formule générale **(I.3)** (composé de formule générale **(I)** dans laquelle X représente le groupe -SO₂-), 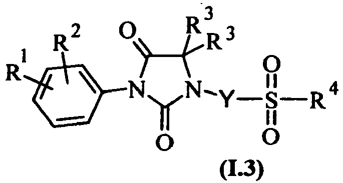 dans laquelle R¹, R², R³, R⁴ et Y sont tels que définis précédemment.

L'invention a également pour objet l'un des composés suivants à titre d'intermédiaire:
- 1-(5-iodopentyl)-5,5-dimethyl-3-[4-nitro-3-(trifluoromethyl)phenyl]imidazolidine-2,4-dione
- 1-(8-iodooctyl)-5,5-dimethyl-3-[4-nitro-3-(trifluoromethyl)phenyl]imidazolidine-2,4-dione
- 1-(9-bromononyl)-5,5-dimethyl-3-[4-nitro-3-(trifluoromethyl)phenyl]imidazolidine-2,4-dione
- 4-[3-(9-bromononyl)-4,4-dimethyl-2,5-dioxoimidazolidin-1-yl]-2-(trifluoromethyl)benzonitrile
- 5,5-dimethyl-3-(3-methyl-4-nitrophenyl)imidazolidine-2,4-dione
- 1-(9-bromononyl)-5,5-dimethyl-3-(3-methyl-4-nitrophenyl)imidazolidine-2,4-dione.

L'invention a également pour objet l'un des composés de formule **(I)** en tant que médicament.

L'invention a également pour objet les compositions pharmaceutiques contenant, à titre de principe actif, au moins un composé de formule **(I)** tel que défini précédemment, en association avec un support pharmaceutiquement acceptable.

L'invention a également pour objet l'utilisation d'un composé de formule **(I)** pour la préparation d'un médicament destiné à traiter les cancers.

De préférence, le médicament est destiné à traiter un cancer hormono-dépendant.

De préférence, le médicament est destiné à traiter un cancer exprimant les récepteurs aux androgènes

De préférence, le médicament est destiné à traiter un cancer du sein ou de la prostate.

### BREVE DESCRIPTION DES FIGURES

La figure 1 représente l'effet des composés 18 et 43a sur la prolifération cellulaire des LNCaP cultivées en milieu sans stéroïde.
Les figures 2 et 3 représentent les effets des composés 18 et 43a sur la diminution de l'expression protéique du récepteur aux androgènes.
La figure 4 représente l'effet du nilutamide sur la diminution de l'expression protéique du récepteur aux androgènes.

### DESCRITION DETAILLEE DE MODES DE REALISATION DE L'INVENTION

L'invention a donc pour objet des composés de formule générale **(I)** 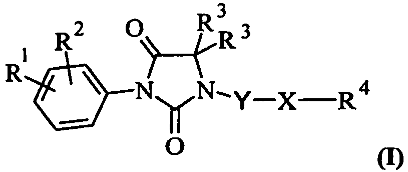 sous forme racémique, d'énantiomère ou toutes combinaisons de ces formes.

R¹ et R² représentent indépendamment un atome d'halogène, ou bien un radical alkyle, haloalkyle, alkoxy, cyano, nitro, amino, alkylamino, dialkylamino, -NR⁸-CO-R⁵, -NR⁸-SO₂-R⁵, -NR⁸-CO-(CH₂)ₙ-NR⁶ R⁷, -NR⁸-SO₂-(CH₂)ₙ-NR⁶R⁷ ou -CO-NH₂.

n représente un entier compris entre 0 et 6.

R⁵ représente un radical alkyle, aryle, ou hétéroaryle. De préférence, R5 est un radical alkyle.

R⁶ et R⁷ représentent indépendamment un atome d'hydrogène, un radical alkyle ou alkyloxycarbonyle.

R⁸ représente un atome d'hydrogène ou un radical alkyle. De préférence, R⁸ représente un atome d'hydrogène.

R³ représente un radical alkyle ou un atome d'hydrogène. Alternativement, les deux radicaux R³ forment ensemble avec l'atome de carbone auquel ils se rattachent un radical cycloalkyle comportant de 3 à 6 chaînons.

R⁴ représente un radical haloalkyle de 2 à 10 atomes de carbone.

Y représente une chaîne alkylène de 2 à 14 atomes de carbone. La chaîne Y peut être linéaire ou ramifiée. Cette chaîne peut être saturée ou insaturée. Elle peut contenir un ou plusieurs chaînons -O- supplémentaires.

X représente -S-, -SO-, -SO₂-, -S=N(R⁹)- ou -S(O)=N(R⁹)-.

R⁹ représente un atome d'hydrogène ou un radical haloalkylcarbonyle.

Les composés de formule (I) peuvent être sous forme d'un sel pharmaceutiquement acceptable.

Par sel pharmaceutiquement acceptable, on entend notamment des sels d'addition d'acides inorganiques tels que chlorhydrate, bromhydrate, sulfate, phosphate, diphosphate et nitrate ou d'acides organiques tels que acétate, maléate, fumarate, tartrate, succinate, citrate, lactate, méthanesulfonate, benzènesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt sélection for basic drugs", Int. J. Pharm. (1986), 33, 201-217.

Dans les définitions indiquées ci-dessus, l'expression halogène (ou halo) représente le radical fluoro, chloro, bromo ou iodo, de préférence chloro, fluoro ou bromo. De façon plus préférentielle, l'expression halogène (ou halo) représente un radical fluoro.

Lorsqu'il n'est pas donné plus de précisions, le terme alkyle au sens de la présente invention représente un radical alkyl linéaire ou ramifié comprenant entre 1 et 12 atomes de carbones tels que les radicaux méthyle, éthyle, propyle, isopropyle, butyle, isobutyle, *sec*-butyle et *tert*-butyle, pentyle ou amyle, isopentyle, neopentyle, hexyle ou isohexyle, heptyle, octyle, nonyle, décyle, undécyle ou dodécyle. De manière préférentielle le radical alkyle sera un radical (C₁-C₆)alkyle, c'est-à-dire représentant un radical alkyle ayant de 1 à 6 atomes de carbone tel que défini ci-dessus, ou un radical (C₁-C₄)alkyle représentant un radical alkyle ayant de 1 à 4 atomes de carbone tel que par exemple les radicaux méthyle, éthyle, propyle, isopropyle, butyle, isobutyle, *sec*-butyle et *tert*-butyle.

Le terme alkyle dans les expressions alkoxy (ou alkyloxy), alkylamino, dialkylamino, haloalkyle ou alkoxycarbonyle (ou alkyloxycarbonyle) représente un radical alkyle tel que défini ci-dessus.

Plus particulièrement, par haloalkyle, on entend un radical alkyle dont au moins l'un des atomes d'hydrogène (et éventuellement tous) est remplacé par un atome d'halogène (halo) comme par exemple, et préférentiellement trifluorométhyle ou des radicaux de formule brute C₅H₆F₅, C₅H₄F₇, C₆H₈F₅, C₆H₆F₇ ou C₆H₄F₉.

Le terme haloalkyle dans l'expression haloalkylcarbonyle réprésente un radical haloalkyle tel que défini ci-dessus.

Par cycloalkyle lorsqu'il n'est pas donné plus de précision, on entend un radical cyclique carboné saturé comprenant 3 à 6 chaînons tel que le cyclopropyle, le cyclobutyle, le cyclopentyle ou le cyclohexyle.

Au sens de la présente invention, les radicaux aryles peuvent être de type mono ou polycycliques aromatiques. Les radicaux aryles monocycliques peuvent être choisis parmi les radicaux phényles, tolyles, xylyles, mésityles, cuményles et de préférence phényles. Les radicaux aryles polycycliques peuvent être choisis parmi les radicaux naphtyle, anthryle, phénanthryle, fluorényle. Ils peuvent être optionnellement substitués par un ou plusieurs radicaux identiques ou différents tels que alkyle, haloalkyle, alkoxy, alkoxycarbonyle, alkylcarbonyloxy, halo, cyano, nitro, aryle, aryloxy, aryloxycarbonyl, ou arylcarbonyloxy.

Au sens de la présente invention, le terme hétéroaryle désigne un cycle insaturé aromatique comprenant un ou plusieurs hétéroatomes identiques ou différents choisis parmi N, O et S tels que furyle, thiènyle isoxazolyle, benzothiadiazolyle, pyridinyle, oxazolyle, pyrazolyle, pyrimidinyle ou quinoxalyle.

Par chaîne alkylène de 2 à 14 atomes de carbone, linéaire ou ramifiée, saturée ou insaturée, on entend par exemple une chaîne méthyle, éthyle, propyle, isopropyle, butyle, isobutyle, *sec*-butyle et *tert*-butyle, pentyle ou amyle, isopentyle, neopentyle, hexyle ou isohexyle, heptyle, octyle, nonyle, décyle, undécyle, dodécyle, éthène, propène, butène, pentène, hexène, heptène, octène, nonène, décylène, undécylène, dodécylène, ou le but-1,3-diène.

Par sel pharmaceutiquement acceptable, on entend notamment des sels d'addition d'acides inorganiques tels que chlorhydrate, bromhydrate, sulfate, phosphate, diphosphate et nitrate ou d'acides organiques tels que acétate, maléate, fumarate, tartrate, succinate, citrate, lactate, méthanesulfonate, benzènesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt selection for basic drugs", Int. J. Pharm. (1986), 33, 201-217.

L'invention a également pour objet un procédé de préparation d'un composé de formule **(I)** tel que défini précédemment. Les composés de formule générale **(I.1)** (composé de formule générale **(I)** dans laquelle X représente l'atome de soufre), 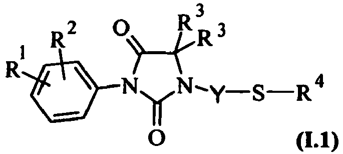 dans laquelle R¹, R², R³, R⁴ et Y sont tels que définis précemment, peuvent être obtenus par condensation des dérivés d'hydantoine de formule générale **(II)** 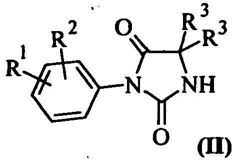 dans laquelle R¹, R², et R³ sont tels que définis précédemment sur les dérivés mésylates de formule générale **(III),** 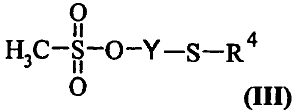 dans laquelle R⁴ et Y sont tels que définis précédemment.

La condensation est réalisée en présence d'une base forte. La condensation est réalisée à une température comprise entre 25 et 60° C. Le solvant est de préférence un solvant polaire anhydre.

Les composés de formule générale **(I.1)** peuvent aussi être obtenus
(i) par le traitement de dérivés thiobenzoyles de formule générale **(V),** 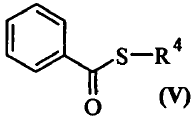
dans laquelle R⁴ est tel que défini précédemment, par un alcoolate dans un solvant protique polaire, traitement suivi de l'addition du dérivé halogéné de formule générale **(IV),** 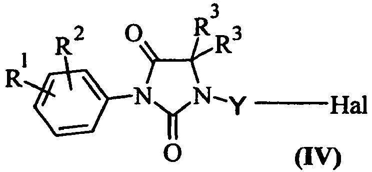 dans laquelle R¹, R², R³ et Y sont tels que définis précédemment, en solution dans un solvant polaire ;

De préférence, les composés de formule générale **(I.1)** sont transformés, par oxydatioh, en sulfoxyde de formule générale **(I.2)** (composé de formule générale **(I)** dans laquelle X représente le radical -SO-), 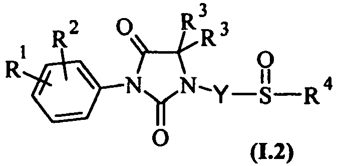 dans laquelle R¹, R², R³, R⁴ et Y sont tels que définis précédemment.

De préférence, les dérivés sulfoxydes de formule générale (I.2) peuvent être transformés, par oxydation, en sulfones de formule générale **(I.3)** (composé de formule générale **(I)** dans laquelle X représente le groupe -SO₂-), 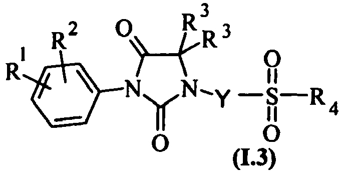 dans laquelle R¹, R², R³, R⁴ et Y sont tels que définis précédemment.

Les composés de formule générale **(I)** selon l'invention peuvent être préparés selon la voie de synthèse représentée dans le Schéma 1 ci-dessous. Les composés de formule générale **(I)** dans laquelle R¹, R², R³, R⁴ et Y sont tels que décrits ci-dessus peuvent être obtenus selon deux approches distinctes selon la facilité d'accès aux intermédiaires. La condensation des dérivés d'hydantoine de formule générale **(II)** sur les dérivés mésylates de formule générale **(III)** (préparés selon un protocole expérimental décrit dans WO 2005077968) est effectuée en présence d'une base, telle que par exemple NaH, de préférence par chauffage entre 25 et 60° C et pour une période de 5 à 15 heures dans un solvant polaire anhydre, tels que par exemple le THF ou le DMF, pour conduire aux composés de formule générale **(I.1).** Alternativement, les composés de formule générale **(I.1)** peuvent être préparés par traitement des dérivés thiobenzoyls de formule générale **(V)** (préparés selon un protocole expérimental décrit dans WO 2005077968) par un alcoolate, tel que par exemple tBuO⁻K⁺, en solution dans un solvant protique polaire, préférentiellement MeOH, pendant une période de 30 minutes à 2 heures, avant l'addition du dérivé halogéné de formule générale **(IV)** en solution dans un solvant polaire, tel que par exemple MeOH. La réaction s'effectue généralement vers 20° C et pendant un temps qui peut varier de 10 à 24 heures. L'oxydation du groupe thioéther des composés de formule générale **(I.1)** en sulfoxyde de formule générale **(I.2)** est effectuée en présence d'une quantité sub-équivalente d'Oxone dans un mélange de solvants tels que THF et eau à une température de 20° C et pendant une période de temps de 15 à 30 minutes.

La transformation des dérivés sulfoxydes de formule générale **(I.2)** en sulfones de formule générale **(I.3)** est également effectuée dans un mélange de solvant tels que THF et eau en présence d'Oxone. Trois équivalents de l'agent oxydant et un temps de réaction de 3 à 5 heures sont généralement nécessaires pour effectuer la conversion totale.

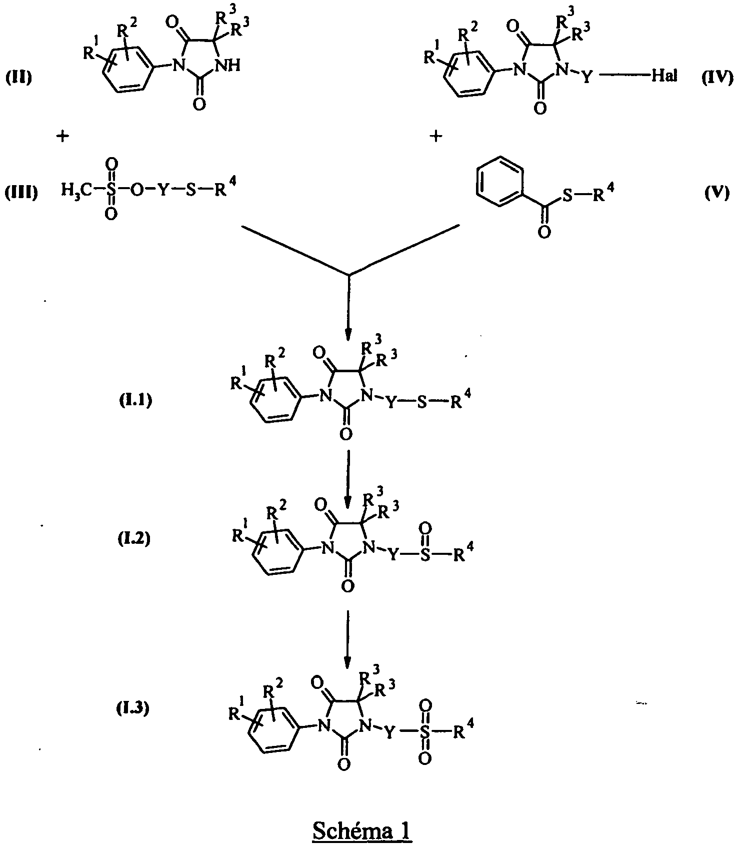

Les dérivés sulfanilidènes de formule générale **(I.2)** dans lesquels R², R³, R⁴, R⁹ et Y sont tels que décrits ci-dessus, sont préparés à partir des dérivés thio alkyle de formule générale **(I.1),** schéma I-bis. La réaction est effectuée dans les conditions expérimentales analogues à celles décrites par C. Bolm et collaborateurs dans Organic Letters 2004, vol.6, N°17, 1305-1307. La réaction s'effectue en faisant réagir un dérivé acétamide R⁹-NH₂ et le composé thio alkyle de formule générale **(I.1)** en présence d'un agent d'oxydation comme par exemple l'oxyde de magnésium, à une température comprise entre 20°C et 30°C (de préférence 25°C), dans un solvant inerte tel que par exemple le tetrahydrofurane ou un solvant chloré et en particulier le dichlorométhane et pour une durée de plusieurs heures. Le dérivé de formule générale **(I.2)-bis** est ensuite mis en réaction à une température comprise entre 20°C et 30°C (de préférence 25°C), en présence d'une base minérale et en particulier l'hydroxyde de potassium dans un solvant inerte tel que par exemple le tetrahydrofurane, un solvant alcooylé et en particulier le méthanol et pour une durée de plusieurs heures pour conduire au composé de formule générale **(I-2).**

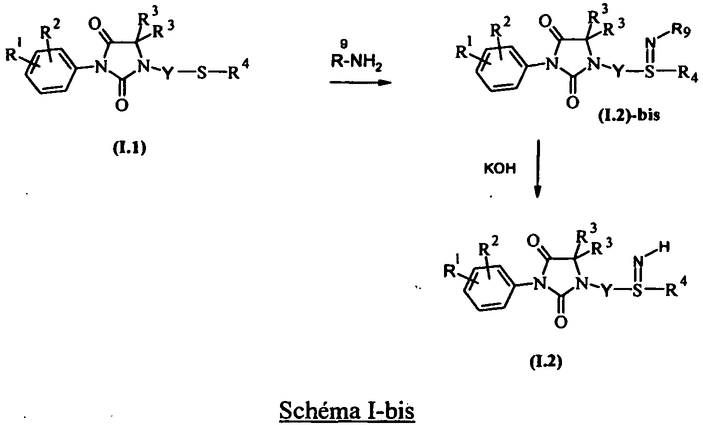

Les dérivés sulfonimides de formule générale **(I.3)** dans lesquels R², R³, R⁴, R⁹ et Y sont tels que décrits ci-dessus, sont préparés à partir des dérivés sulfoxyde de formule générale **(I.2),** schéma I-ter. La réaction est effectuée dans les conditions expérimentales analogues à celles décrites par C. Bolm et collaborateurs dans Organic Letters 2004, vol.6, N°17, 1305-1307. La réaction s'effectue en faisant réagir un dérivé acétamide R⁹-NH₂ et le composé sulfoxyde de formule générale **(I.2)** en présence d'un agent d'oxydation comme par exemple l'oxyde de magnésium, à une température comprise entre 20°C et 30°C (de préférence 25°C), dans un solvant inerte tel que par exemple le tetrahydrofurane ou un solvant chloré et en particulier le dichlorométhane et pour une durée de plusieurs heures. Le dérivé de formule générale **(I.3)-ter** est ensuite mis en **réaction** à une température comprise entre 20°C et 30°C (de préférence 25°C), en présence d'une base minérale et en particulier du carbonate de potassium dans un solvant inerte tel que par exemple le tetrahydrofurane ou un solvant alcooylé et en particulier le méthanol et pour une durée de plusieurs heures pour obtenir le composé de formule **(I.3).**

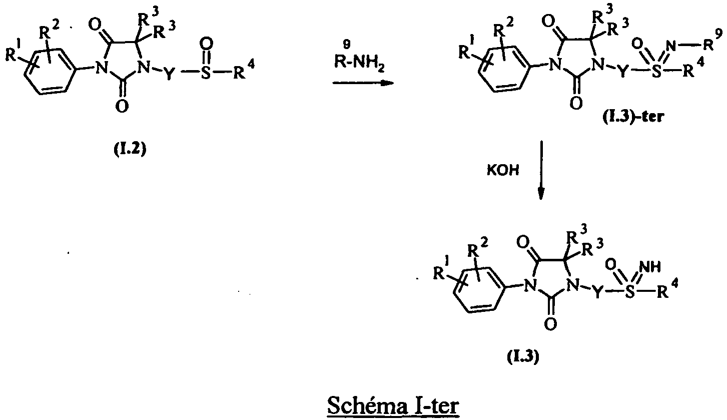

### A) Préparation des composés de formule générale (I.1) dans lesquels R¹ est un groupe amino éventuellement substitué :

### A.1) Préparation des dérivés anilines :

Dans le cas particulier où R¹ est un groupe nitro, R², R³ et R⁴ étant tels que décrits ci-dessus, la préparation des dérivés aniline de formule générale **(I.1)_{A1}** est représentée dans le schéma A1. La réduction des groupements nitro des composés de formule générale **(I.1)** est effectuée à l'aide de SnCl₂, 2H₂I (J. Heterocyclic Chem. 1987, 24, 927-930 ; Tetrahedron Letters 1984, 25 (8), 839-842) par chauffage du mélange réactionnel à reflux, dans un solvant tel que l'acétate d'éthyle, pendant plusieurs heures.

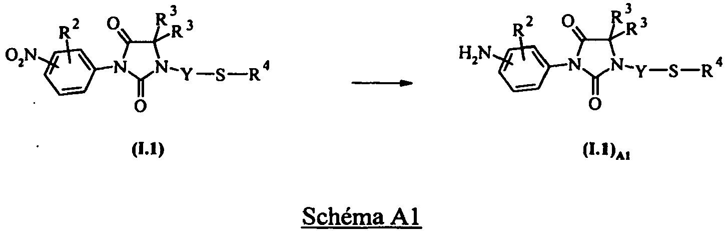

### A.2) Préparation des dérivés acétamides :

Les dérivés acétamides de formule générale **(I.1)_{A2}** dans lesquels R², R³, R⁴ et R⁵ sont tels que décrits ci-dessus, sont accessibles en une étape à partir des dérivés anilines de formule générale **(I.1)_{A1},** schéma A2. La réaction d'acylation peut être effectuée à l'aide d'un excès de chlorure d'acide de formule générale R⁵-COCl, par exemple le chlorure d'acétyle, ou bien d'un anhydride de type (R⁵-CO)₂O, tel que l'anhydride acétique. La réaction est effectuée généralement vers 20° C, dans un solvant anhydre tel que par exemple le dichlorométhane, et pour une durée de quelques heures.

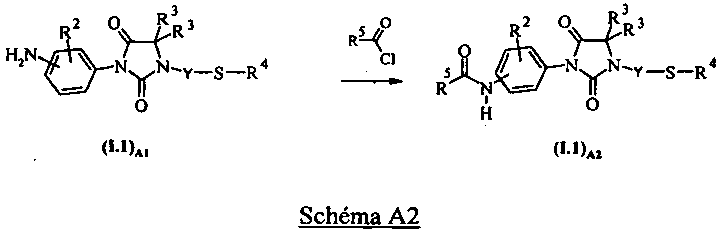

La synthèse des intermédiaires de formule générale **(I.1)A2bis,** schéma A2bis, dans lesquels R², R³, R⁴, R⁵, R⁸ et Y sont tels que décrits ci-dessus est effectuée par substitution nucléophile d'un atome d'halogène du réactif halogéné de formule générale R⁸-Hal, par l'anion de l'acétamide de formule générale **(I.1)A2** généré en présence d'une base telle que, par exemple NaH, dans un solvant polaire aprotique tel que le DMF ou le DMSO. La réaction s'effectue généralement à une température variant de 20 à 30° C et pour une durée de 1 à 5 heures.

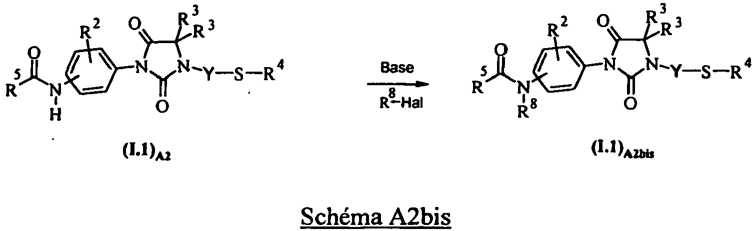

### A.3) Préparation des dérivés urée :

Les dérivés d'urée de formule générale **(I.1)_{A3}** dans lesquels R², R³, R⁴ et R⁶ sont tels que décrits ci-dessus, sont préparés en une étape à partir des dérivés anilines de formule générale **(I.1)_{A1},** schéma A3. La réaction s'effectue en présence d'un dérivé d'isocyanate R⁶-N=C=O, par chauffage du mélange réactionnel à une température comprise entre 70 et 120° C, généralement dans un solvant inerte tel que, par exemple, un solvant chloré et en particulier le 1,2-dichloroéthane et pour une durée de plusieurs heures.

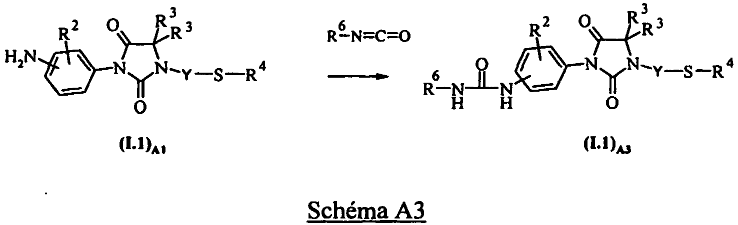

### A.4) Préparation des dérivés sulfamides :

Les dérivés sulfamides de formule générale **(I.1)_{A3}** dans lesquels R², R³, R⁴ et R⁶ sont tels que décrits ci-dessus, sont préparés à partir des dérivés anilines de formule générale **(I.1)_{A1},** schéma A4. La réaction est effectuée dans les conditions expérimentales analogues à celles décrites dans WO2007125197.

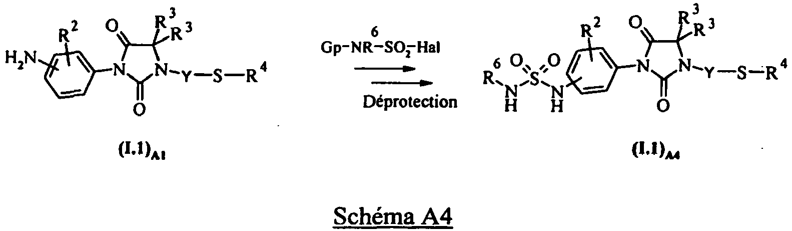

### A.5) Préparation des dérivés sulfonamides :

Les dérivés sulfonamides de formule générale **(I.1)_{A5}** dans lesquels R², R³, R⁴, R⁵ et Y sont tels que décrits ci-dessus, sont préparés à partir des dérivés anilines de formule générale **(I.1)_{A1},** schéma A5. La réaction est effectuée dans les conditions expérimentales analogues à celles décrites par G.H. Chu et collaborateurs dans Bioorg. Med. Chem. Lett. 2007, 17, 1951-1955. La réaction s'effectue en faisant réagir un dérivé chlorure de sulfonyl R⁵-SO₂-Cl et le composé aniline de formule générale **(I.1)_{A1}** dans lequel R², R³, R⁴, ont été définis précédemment, à une température comprise entre 20°C et 30°C (de préférence 25°C), dans un solvant inerte tel que par exemple le tetrahydrofurane, un solvant chloré et en particulier le 1,2-dichloroéthane et pour une durée de plusieurs heures. Le dérivé disulfonamide de formule générale **(I.1)A5-1** est ensuite mis en réaction à une température comprise entre 20°C et 30°C (de préférence 25°C), en présence d'une base minérale et en particulier l'hydroxyde de lithium dans un solvant inerte tel que par exemple le tetrahydrofurane, un solvant alcooylé et en particulier le méthanol et pour une durée de plusieurs heures.

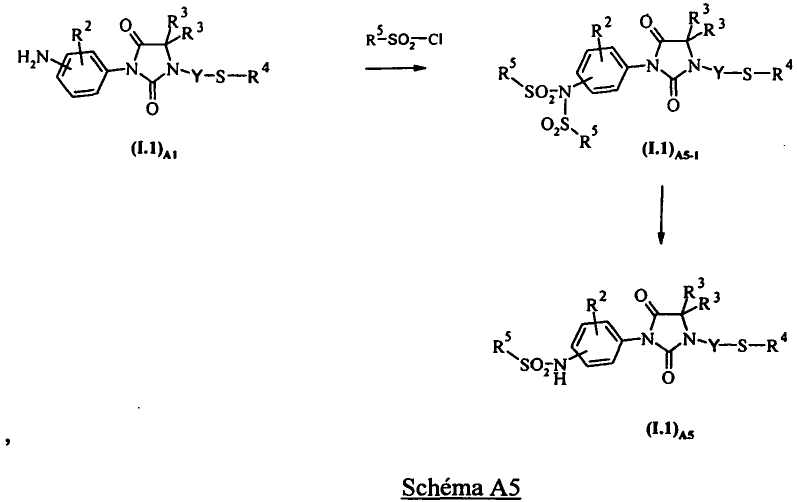

### B) Synthèses pour préparer les composés de formule générale (I.1) dans lesquels R¹ est un groupe carboxamide :

### B.1) Préparation des dérivés carboxamides :

Les dérivés carboxamides de formule générale **(I.1)_{B1}** dans lesquels R², R³ et R⁴ sont tels que décrits ci-dessus, sont préparés en une étape à partir des dérivés nitriles de formule générale **(I.1)**, schéma B1. La réaction d'hydrolyse est effectuée dans un mélange d'acide trifluoroacétique et d'acide sulfurique, suivi d'un traitement en milieu aqueux (J. Org. Chem. 2005, 70 (5), 1926-1929).

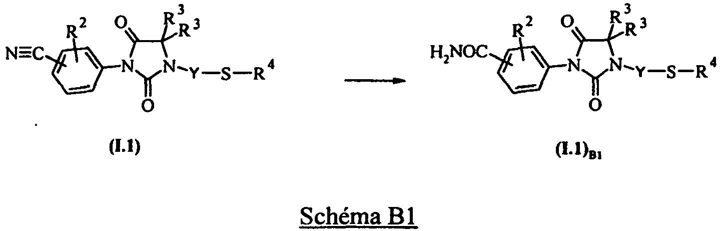

### C) Préparation des intermédiaires de formule générale (II) :

La synthèse des arylhydantoïnes intermédiaires de formule générale **(II)** dans lesquels R¹, R² et R³ sont tels que décrits ci-dessus peuvent être effectuées selon les stratégies décrites dans les schémas ci-dessous :

### C.1) Préparation des arylhydantoïnes par condensation :

La synthèse de l'arylhydantoïne de formule générale **(II),** schéma B1, peut être effectuée par substitution nucléophile de l'atome de fluor porté par le noyau aromatique du composé de formule générale **(II)₁** par l'anion de l'hydantoine de formule générale **(II)₂** généré en présence d'une base telle que, par exemple K₂CO₃. La réaction s'effectue par chauffage à une température comprise entre 65 et 140° C et dans un solvant polaire aprotique tel que, par exemple, le DMF ou le DMSO. La température et le temps de réaction sont fonction du caractère nucléofuge de l'atome de fluor qui est fortement dépendant de la nature de R¹ et R². Les hydantoïnes de formule générale **(II)₂** non commerciales peuvent être préparées selon des méthodes décrites dans la littérature (p. ex. J. Med. Chem. 1984, 27 (12), 1663-8). Les hydantoïnes de formule générale **(II)₂** non commerciales peuvent être préparées selon des méthodes décrites dans la littérature (p. ex. J. Med. Chem. 1984, 27 (12), 1663-8).

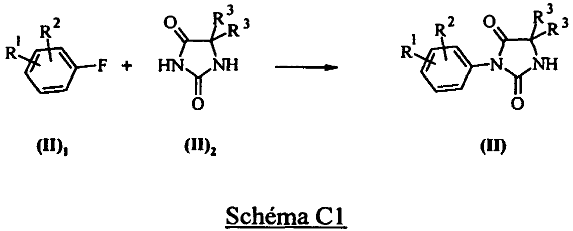

Dans les cas où R¹ et R² ne sont pas suffisamment électro-attracteurs pour favoriser la substitution nucléophile aromatique décrite dans le schéma C1, une approche par couplage entre un acide aryl boronique et l'hydantoine de formule générale **(II)₂** en présence d'acétate de cuivre peut être envisagée (Synlett 2006, 14, 2290-2) pour obtenir les composés de formule générale **(II).**

### C.2) Préparation des arylhydantoïnes par construction du cycle hydantoïne à partir d'arylisocyanate :

L'accès aux hydantoines de formule générale **(II)** se fait, dans ce cas, selon un protocole décrit dans Bioorg. Med. Chem. Lett. 2004, 14, 5285.

### C.3) Préparation des arylhydantoïnes par construction du cycle hydantoïne à partir des isocyanates d'aminoesters :

Alternativement, les arylhydantoïnes de formule générale **(II)** peuvent être synthétisées à partir d'isocyanate d'aminoesters tel que décrit dans Eur. J Med. Chem. 1984, 19 (3), 261.

### D) Préparation des intermédiaires de formule générale (IV) :

La synthèse des intermédiaires de formule générale **(IV),** schéma D1, dans lesquels R¹, R¹, R³ et Y sont tels que décrits ci-dessus est effectuée par substitution nucléophile d'un atome d'halogène du réactif dihalogéné de formule générale Hal-(CH₂)ₙ-Hal, par l'anion de l'hydantoine de formule générale **(II)** généré en présence d'une base telle que, par exemple NaH, dans un solvant polaire aprotique tel que le DMF ou le DMSO. La réaction s'effectue généralement à une température variant de 20 à 60° C et pour une durée de 1 à 15 heures.

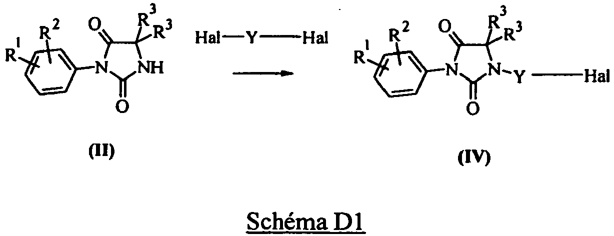

Ainsi, l'invention a également pour objet un procédé de préparation d'un composé de formule **(I)** tel que défini ci-dessus, procédé comprenant l'obtention de composés de formule générale **(I.1)** (composé de formule générale **(I),** dans laquelle X représente l'atome de soufre), 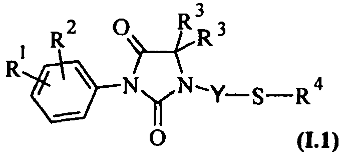 dans laquelle R¹, R², R³, R⁴ et Y sont tels que définis précédemment,
soit par condensation des dérivés d'hydantoine de formule générale **(II),** 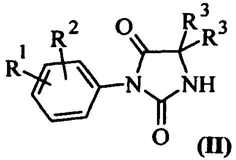 dans laquelle R¹, R², et R³ sont tels que définis précédemment, en présence d'une base forte à une température comprise entre 25 et 60° C, dans un solvant polaire anhydre, sur les dérivés mésylates de formule générale **(III)**, 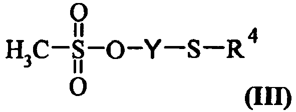 dans laquelle R⁴ et Y sont tels que définis précédemment,
soit par le traitement de dérivés thiobenzoyls de formule générale **(V)**, 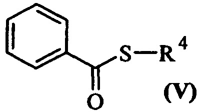 dans laquelle R⁴ est tel que défini précédemment, par un alcoolate dans un solvant protique polaire suivi de l'addition du dérivé halogéné de formule générale **(IV)**, 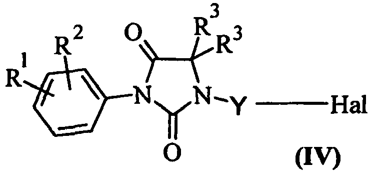 dans laquelle R¹, R², R³ et Y sont tels que définis précédemment, en solution dans un solvant polaire ;
composés de formule générale **(I.1)** dont le groupe thioéther est oxydé en sulfoxyde de formule générale **(I.2)** (composé de formule générale **(I)** dans laquelle X représente le radical -SO-), 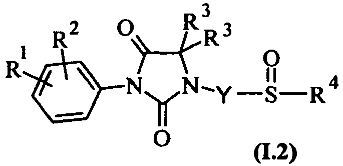 dans laquelle R¹, R², R³, R⁴ et Y sont tels que définis précédemment,
dérivés sulfoxydes de formule générale **(I.2)** qui sont oxydés en sulfones de formule générale **(I.3)** (composé de formule générale **(I)** dans laquelle X représente le groupe -SO₂-), 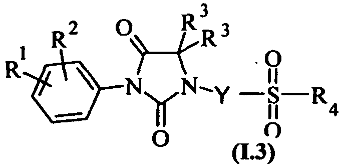 dans laquelle R¹, R², R³, R⁴ et Y sont tels que définis précédemment.

De manière préférentielle, les oxydations mentionnées ci-dessus ont lieu en présence d'Oxone®.

La salification des composés de formule (I) peut être effectuée par toute méthode connue de l'homme du métier. Par exemple, la salification peut être réalisée par ajout de base ou d'acide, par exemple, d'hydroxyde de sodium, de potassium, ou d'acide chlorhydrique.

Selon une variante, l'objet de l'invention est l'un des composés suivants, à titre d'intermédiaire :
- 1-(5-iodopentyl)-5,5-dimethyl-3-[4-nitro-3-(trifluoromethyl)phenyl]imidazolidine-2,4-dione 1-(8-iodooctyl)-5,5-dimethyl-3-[4-nitro-3-(trifluoromethyl)phenyl]imidazolidine-2,4-dione

- 1-(9-bromononyl)-5,5-dimethyl-3-[4-nitro-3-(trifluoromethyl)phenyl]imidazolidine-2,4-dione
- 4-(4,4-dimethyl-2,5-dioxoimidazolidin-1-yl)-2-(trifluoromethyl)benzonitrile
- 4-[3-(9-bromononyl)-4,4-dimethyl-2,5-dioxoimidazolidin-1-yl]-2-(trifluoromethyl)benzonitrile
- 5,5-dimethyl-3-(3-methyl-4-nitrophenyl)imidazolidine-2,4-dione
- 1-(9-bromononyl)-5,5-dimethyl-3-(3-methyl-4-nitrophenyl)imidazolidine-2,4-dione.

Les composés de formule (I) selon la présente invention possèdent d'intéressantes propriétés pharmacologiques. En effet, on a découvert que les composés de formule **(I)** de la présente invention possèdent une activité anti-tumorale (anti-cancéreuse), et plus particulièrement une activité inhibitrice de la prolifération cellulaire des cellules exprimant des récepteurs aux androgènes telles que les cellules prostatiques de type LnCAP. Ainsi, les composés de la présente invention peuvent être utilisés dans différentes applications thérapeutiques. Ils peuvent avantageusement être utilisés pour le traitement des cancers, particulièrement des cancers hormonodépendants, des cancers exprimant des récepteurs aux androgènes et plus particulièrement des cancers du sein et de la prostate. On trouvera ci-après, dans la partie expérimentale, une illustration des propriétés pharmacologiques des composés de l'invention.

La présente demande a donc également pour objet, les composé de formule **(I)** tels que définis précédemment en tant que médicaments.

Également, la présente demande a pour objet les composé de formule **(I)** tels que définis précédemment en tant que médicaments destinés à traiter les maladies prolifératives, préférentiellement les cancers, très préférentiellement les cancers hormonodépendants ou bien les cancers exprimant des récepteurs aux androgènes, ou encore les cancers de la prostate et du sein et de manière très préférentielle les cancers de la prostate.

La présente demande a également pour objet, des compositions pharmaceutiques contenant, à titre de principe actif, au moins un composé de formule **(I)** tel que défini ci-dessus, en association avec un support pharmaceutiquement acceptable.

La présente demande a également pour objet l'utilisation d'un composé de formule **(I)** selon la présente invention, pour la préparation d'un médicament anti-tumoral.

La présente demande a également pour objet l'utilisation d'un composé de formule **(I)** selon la présente invention, pour la préparation d'un médicament destiné à inhiber la prolifération cellulaire.

La présente demande a également pour objet l'utilisation d'un composé de formule **(I)** selon la présente invention, pour la préparation d'un médicament destiné à traiter les maladies prolifératives, préférentiellement les cancers, très préférentiellement les cancers hormono-dépendants ou les cancers exprimant des récepteurs aux androgènes, ou les cancers de la prostate et du sein et de manière très préférentielle les cancers de la prostate.

La composition pharmaceutique peut être sous forme d'un solide, par exemple, des poudres, des granules, des comprimés, des gélules. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau, additionnés à des huiles ou des graisses pharmaceutiquement acceptables. Les compositions liquides stériles peuvent être utilisées pour les injections intramusculaires, intrapéritonéales ou sous-cutanées et les compositions stériles peuvent également être administrées par voie intraveineuse.

Tous les termes techniques et scientifiques utilisés dans le présent texte ont la signification connue de l'homme de l'art. Par ailleurs, tous les brevets (ou demandes de brevet) ainsi que les autres références bibliographiques sont incorporés par référence.

### Partie expérimentale

Suivant les définitions des variables R¹, R², R³, X, n et R⁴, les composés selon l'invention peuvent être préparés selon les différentes méthodes décrites ci-dessus.

Les exemples sont présentés pour illustrer les procédures ci-dessus et ne doivent en aucun cas être considérés comme une limite à la portée de l'invention.

### Exemple 1 : 5,5-diméthyl-3-[4-nitro-3-(trifluorométhyl)phényl]-1-{9-[(4,4,5,5,5-pentafluoropentyl)thio]nonyl}imidazolidine-2,4-dione.

Sous argon, on ajoute NaH (à 60 %) (44 mg, 1,1 mmole) à une solution de 5,5-diméthyl-3-[4-nitro-3-(trifluorométhyl)phényl]-imidazolidine-2,4-dione (317 mg, 1 mmole) dans du DMF anhydre (8 ml). Un dégagement gazeux accompagne le changement de coloration du milieu réactionnel qui devient orange. L'agitation est maintenue 1 heure à 23° C avant d'ajouter le 9-[(4,4,5,5,5-pentafluoropentyl)-thio]nonyl méthanesulfonate (préparé selon un protocole expérimental décrit dans WO 2005077968) (332 mg, 0,8 mmole). Après 15 heures de réaction, le milieu réactionnel est versé dans de l'eau (25 ml) et extrait par AcOEt (2 x 25 ml). Les phases organiques sont rassemblées et lavées successivement par de l'eau (25 ml) et de la saumure (25 ml). Après séchage sur Na₂SO₄, la solution organique est filtrée et concentrée sous vide. Le résidu d'évaporation est purifié sur une colonne de silice (éluant : CH₂Cl₂/AcOEt : 1/1 jusqu'à 0/1). Le composé attendu est obtenu sous forme d'une huile jaune pâle avec un rendement de 72 % (364 mg).
¹H NMR 400MHz (DMSO-*d*₆) δ : 8,32 (d, 1H, Ph) ; 8,20 (d, 1H, Ph) ; 8,07 (dd, 1H, Ph) ; 3,29 (m, 2H, NCH₂) ; 2,57 (m, 2H, SCH₂) ; 2,48 (m, 2H, SCH₂) ; 2,28 (m, 2H, CH₂) ; 1,76 (m, 2H, CH₂) ; 1,61 (m, 2H, CH₂) ; 1,50 (m, 2H, CH₂) ; 1,46 (s, 6H, 2 x CH₃) ; 1,30 (m, 10H, 5 x CH₂).

### Exemple 2 : 5,5-diméthyl-3-[4-nitro-3-(trifluorométhyl)phényl]-1-{9-[(4,4,5,5,5-pentafluoropentyl)sulfinyl]nonyl}imidazolidine-2,4-dione

A une solution du composé décrit dans l'exemple 1 (364 mg, 0,57 mmole) dans le THF (75 ml) on ajoute de l'Oxone® (211 mg, 0,34 mmole) et de l'eau (10 ml). Le mélange réactionnel est agité pendant 20 minutes, temps nécessaire à la conversion complète du produit de départ, et versé ensuite dans de l'eau (100 ml). Le composé est extrait par AcOEt (2 x 75 ml), les phases organiques sont rassemblées et lavées par de la saumure. Après séchage sur Na₂SO₄ la solution organique est filtrée et concentrée sous vide. Le résidu d'évaporation est purifié sur une colonne de silice (éluant : CH₂Cl₂/AcOEt : 1/1 jusqu'à 1/4). Le composé attendu est obtenu sous forme d'une huile incolore avec un rendement de 86 % (321 mg).
¹H NMR 400MHz (DMSO-*d*₆) δ : 8,42 (d, 1H, Ph) ; 8,30 (d, 1H, Ph) ; 8,17 (dd, 1, Ph) ; 3,39 (ni, 2H, NCH₂) ; 2,88 (m, 4H, CH₂S(=O)CH₂) ; 2,48 (m, 2H, CH₂) ; 2,02 (m, 2H, CH₂) ; 1,72 (m, 4H, 2 x CH₂) ; 1,56 (s, 6H, 2 x CH₃) ; 1,51 (m, 2H, CH₂) ; 1,42 (m, 8H, 4 x CH₂).

### Exemple 3 : 5,5-diméthyl-3-[4-nitro-3-(trifluorométhyl)phényl]-1-{9-[(4,4,5,5,5-pentafluoropentyl)sulfonyl]nonyl}imidazolidine-2,4-dione

On ajoute de l'Oxone® (566 mg, 0,93 mmole) à une solution du composé de l'exemple 2 (200 mg, 0,31 mmole) dans le THF (6 ml). Le mélange réactionnel est complété par de l'eau (3 ml) et agité à 23° C pendant 3 h 30, temps nécessaire à la conversion totale du produit de départ. L'ensemble est ensuite versé dans de l'eau (25 ml) et extrait par AcOEt (2 x 25 ml). Après décantation, les phases organiques sont rassemblées et lavées par de la saumure (20 ml), séchées sur Na₂SO₄, filtrées et concentrées à sec sous vide. Le résidu d'évaporation est purifié sur une colonne de silice (éluant : Heptane/AcOEt : 1/1 jusqu'à 3/7). Le composé attendu est obtenu sous forme d'une huile incolore avec un rendement de 89 % (185 mg).
¹H NMR 400MHz (DMSO-*d*₆) δ : 8,28 (d, 1H, Ph) ; 8,15 (d, 1H, Ph) ; 8,04 (dd, 1H, Ph); 3,24 (m, 2H, NCH₂); 3,15 (m, 2H, CH₂S(=O)₂); 3,05 (m, 2H, CH₂S(=O)₂) ; 2,32 (m, 2H, CH₂) ; 1,87 (m, 2H, CH₂) ; 1,59 (m, 4H, 2 x CH₂) ; 1,41 (s, 6H, 2 x CH₃) ; 1,35 (m, 2H, CH₂) ; 1,24 (m, 8H, 4 x CH₂).

### Exemple 4 : 5,5-diméthyl-3-[4-nitro-3-(trifluorométhyl)phényl]-1-{5-[(4,4,5,5,5-pentafluoropentyl)thio]pentyl}imidazolidine-2,4-dione

### 4.1) 1-(5-iodopentyl)-5,5-diméthyl-3-[4-nitro-3-(trifluorométhyl)phényl]imidazolidine-2,4-dione

Sous argon, on ajoute NaH (à 60 %) (65 mg, 1,6 mmole) à une solution de de 5,5-diméthyl-3-[4-nitro-3-(trifluorométhyl)phényl]-imidazolidine-2,4-dione (500 mg, 1,6 mmole) dans du DMF anhydre (20 ml). Un dégagement gazeux accompagne le changement de coloration du milieu réactionnel qui devient orange. L'agitation est maintenue 1 heure à 23° C avant d'ajouter, sans dilution, le 1,5-diiodopentane (350 µl, 2,4 mmoles). Après 15 heures de réaction, le milieu réactionnel est versé dans une solution aqueuse saturée de NH₄Cl (25 ml) et extrait par AcOEt (2 x 25 ml). Les phases organiques sont réunies et lavées successivement par de l'eau (25 ml) et de la saumure (25 ml). Après séchage sur Na₂SO₄, la solution organique est filtrée et concentrée sous vide. Le résidu d'évaporation est purifié sur une colonne de silice (éluant : Heptane/AcOEt : 7/3 jusqu'à 6/4). Le composé attendu est obtenu sous forme d'une huile incolore avec un rendement de 47 % (380 mg).
¹H NMR 400MHz (DMSO-*d₆*) δ : 8,32 (d, 1H, Ph) ; 8,20 (d, 1H, Ph) ; 8,07 (dd, 1H, Ph) ; 3,29 (m, 4H, 2 x CH₂) ; 1,81 (m, 2H, CH₂) ; 1,65 (m, 2H, CH₂) ; 1,47 (s, 6H, 2 x CH₃) ; 1,41 (m, 2H, CH₂).

### 4.2) 5,5-diméthyl-3-[4-nitro-3-(trifluorométhyl)phényl]-1-[5-[(4,4,5,5,5-pentafluoropentyl)thio]pentyl}imidazolidine-2,4-dione

Sous argon, à une solution de S-(4,4,5,5,5-pentafluoropentyl) benzene-carbothioate (préparé selon un protocole expérimental décrit dans WO 2005077968) (221 mg, 0,74 mmole) dans MeOH (10 ml) on ajoute tBuO⁻KC⁺ (124 mg, 1,11 mmole) et l'agitation est maintenue 30 minutes à 23° C. L'intermédiaire 4.1 (380 mg, 0,74 mmole) en solution dans MeOH (10 ml) est ensuite ajouté en une portion. Après 24 heures d'agitation, le mélange réactionnel est versé dans de l'eau (25 ml) et extrait par AcOEt (2 x 25 ml). Après décantation, les phases organiques sont réunies et lavées par de l'eau (20 ml) et de la saumure (20 ml), séchées sur Na₂SO₄, filtrées et concentrées à sec sous vide. Le résidu d'évaporation est purifié sur une colonne de silice (éluant : Heptane/AcOEt : 7/3 jusqu'à 6/4). Le composé attendu est obtenu sous forme d'une huile incolore avec un rendement de 75 % (320 mg).
¹H NMR 400MHz (DMSO-d₆) δ : 8,32 (d, 1H, Ph) ; 8,20 (d, 1H, Ph) ; 8,07 (dd, 1H, Ph) ; 3,30 (m, 2H, NCH₂) ; 2,59 (m, 2H, SCH₂) ; 2,50 (m, 2H, SCH₂) ; 2,29 (m, 2H, CH₂) ; 1,76 (m, 2H, CH₂); 1,63 (m, 2H, CH₂) ; 1,55 (m, 2H, CH₂) ; 1,46 (s, 6H, 2 x CH₃) ; 1,41 (m, 2H, CH₂).

### Exemple 5 : 5,5-diméthyl-3-[4-nitro-3-(trifluorométhyl)phényl]-1-{5-[(4,4,5,5,5-pentafluoropentyl)sulfinyl]pentyl}imidazolidine-2,4-dione

Le protocole expérimental utilisé est le même que celui décrit pour la synthèse du composé de l'exemple 2, le composé de l'exemple 4 remplaçant le composé de l'exemple 1. On obtient 136 mg d'un solide blanc (73 %). Point de fusion : 95-96° C.
¹H NMR 400MHz (DMSO-*d₆*) δ : 8,32 (d, 1H, Ph) ; 8,20 (d, 1H, Ph) ; 8,07 (dd, 1H, Ph) ; 3,33 (m, 2H, NCH₂) ; 2,80 (m, 2H, S(=O)CH₂) ; 2,76 (m, 2H, S(=O)CH₂) ; 2,37 (m, 2H, CH₂) ; 1,92 (m, 2H, CH₂) ; 1,67 (m, 4H, 2 x CH₂) ; 1,47 (m, 8H, (2 x CH₃) + CH₂).

### Exemple 6 : 5,5-diméthyl-3-[4-nitro-3-(trifluorométhyl)phényl]-1-{8-[(4,4,5,5,5-pentafluoropentyl)thio]octyl}imidazolidine-2,4-dione

### 6.1) 1-(8-iodooctyl)-5,5-diméthyl-3-[4-nitro-3-(trifluorométhyl)phényl]imidazolidine-2,4-dione

Le protocole expérimental utilisé est le même que celui décrit pour la synthèse de l'intermédiaire 4.1, le 1,8-diiodooctane remplaçant le 1,5-diiodopentane. Le composé attendu est obtenu sous forme d'une huile jaune pâle avec un rendement de 44 % (229 mg).
¹H NMR 400MHz (DMSO-*d₆*) δ : 8,32 (d, 1H, Ph) ; 8,20 (d, 1H, Ph) ; 8,07 (dd, 1H, Ph) ; 3,28 (m, 4H, CH₂I + NCH₂) ; 1,73 (m, 2H, CH₂) ; 1,63 (m, 2H, CH₂) ; 1,46 (s, 6H, 2 x CH₃) ; 1,30 (m, 8H, 4 x CH₂).

### 6.2) 5,5-diméthyl-3-[4-nitro-3-(trifluorométhyl)phényl]-1-{8-[(4,4,5,5,5-pentafluoropentyl)thio]octyl}imidazolidine-2,4-dione

Le protocole expérimental utilisé est le même que celui décrit pour la synthèse du composé de l'exemple 4, l'intermédiaire 6.1 remplaçant l'intermédiaire 4.1. On obtient une huile jaune pâle avec un rendement de 78 %.
¹H NMR 400MHz (DMSO-*d₆*) δ : 8,32 (d, 1H, Ph) ; 8,20 (d, 1H, Ph) ; 8,07 (dd, 1H, Ph) ; 3,28 (m, 2H, NCH₂) ; 2,58 (m, 2H, SCH₂) ; 2,47 (m, 2H, SCH₂) ; 2,27 (m, 2H, CH₂) ; 1,76 (m, 2H, CH₂) ; 1,62 (m, 2H, CH₂) ; 1,51 (m, 2H, CH₂) ; 1,46 (s, 6H, 2 x CH₃) ; 1,30 (m, 8H, 4 x CH₂).

### Exemple 7 5,5-diméthyl-3-[4-nitro-3-(trifluorométhyl)phényl]-1-{8-[(4,4,5,5,5-pentafluoropentyl)sulfnyl]octyl}imidazolidine-2,4-dione

Le protocole expérimental utilisé est le même que celui décrit pour la synthèse du composé de l'exemple 2, l'exemple 6 remplaçant le composé de l'exemple 1. On obtient 61 mg d'une huile jaune pâle (64 %).
¹H NMR 400MHz (DMSO-*d₆*) δ : 8,32 (d, 1H, Ph) ; 8,20 (d, 1H, Ph) ; 8,07 (dd, 1H, Ph) ; 3,28 (m, 2H, NCH₂) ; 2,74 (m, 4H, CH₂S(=O)CH₂) ; 2,37 (m, 2H, CH₂) ; 1,90 (m, 2H, CH₂) ; 1,63 (m, 4H, 2 x CH₂) ; 1,46 (s, 6H, 2 x CH₃) ; 1,40 (m, 2H, CH₂) ; 1,32 (m, 6H, 3 x CH₂).

### Exemple 8 : 5,5-diméthyl-3-[4-nitro-3-(trifluorométhyl)phényl]-1-{10-[(4,4,5,5,5-pentafluoropentyl)thio]decyl}imidazolidine-2,4-dione

Le protocole expérimental utilisé est le même que celui décrit pour la synthèse du composé de l'exemple 1, le 10-[(4,4,5,5,5-pentafluoropentyl)thio]decyl méthanesulfonate (préparé selon un protocole expérimental analogue à celui décrit dans WO 2005077968) remplaçant le 9-[(4,4,5,5,5-pentafluoropentyl)-thio]nonyl méthanesulfonate. Le composé attendu est obtenu sous forme d'une huile jaune pâle avec un rendement de 13 % (125 mg).
¹H NMR 400MHz (DMSO-*d₆*) δ : 8,32 (d, 1H, Ph) ; 8,20 (d, 1H, Ph) ; 8,07 (dd, 1H, Ph) ; 3,29 (m, 2H, NCH₂) ; 2,57 (m, 2H, SCH₂) ; 2,47 (m, 2H, SCH₂) ; 2,27 (m, 2H, CH₂) ; 1,75 (m, 2H, CH₂) ; 1,61 (m, 2H, CH₂) ; 1,50 (m, 2H, CH₂) ; 1,46 (s, 6H, 2 x CH₃) ; 1,29 (m, 12H, 6 x CH₂).

### Exemple 9 : 5,5-diméthyl-3-[4-nitro-3-(trifluorométhyl)phényl]-1-{10-[(4,4,5,5,5-pentafluoropentyl)sulfinyl]décyl}imidazolidine-2,4-dione

Le protocole expérimental utilisé est le même que celui décrit pour la synthèse du composé de l'exemple 2, le composé de l'exemple 8 remplaçant le composé de l'exemple 1. Le composé attendu est obtenu sous forme d'huile jaune pâle avec un rendement de 74 % (71 mg).
¹H NMR 400MHz (DMSO-*d₆*) δ : 8,32 (d, 1H, Ph) ; 8,20 (d, 1H, Ph) ; 8,07 (dd, 1H, Ph) ; 3,28 (m, 2H, NCH₂) ; 2,80 (m, 2H, S(=O)CH₂) ; 2,71 (m, 2H, S(=O)CH₂) ; 2,37 (m, 2H, CH₂) ; 1,92 (m, 2H, CH₂) ; 1,60 (m, 4H, 2 x CH₂) ; 1,46 (s, 6H, 2 x CH₃) ; 1,33 (m, 12H, 6 x CH₂).

### Exemple 10 : 5,5-diméthyl-3-[4-nitro-3-(trifluorométhyl)phényl]-1-{11-[(4,4,5,5,5-pentafluoropentyl)thio]undecyl}imidazolidine-2,4-dione

Le protocole expérimental utilisé est le même que celui décrit pour la synthèse du composé de l'exemple 1, le 10-[(4,4,5,5,5-pentafluoropentyl)thio]undécyl méthanesulfonate (préparé selon un protocole expérimental analogue à celui décrit dans WO 2005077968) remplaçant le 9-[(4,4,5,5,5-pentafluoropentyl)-thio]nonyl méthanesulfonate. Le composé attendu est obtenu sous forme d'une huile jaune pâle avec un rendement de 43 % (328 mg).
¹H NMR 400MHz (DMSO-*d₆*) δ : 8,42 (d, 1H, Ph) ; 8,30 (d, 1H, Ph) ; 8,18 (dd, 1H, Ph) ; 3,38 (m, 2H, NCH₂) ; 2,67 (m, 2H, SCH₂) ; 2,57 (m, 2H, SCH₂) ; 2,37 (m, 2H, CH₂) ; 1,85 (m, 2H, CH₂) ; 1,71 (m, 2H, CH₂) ; 1,58 (m, 2H, CH₂) ; 1,56 (s, 6H, 2 x CH₃) ; 1,40 (m, 14H, 7 x CH₂).

### Exemple 11 : 5,5-diméthyl-3-[4-nitro-3-(trifluorométhyl)phényl]-1-{11-[(4,4,5,5,5-pentafluoropentyl)sulfinyl]undécyl}imidazolidine-2,4-dione

Le protocole expérimental utilisé est le même que celui décrit pour la synthèse du composé de l'exemple 2, l'exemple 10 remplaçant le composé de l'exemple 1. Le composé attendu est obtenu sous forme d'une huile jaune pâle avec un rendement de 72 % (240 mg).
¹H NMR 400MHz (DMSO-*d₆*) δ : 8,32 (d, 1H, Ph) ; 8,20 (d, 1H, Ph) ; 8,07 (dd, 1H, Ph) ; 3,28 (m, 2H, NCH₂) ; 2,75 (m, 4H, CH₂S(=O)CH₂) ; 2,40 (m, 2H, CH₂) ; 1,90 (m, 2H, CH₂) ; 1,60 (m, 4H, 2 x CH₂) ; 1,46 (s, 6H, 2 x CH₃) ; 1,39 (m, 2H, CH₂) ; 1,30 (m, 12H, 6 x CH₂).

### Exemple 12 : 5,5-diméthyl-3-[4-nitro-3-(trifluorométhyl)phényl)-1-{9-[(4,4,4-trifluorobutyl)thio]nonyl}imidazolidine-2,4-dione

### 12.1) 1-(9-bromononyl)-5,5-diméthyl-3-[4-nitro-3-(trifluorométhyl)phényl]-imidazolidine-2,4-dione

Le protocole expérimental utilisé est le même que celui décrit pour la synthèse de l'intermédiaire 4.1, le 1,9-dibromononane remplaçant le 1,5-diiodopentane. Le composé attendu est obtenu sous forme d'une huile jaune pâle avec un rendement de 45 % (1,24 g).
¹H NMR 400MHz (DMSO-*d₆*) δ : 8,32 (d, 1H, Ph) ; 8,20 (d, 1H, Ph) ; 8,07 (dd, 1H, Ph) ; 3,51 (m, 2H, CH₂Br) ; 3,29 (m, 2H, NCH₂) ; 1,78 (m, 2H, CH₂) ; 1,62 (m, 2H, CH₂) ; 1,46 (s, 6H, 2 x CH₃) ; 1,32 (m, 10H, 5 x CH₂).

### 12.2) 5,5-diméthyl-3-[4-nitro-3-(trifluorométhyl)phényl]-1-{9-[(4,4,4-trifluorobutyl)thio]nonyl}imidazolidine-2,4-dione

Le protocole expérimental utilisé est le même que celui décrit pour la synthèse de l'exemple 4, l'intermédiaire 12.1 remplaçant l'intermédiaire 4.1 et le S-(4,4,4-trifluorobutyl) benzenecarbothioate (préparé selon WO 2005077968) remplaçant l'intermédiaire S-(4,4,5,5,5-pentafluoropentyl) benzene-carbothioate. Le composé attendu est obtenu sous forme d'une huile jaune pâle avec un rendement de 83% (231 mg).
¹H NMR 400MHz (DMSO-*d₆*) δ : 8,42 (d, 1H, Ph) ; 8,30 (d, 1H, Ph) ; 8,18 (dd, 1H, Ph) ; 3,38 (m, 2H, NCH₂); 2,60 (m, 4H, CH₂SCH₂) ; 2,42 (m, 2H, CH₂) ; 1,82 (m, 2H, CH₂) ; 1,71 (m, 2H, CH₂) ; 1,61 (m, 2H, CH₂) ; 1,46 (s, 6H, 2 x CH₃) ; 1,32 (m, 10H, 5 x CH₂).

### Exemple 13 : 5,5-diméthyl-3-[4-nitro-3-(trifluorométhyl)phényl]-1-{9-[(4,4,4-trifluorobutyl)sulfinyl]nonyl}imidazolidine-2,4-dione

Le protocole expérimental utilisé est le même que celui décrit pour la synthèse du composé de l'exemple 2, le composé de l'exemple 12 remplaçant le composé de l'exemple 1. On obtient une huile jaune pâle avec un rendement de 73 % (140 mg).
¹H NMR 400MHz (DMSO-*d₆*) δ : 8,32 (d, 1H, Ph) ; 8,20 (d, 1H, Ph) ; 8,08 (dd, 1H, Ph) ; 3,30 (m, 2H, NCH₂) ; 2,71 (m, 4H, CH₂S(=O)CH₂) ; 2,41 (m, 2H, CH₂) ; 1,86 (m, 2H, CH₂) ; 1,62 (m, 4H, 2 x CH₂) ; 1,46 (s, 6H, 2 x CH₃) ; 1,35 (m, 10H, 5 x CH₂).

### Exemple 14 : 5,5-diméthyl-3-[4-nitro-3-(trifluorométhyl)phényl]-1-{9-[(3,3,4,4,5,5,6,6,6-nonafluorohexyl)thio]nonyl}imidazolidine-2,4-dione

Le protocole expérimental utilisé est le même que celui décrit pour la synthèse de l'exemple 4, l'intermédiaire 12.1 remplaçant l'intermédiaire 4.1 et le S-(3,3,4,4,5,5,6,6,6-nonafluorohexyl) benzenecarbothioate (préparé selon WO 2005077968) remplaçant l'intermédiaire S-(4,4,5,5,5-pentafluoropentyl) benzene-carbothioate. Le composé attendu est obtenu sous forme d'une huile jaune pâle avec un rendement de 78 % (212 mg).
¹H NMR 400MHz (DMSO-*d₆*) δ : 8,42 (d, 1H, Ph) ; 8,30 (d, 1H, Ph) ; 8,18 (dd, 1H, Ph) ; 3,39 (m, 2H, NCH₂) ; 2,80 (m, 2H, SCH₂) ; 2,64 (m, 2H, SCH₂) ; 2,59 (m, 2H, CH₂) ; 1,71 (m, 2H, CH₂) ; 1,61 (m, 2H, CH₂) ; 1,56 (s, 6H, 2 x CH₃) ; 1,41 (m, 10H, 5 x CH₂).

### Exemple 15 : 5,5-diméthyl-3-[4-nitro-3-(trifluorométhyl)phényl]-1-{9-[(3,3,4,4,5,5,6,6,6-nonafluorohexyl)sulfinyl]nonyl}imidazolidine-2,4-dione

Le protocole expérimental utilisé est le même que celui décrit pour la synthèse du composé de l'exemple 2, le composé de l'exemple 14 remplaçant le composé de l'exemple 1. On obtient une huile jaune pâle avec un rendement de 85 % (145 mg).
¹H NMR 400MHz (DMSO-*d₆*) δ : 8,32 (d, 1H, Ph) ; 8,20 (d, 1H, Ph) ; 8,07 (dd, 1H, Ph) ; 3,29 (m, 2H, NCH₂); 3,00 (m, 2H, S(=O)CH₂) ; 2,78 (m, 2H, S(=O)CH₂) ; 2,65 (m, 2H, CH₂) ; 1,62 (m, 4H, 2 x CH₂) ; 1,46 (s, 6H, 2 x CH₃) ; 1,39 (m, 2H, CH₂) ; 1,31 (m, 8H, 4 x CH₂).

### Exemple 16 : 3-[4-amino-3-(trifluorométhyl)phényl]-5,5-diméthyl-1-{9-[(4,4,5,5,5-pentafluoropentyl)thio]nonyl}imidazolidine-2,4-dione

Au composé de l'exemple 1 (1,08 g, 1,7 mmole) dissous dans AcOEt (30 ml) on ajoute SnCl₂, 2H₂O (3,84 g, 17 mmoles). Le mélange réactionnel est chauffé à reflux jusqu'à disparition du composé de départ (5 h 30) et ensuite refroidi à l'aide d'un bain de glace. Après dilution par AcOEt (30 ml), l'ensemble est versé sur une solution aqueuse 1M de NaHCO₃ (120 ml). Le mélange est agité quelques heures au cours desquelles un précipité blanc se forme. Ce précipité est éliminé par filtration sur célite. Le filtrat est décanté et la solution organique est séchée sur Na₂SO₄, filtrée et concentrée à sec sous vide. Le composé attendu est obtenu sous forme d'une huile jaune avec un rendement de 84 % (868 mg).
¹H NMR 400MHz (DMSO-*d₆*) δ : 7,31 (d, 1H, Ph) ; 7,22 (dd, 1H, Ph) ; 6,86 (d, 1H, Ph) ; 5,80 (s, 2H, NH₂) ; 3,27 (m, 2H, NCH₂) ; 2,57 (m, 2H, SCH₂) ; 2,47 (m, 2H, SCH₂) ; 2,29 (m, 2H, CH₂) ; 1,74 (m, 2H, CH₂) ; 1,58 (m, 2H, CH₂) ; 1,48 (m, 2H, CH₂) ; 1,39 (s, 6H, 2 x CH₃) ; 1,25 (m, 10H, 5 x CH₂).

### Exemple 17 : N-[4-(4,4-diméthyl-2,5-dioxo-3-{9-[(4,4,5,5,5-pentafluoropentyl)thio]-nonyl}imidazolidin-1-yl)-2-(trifluorométhyl)phényl]acetamide

A une solution de l'exemple 16 (230 mg, 0,38 mmole) dans CH₂Cl₂ anhydre (2 ml) on ajoute goutte-à-goutte du chlorure d'acétyle (1 ml, 37 éq.), à 23° C. Le mélange réactionnel est agité pendant 1 heure et concentré à sec sous vide. Le résidu d'évaporation est purifié sur une colonne de silice (éluant : Heptane/AcOEt : 1/1 jusqu'à 3/7). Après collection et concentration des fractions pures, le composé attendu est obtenu sous forme d'une huile incolore qui cristallise lentement avec un rendement de 91 % (225 mg). Point de fusion : 84-86° C.
¹H NMR 400MHz (DMSO-*d₆*) δ : 9,67 (s, 1H, NH) ; 7,80 (d, 1H, Ph) ; 7,69 (dd, 1H, Ph) ; 7,58 (d, 1H, Ph) ; 3,28 (m, 2H, NCH₂) ; 2,57 (m, 2H, SCH₂); 2,48 (m, 2H, SCH₂) 2,30 (m, 2H, CH₂) ; 2,06 (s, 3H, CH₃-CO) ; 1,74 (m, 2H, CH₂) ; 1,60 (m, 2H, CH₂) ; 1,48 (m, 2H, CH₂) ; 1,43 (s, 6H, 2 x CH₃) ; 1,28 (m, 10H, 5 x CH₂).

### Exemple 18 : N-[4-(4,4-diméthyl-2,5-dioxo-3-{9-[(4,4,5,5,5-pentafluoropentyl)sulfinyl]-nonyl}imidazolidin-1-yl)-2-(trifluorométhyl)phényl]acetamide

Le protocole expérimental utilisé est le même que celui décrit pour la synthèse du composé de l'exemple 2, le composé de l'exemple 17 remplaçant le composé de l'exemple 1. On obtient une huile incolore avec un rendement de 61 % (98 mg).
¹H NMR 400MHz (DMSO-*d₆*) δ : 9,63 (s, 1H, NH) ; 7,81 (d, 1H, Ph) ; 7,68 (dd, 1H, Ph) ; 7,58 (d, 1H, Ph) ; 3,28 (m, 2H, NCH₂) ; 2,72 (m, 4H, CH₂S(=O)CH₂) ; 2,38 (m, 2H, CH₂) ; 2,06 (s, 3H, CH₃-CO) ; 1,90 (m, 2H, CH₂) ; 1,60 (m, 4H, 2 x CH₂) ; 1,44 (s, 6H, 2 x CH₃) ; 1,39 (m, 2H, CH₂) ; 1,30 (m, 8H, 4 x CH₂).

### Exemple 19 : 4-(4,4-dimethyl-2,5-dioxo-3-{9-[(4,4,5,5,5-pentafluoropentylsulfanyl]nonyl} imidazolidin-1-yl)-2-(trifluoromethyl)benzonitrile

### 19.1) 4-(4,4-dimethyl-2,5-dioxoimidazolidin-1-yl)-2-(trifluoromethyl)benzonitrile

Un mélange de 4-fluoro-2-(trifluorométhyl)benzonitrile (5,67 mg, 30 mmoles), 5,5-diméthyl-hydantoine (7,68 g, 60 mmoles), K2CO3 (8,28 g, 60 mmoles) dans le DMF (45 ml) est réparti en parts égales dans trois tubes destinés au four à micro-onde. Sous agitation magnétique, chaque tube est irradié à 140° C pendant 20 minutes. Les masses réactionnelles sont ensuite réunies, versées dans de l'eau (200 ml) et extraites par AcOEt (2 x 75 ml). Les phases organiques sont réunies, lavées par de la saumure, séchées sur Na2SO4 et filtrées. Le filtrat est concentré sous pression réduite et le résidu cristallisé dans Et2O (25 ml). Après recristallisation dans EtOH (75 ml), la poudre est filtrée et séchée sous vide. Le composé attendu est obtenu sous forme d'un solide blanc avec un rendement de 46 % (4,1 g). Point de fusion : 212-213° C.
¹H NMR 400MHz (DMSO-d₆) δ : 8,80 (s, 1H, NH) ; 8,29 (d, 1H, Ph) ; 8,18 (s, 1H, Ph) ; 8,02 (d, 1H, Ph) ; 1,42 (s, 6H, 2 x CH₃).

### 19.2) 4-[3-(9-bromononyl)-4,4-dimethyl-2,5-dioxoimidazolidin-1-yl]-2-(trifluoromethyl) benzonitrile

Le protocole expérimental utilisé est le même que celui décrit pour la synthèse de l'intermédiaire 12.1, l'intermédiaire 19.1 remplaçant le 5,5-dimethyl-3-[4-nitro-3-(trifluoromethyl)phenyl]imidazolidine-2,4-dione. Le composé attendu est obtenu sous forme d'une huile jaune avec un rendement de 80 %.
¹H NMR 400MHz (DMSO-d₆) δ: 8,29 (d, 1H, Ph) ; 8,18 (d, 1H, Ph) ; 8,04 (dd, 1H, Ph) ; 3,50 (m, 2H, CH₂Br) ; 3,29 (m, 2H, NCH₂) ; 1,78 (m, 2H, CH₂) ; 1,61 (m, 2H, CH₂) ; 1,46 (s, 6H, 2 x CH₃) ; 1,32 (m, 10H, 5 x CH₂).

### 19.3) 4-(4,4-dimethyl-2,5-dioxo-3-{9-[(4,4,5,5,5-pentafluoropentylsulfanyl]nonyl} imidazolidin-1-yl)-2-(trifluoromethyl)benzonitrile

Le protocole expérimental utilisé est le même que celui décrit pour la synthèse de l'intermédiaire 4.2, l'intermédiaire 19.2 remplaçant l'intermédiaire 4.1. Le composé attendu est obtenu sous forme d'une huile jaune avec un rendement de 89 %.
¹H NMR 400MHz (DMSO-d₆) δ: 8,24 (d, 1H, Ph) ; 8,13 (d, 1H, Ph) ; 7,99 (dd, 1H, Ph) ; 3,22 (m, 2H, NCH₂) ; 2,52 (m, 2H, SCH₂) ; 2,44 (m, 2H, SCH₂) ; 2,26 (m, 2H, CH₂) ; 1,70 (m, 2H, CH₂) ; 1,56 (m, 2H, CH₂) ; 1,48 (m, 2H, CH₂) ; 1,40 (s, 6H, 2 x CH₃) ; 1,21 (m, 10H, 5 x CH₂).

### Exemple 20 : 4-(4,4-dimethyl-2,5-dioxo-3-{9-[(4,4,5,5,5-pentafluoropentyl)sulfinyl] nonyl}imidazolidin-1-yl)-2-(trifluoromethyl)benzonitrile

Le protocole expérimental utilisé est le même que celui décrit pour la synthèse du composé de l'Exemple 2, le composé de l'Exemple 19 remplaçant le composé de l'Exemple 1. On obtient une huile incolore avec un rendement de 78 %.
¹H NMR 400MHz (DMSO-d₆) δ: 8,29 (d, 1H, Ph) ; 8,18 (d, 1H, Ph) ; 8,03 (dd, 1H, Ph) ; 3,28 (m, 2H, NCH₂) ; 2,82 (m, 4H, CH₂S(=O)CH₂) ; 2,38 (m, 2H, CH₂) ; 1,89 (m, 2H, CH₂) ; 1,62 (m, 4H, 2 x CH₂) ; 1,45 (s, 6H, 2 x CH₃) ; 1,31 (m, 10H, 5 x CH₂).

### Exemple 21 : 4-(4,4-dimethyl-2,5-dioxo-3-{9-[(4,4,5,5,5-pentafluoropentyl)sulfanyl] nonyl}imidazolidin-1-yl)-2-(trifluoromethyl)benzamide

Le composé de l'Exemple 19 (359 mg, 0,58 mmole) est mélangé à de l'acide trifluoroacétique (4 ml) et de l'acide sulfurique (1 ml). Après 15 heures d'agitation à 60° C, le milieu réactionnel est versé sur un mélange eau-glace et extrait par AcOEt (2 x 50 ml). Les phases organiques sont réunies et lavées successivement par de l'eau, une solution aqueuse saturée de NaHCO₃ et de la saumure. La solution organique est ensuite séchée sur Na₂SO₄, filtrée et le solvant évaporé sous pression réduite. Le résidu d'évaporation est purifié par chromatographie sur une colonne de silice (éluant : Heptane/AcOEt : 9/1 jusqu'à 4/6). Le composé attendu est obtenu sous forme d'une huile jaune avec un rendement de 70 %.
LC-MS (UV) : pureté (220 nM) : 99 %. ES⁻ : (M+TFA-H)⁻ : 746.

### Exemple 22 : 4-(4,4-dimethyl-2,5-dioxo-3-{9-[(4,4,5,5,5-pentafluoropentyl)sulfinyl] nonyl}imidazolidin-1-yl)-2-(trifluoromethyl)benzamide

Le protocole expérimental utilisé est le même que celui décrit pour la synthèse du composé de l'Exemple 2, le composé de l'Exemple 21 remplaçant le composé de l'Exemple 1. On obtient une huile incolore avec un rendement de 50 %.
¹H NMR 400MHz (DMSO-d₆) δ:7,96 (s large, 1H, ½ NH₂) ; 7,81 (s, 1H, Ph) ; 7,70 (d, 1H, Ph) ; 7,59 (m, 2H, Ph + ½ NaH₂) ; 3,25 (m, 2H, NCH₂) ; 2,70 (m, 4H, CH₂S(=O)CH₂) ; 2,30 (m, 2H, CH₂) ; 1,83 (m, 2H, CH₂) ; 1,57 (m, 4H, 2 x CH₂) ; 1,40 (s, 6H, 2 x CH₃) ; 1,25 (m, 10H, 5 x CH₂).

### Exemple 23 : 5,5-dimethyl-3-(3-methyl-4-nitrophenyl)-1-{9-[(4,4,5,5,5-pentafluoro pentyl)sulfanyl]nonyl}imidazolidine-2,4-dione

### 23.1) 5,5-dimethyl-3-(3-methyl-4-nitrophenyl)imidazolidine-2,4-dione

Un mélange de 5-fluoro-2-nitrotoluene (1,55 g, 10 mmoles), 5,5-diméthylhydantoine (1,28 g, 10 mmoles), K₂CO₃ (1,38 g, 10 mmoles) dans le DMF (15 ml) est introduit dans une vial destinée au four à micro-onde et irradié à 100° C pendant 70 minutes, sous agitation magnétique. Le mélange réactionnel est ensuite versé dans de l'eau (200 ml) et extrait par AcOEt (2 x 75 ml). Les phases organiques sont réunies, lavées par de la saumure, séchées sur Na₂SO₄ et filtrées. Le filtrat est concentré sous pression réduite et le résidu est purifié par chromatographie sur une colonne de silice (éluant : Heptane/AcOEt : 7/3). Le composé attendu est obtenu sous forme d'un solide blanc avec un rendement de 25 % (666 mg). Point de fusion : 177-178° C.
¹H NMR 400MHz (DMSO-d₆) δ : 8,70 (s, 1H, NH) ; 8,10 (d, 1H, Ph) ; 7,58 (s, 1H, Ph) ; 7,52 (dd, 1H, Ph) ; 2,54 (s, 3H, CH₃) ; 1,41 (s, 6H, 2 x CH₃).

### 23.2) 1-(9-bromononyl)-5,5-dimethyl-3-(3-methyl-4-nitrophenyl)imidazolidine-2,4-dione

Le protocole expérimental utilisé est le même que celui décrit pour la synthèse de l'intermédiaire 12.1, l'intermédiaire 23.1 remplaçant le 5,5-dimethyl-3-[4-nitro-3-(trifluoromethyl)phenyl]imidazolidine-2,4-dione. Le composé attendu est obtenu sous forme d'une huile jaune avec un rendement de 74 %.
¹H NMR 400MHz (DMSO-d₆) δ: 8,10 (d, 1H, Ph); 7,59 (d, 1H, Ph); 7,53 (dd, 1H, Ph) ; 3,51 (m, 2H, CH₂Br) ; 3,29 (m, 2H, NCH₂) ; 2,54 (s, 3H, 1 CH₃) ; 1,78 (m, 2H, CH₂) ; 1,61 (m, 2H, CH₂) ; 1,44 (s, 6H, 2 x CH₃) ; 1,32 (m, 10H, 5 x CH₂).

### 23.3) 5,5-dimethyl-3-(3-methyl-4-nitrophenyl)-1-{9-[(4,4,5,5,5-pentafluoropentyl) sulfanyl]nonyl}imidazolidine-2,4-dione

Le protocole expérimental utilisé est le même que celui décrit pour la synthèse de l'intermédiaire 4.2, l'intermédiaire 23.2 remplaçant l'intermédiaire 4.1. Le composé attendu est obtenu sous forme d'une huile jaune avec un rendement de 90 %.
¹H NMR 400MHz (DMSO-d₆) δ: 8,10 (d, 1H, Ph) ; 7,59 (d, 1H, Ph) ; 7,54 (dd, 1H, Ph) ; 3,26 (m, 2H, NCH₂) ; 2,56 (m, 5H, SCH₂ + CH₃) ; 2,49 (m, 2H, SCH₂) ; 2,28 (m, 2H, CH₂) ; 1,74 (m, 2H, CH₂) ; 1,61 (m, 2H, CH₂) ; 1,52 (m, 2H, CH₂) ; 1,45 (s, 6H, 2 x CH₃) ; 1,28 (m, 10H, 5 x CH₂).

### Exemple 24 : 5,5-dimethyl-3-(3-methyl-4-nitrophenyl)-1-{9-[(4,4,5,5,5-pentafluoro pentyl)sulfinyl]nonyl}imidazolidine-2,4-dione

Le protocole expérimental utilisé est le même que celui décrit pour la synthèse du composé de l'Exemple 2, le composé de l'Exemple 23 remplaçant le composé de l'Exemple 1. On obtient une huile incolore avec un rendement de 50%.
¹H NMR 400MHz (DMSO-d₆) δ : 8,05 (s, 1H, Ph) ; 7,53 (d, 1H, Ph) ; 7,49 (m, 1H, Ph) ; 3,24 (m, 2H, NCH₂) ; 2,70 (m, 4H, CH₂S(=O)CH₂) ; 2,49 (s, 3H, CH₃) ; 2,30 (m, 2H, CH₂) ; 1,85 (m, 2H, CH₂) ; 1,57 (m, 4H, 2 x CH₂) ; 1,40 (s, 6H, 2 x CH₃) ; 1,25 (m, 10H, 5 x CH₂).

### Exemple 25 : 3-(4-amino-3-methylphenyl)-5,5-dimethyl-1-{9-[(4,4,5,5,5-pentafluoro pentyl)sulfanyl]nonyl}imidazolidine-2,4-dione

Le protocole expérimental est le même que celui décrit pour le composé de l'Exemple 16, le composé de l'Exemple 23 remplaçant le composé de l'Exemple 1. Le composé attendu est obtenu sous forme d'une huile brune avec un rendement de 93 %.
¹H NMR 400MHz (DMSO-d₆) δ: 6,81 (d, 1H, Ph) ; 6,78 (dd, 1H, Ph) ; 6,60 (d, 1H, Ph); 5,02 (s, 2H, NH₂); 3,25 (m, 2H, NCH₂); 2,57 (m, 2H, SCH₂) ; 2,48 (m, 2H, SCH₂) ; 2,30 (m, 2H, CH₂) ; 2,04 ( s, 3H, CH₃) ; 1,75 (m, 2H, CH₂) ; 1,55 (m, 2H, CH₂) ; 1,48 (m, 2H, CH₂) ; 1,37 (s, 6H, 2 x CH₃) ; 1,22 (m, 10H, 5 x CH₂).

### Exemple 26 : 3-(4-amino-3-methylphenyl)-5,5-dimethyl-1-{9-[(4,4,5,5,5-pentafluoro pentyl)sulfinyl]nonyl}imidazolidine-2,4-dione

Le protocole expérimental utilisé est le même que celui décrit pour la synthèse du composé de l'Exemple 2, le composé de l'Exemple 25 remplaçant le composé de l'Exemple 1. On obtient une huile incolore avec un rendement de 56 %.
¹H NMR 400MHz (DMSO-d₆) δ : 6,82 (d, 1H, Ph) ; 6,79 (dd, 1H, Ph) ; 6,60 (d, 1H, Ph) ; 5,02 (s, 2H, NH₂) ; 3,25 (m, 2H, NCH₂) ; 2,72 (m, 4H, CH₂S(=O)CH₂) ; 2,34 (m, 2H, CH₂) ; 2,04 (s, 3H, CH₃) ; 1,91 (m, 2H, CH₂) ; 1,61 (m, 4H, 2 x CH₂) ; 1,41 (m, 8H, 2 x CH₃ + CH₂) ; 1,25 (m, 8H, 4 x CH₂).

### Exemple 27 : 1-[4-(4,4-dimethyl-2,5-dioxo-3-{9-[(4,4,5,5,5-pentafluoropentyl)sulfanyl] nonyl}imidazolidin-1-yl)-2-methylphenyl]-3-(1-methylpropyl)urea

Sous atmosphère d'argon, le composé de l'exemple 25 (395 mg, 0,72 mmole) est dissous dans du 1,2-dichloroéthane anhydre (10 ml) avant l'addition goutte-à-goutte de sec-butylisocyanate (0,35 ml, 3 mmoles) à 23° C. L'ensemble est ensuite chauffé à reflux pendant 24 heures. Le milieu réactionnel est alors versé sur de l'eau froide et extrait à l'aide de CH₂Cl₂. Après décantation, la phase organique est lavée par de l'eau et de la saumure. La solution organique est ensuite séchée sur MgSO₄, filtrée, concentrée à sec sous vide et le résidu d'évaporation est purifié sur une colonne de silice (éluant : heptane/AcOEt : 1/0 jusqu'à 4/6). Le composé attendu est obtenu sous forme d'une huile incolore avec un rendement de 36 %.
¹H NMR 400MHz (DMSO-d₆) δ: 7,96 (d, 1H, NH) ; 7,61 (s, 1H, NH) ; 7,07 (d, 1H, Ph) ; 7,02 (dd, 1 H, Ph) ; 6,48 (d, 1H, Ph) ; 3,59 (m, 1H, CH) ; 3,26 (m, 2H, NCH₂) ; 2,57 (m, 2H, SCH₂) ; 2,48 (m, 2H, SCH₂) ; 2,28 (m, 2H, CH₂) ; 2,18 (s, 3H, CH₃) ; 1,74 (m, 2H, CH₂) ; 1,58-1,42 (m, 6H, 3 x CH₂) ; 1,40 (s, 6H, 2 x CH₃) ; 1,30 (m, 10H, 5 x CH₂) ; 1,07 (d, 3H, CH₃) ; 0,88 (t, 3H, CH₃).

### Exemple 28 : 1-[4-(4,4-dimethyl-2,5-dioxo-3-{9-[(4,4,5,5,5-pentafluoropentyl)sulfinyl] nonyl}imidazolidin-1-yl)-2-methylphenyl]-3-(1-methylpropyl)urea

Le protocole expérimental utilisé est le même que celui décrit pour la synthèse du composé de l'Exemple 2, le composé de l'Exemple 25 remplaçant le composé de l'Exemple 1. On obtient une huile incolore avec un rendement de 63 %.
¹H NMR 400MHz (DMSO-d₆) δ: 7,96 (d, 1H, NH) ; 7,61 (s, 1H, NH) ; 7,07 (d, 1H, Ph) ; 7,02 (dd, 1H, Ph) ; 6,48 (d, 1H, Ph) ; 3,59 (m, 1H, CH) ; 3,26 (m, 2H, NCH₂) ; 2,86-2,65 (m, 4H, CH₂S(=O)CH₂) ; 2,40 (m, 2H, CH₂) ; 2,18 (s, 3H, CH₃) ; 1,91 (m, 2H, CH₂) ; 1,61 (m , 4H, 2 x CH₂) ; 1,41 (m, 10H, 2 x CH₂ + 2 x CH₃) ; 1,30 (m, 8H, 4 x CH₂) ; 1,07 (d, 3H, CH₃) ; 0,88 (t, 3H, CH₃).

### Exemple 29 : tert-butyl {[4-(4,4-dimethyl-2,5-dioxo-3-{9-[(4,4,5,5,5-pentafluoropentyl)sulfanyl] nonyl}imidazolidin-1-yl)-2-methylphenyl]sulfamoyl} carbamate

Une solution du composé de l'Exemple 25 (612 mg, 1,1 mmole) et de Et₃N (0,19 ml, 1,33 mmole) dans CH₂Cl₂ anhydre (30 ml) est agitée à 0° C. D'autre part, une solution de chlorosulfonyl isocyanate (0,11 ml, 1,22 mmole) dans CH₂Cl₂ anhydre (10 ml), refroidie à 0° C, est complétée par t-BuOH (0,12 ml, 1,22 mmole) avant d'être ajoutée rapidement à la solution du composé de l'Exemple 25. L'ensemble est agité 30 min à 0° C suivi de 1 heure et demi à 23° C. Le milieu réactionnel est ensuite lavé successivement par de l'eau (2 x 100 ml), une solution aqueuse saturée de NaHCO₃ (50 ml) et de la saumure (50 ml). La solution organique est séchée sur Na₂SO₄, filtrée et concentrée à sec. Le résidu d'évaporation est purifié sur une colonne de silice (éluant : Heptane/AcOEt : 1/0 jusqu'à 6/4). Le composé attendu est obtenu sous forme d'un solide blanc avec un rendement de 85 %. Point de fusion 122-124° C.
¹H NMR 400MHz (DMSO-d₆) δ: 11,07 (s, 1H, NH) ; 9,64 (s, 1H, NH) ; 7,28 (d, 1H, Ph) ; 7,20 (m, 2H, Ph) ; 3,28 (m, 2H, NCH₂) ; 2,56 (m, 2H, SCH₂) ; 2,48 (m, 2H, SCH₂) ; 2,29 (m, 5H, CH₂ + CH₃) ; 1,75 (m, 2H, CH₂) ; 1,58 (m, 2H, CH₂) ; 1,49 (m, 2H, CH₂) ; 1,42 (s, 15H, 2 x CH₃ + tBu) ; 1,28 (m, 10H, 5 x CH₂).

### Exemple 30 : N-[4-(4,4-dimethyp-2,5-dioxo-3-{9-[(4,4,5,5,5-pentafluoropentyl)sulfanyl] nonyl}imidazolidin-1-yl)-2-methylphenyl]sulfamide

A une solution de l'exemple 29 (250 mg, 0,36 mmole) dans un mélange CH₂Cl₂/AcOEt (5 ml/3ml) refroidie à 0° C, une solution d'HCl dans l'éther éthylique (2N, 3 ml) est ajoutée en 3 portions de 1 ml et l'ensemble est agité pendant 60 heures à 23° C. Les volatiles sont évaporés sous vide et le résidu est repris dans un mélange CH₂Cl₂ (50 ml) et NaHCO₃ (50 ml). Après agitation et décantation, la phase organique est lavée par de l'eau (50 ml) suivi de saumure. Après séchage sur Na₂SO₄, filtration et concentration à sec, le résidu est purifié sur une colonne de silice (Heptane/AcOEt : 10/0 jusqu'à 1/1). Le produit attendu est obtenu sous forme d'un solide blanc avec un rendement de 76 %. Point de fusion : 102-104° C.
¹H NMR 400MHz (DMSO-d₆) δ: 8,60 (s, 2H, NH) ; 7,42 (d, 1H, Ph) ; 7,14 (m, 2H, Ph) ; 6,97 (s, 2H, NH₂) ; 3,26 (m, 2H, NCH₂) ; 2,57 (m, 2H, SCH₂) ; 2,48 (m, 2H, SCH₂) ; 2,27 (m, 5H, CH₂ + CH₃) ; 1,75 (m, 2H, CH₂) ; 1,58 (m, 2H, CH₂) ; 1,52 (m, 2H, CH₂) ; 1, 48 (m, 2H, CH₂) ; 1,41 (s, 6H, 2 x CH₃) ; 1,30 (m, 10H, 5 x CH₂).

### Exemple 31 : N-[4-(4,4-dimethyl-2,5-dioxo-3-{9-[(4,4,5,5,5-pentafluoropentyl)sulfinyl] nonyl}imidazolidin-1-yl)-2-methylphenyl]sulfamide

Le protocole expérimental utilisé est le même que celui décrit pour la synthèse du composé de l'Exemple 2, le composé de l'Exemple 30 remplaçant le composé de l'Exemple 1. On obtient une solide blanc avec un rendement de 62 %.
¹H NMR 400MHz (DMSO-d₆) δ: 8,60 (s, 1H, NH) ; 7,42 (d, 1H, Ph) ; 7,14 (m, 2H, Ph) ; 6,97 (s, 2H, NH₂) ; 3,28 (m, 2H, NCH₂) ; 2,78 (m, 4H, CH₂S(=O)CH₂); 2,38 (m, 2H, CH₂) ; 2,29 (s, 3H, CH₃) ; 1,90 (m, 2H, CH₂) ; 1,60 (m, 4H, 2 x CH₂) ; 1,40 (m, 8H, 2 x CH₃ + CH₂) ; 1,30 (m, 8H, 4 x CH₂).

### Exemple 32 : N-[4-(4,4-dimethyl-2,5-dioxo-3-{9-[(4,4,5,5,5-pentafluoropentyl)sulfonyl] nonyl}imidazolidin-1-yl)-2-methylphenyl]sulfamide

Le protocole expérimental utilisé est le même que celui décrit pour la synthèse du composé de l'Exemple 3, le composé de l'Exemple 31 remplaçant le composé de l'Exemple 2. On obtient une huile incolore.
¹H NMR 400MHz (DMSO-d₆) δ: 8,55 (s, 1H, NH) ; 7,37 (d, 1H, Ph) ; 7,09 (m, 2H, Ph) ; 6,92 (s, 2H, NH₂) ; 3,15 (m, 4H, CH₂S(=O)₂CH₂) ; 3,05 (m, 2H, NCH₂) ; 2,32 (m, 2H, CH₂) ; 2,24 (s, 3H, CH₃) ; 1,91 (m, 2H, CH₂) ; 1,60 (m, 4H, 2 x CH₂) ; 1,39 (m, 8H, 2 x CH₃ + CH₂) ; 1,22 (m, 8H, 4 x CH₂).

### Exemple 33 : 3-[4-amino-3-(trifluorométhyl)phényl]-5,5-diméthyl-1-{9-[(4,4,5,5,5-pentafluoropentyl)sulfinyl]nonyl}imidazolidine-2,4-dione

Le protocole expérimental utilisé est le même que celui décrit pour la synthèse du composé de l'Exemple 3, le composé de l'Exemple 16 remplaçant le composé de l'Exemple 2.
¹H NMR 400MHz (DMSO-*d₆*) δ : 7,30 (d, 1H, Ph) ; 7,21 (dd, 1H, Ph) ; 6,85 (d, 1H, Ph) ; 5,80 (s, 1H, NH) ; 3,27 (m, 2H, NCH₂) ; 2,74 (m, 4H, CH₂S(=O)CH₂) ; 2,38 (m, 2H, CH₂) ; 1,90 (m, H, CH₂) ; 1,60 (m, 4H, 2 x CH₂) ; 1,40 (s, 8H, 2 x CH₃ + CH₂) ; 1,29 (m, 8H, 4 x CH₂).

### Exemple 34 : 7-[4-nitro-3-(trifluoromethyl)phenyl]-5-{9-[(4,4,5,5,5-pentafluoropentyl)sulfinyl]nonyl}-5,7-diazaspiro[3.4]octane-6,8-dione

### 34.1) 5,7-diazaspiro[3.4]octane-6,8-dione

Sous argon et à 23°, on ajoute le cyanure de sodium (1.47g, 30 mmole) puis le carbonate d'ammonium (7.5g, 78 mmole) à une solution de cyclobutanone (1.49 ml, 20 mmole) dilué dans un mélange de solvant éthanol-eau (16ml). Le mélange réactionnel est chauffé 6 heures à 70° C. Après refroidissement du milieu réactionnel, on verse de l'eau (15ml) puis on ajoute avec précaution une solution d'acide chlorhydrique concentré (13 ml). Agiter 10 heures à 23°C puis évaporer au rotavapor l'éthanol et une partie de l'eau contenu dans le mélange réactionnel. Filtrer le précipité sur fritté puis laver à l'eau. Le composé attendu est obtenu sous forme d'une poudre beige avec un rendement de 39 % (1.1 g).
¹H NMR 400MHz (DMSO-*d₆*) δ : 10,49 (sé, 1H, NH) ; 8,27 (s, 1H, NH) ; 2,31-2.37 (m, 2H, CH2) ; 2,19-2.27 (m, 2H, CH₂) ; 1,83-1.90 (m, 1H, CH_{A}) ; 1,71-1.76 (m, 1H, CH_{B}).
Point de fusion : 223-225°C

### 34.2) 7-[4-nitro-3-(trifluoromethyl)phenyl}-5,7-diazaspiro[3.4]octane-6,8-dione

Sous argon, à une solution de 5,7-diazaspiro[3.4]octane-6,8-dione (préparé précedemment) (221 mg, 0,74 mmole) dans DMF (6 ml) on ajoute le carbonate de potassium (1.09 g, 7,85 mmole) et le composé 5-fluoro 2nitro-3-(trifluorométhyl)phényl]-imidazolidine-2,4-dione (500 mg, 1,6 mmole). On chauffe à 65°C pendant 2 heures puis l'agitation est maintenue 12 heures à 23°C. Le milieu réactionnel est versé dans une solution aqueuse saturée de NH₄Cl (25 ml) et extrait par AcOEt (2 x 25 ml). Les phases organiques sont réunies et lavées successivement par de l'eau (25 ml) et de la saumure (25 ml). Après séchage sur Na₂SO₄, la solution organique est filtrée et concentrée sous vide. Le résidu d'évaporation est purifié sur une colonne de silice (éluant : Heptane/AcOEt : 7/3 jusqu'à 3/7). Après lavage à l'isopentane puis filtration, le composé attendu est obtenu sous forme d'une poudre jaune pâle avec un rendement de 50 % (645 mg).
¹H NMR 400MHz (DMSO-*d₆*) δ : 9,15 (s, 1H, NH) ; 8,30 (d, 1H, Ph) ; 8,18 (d, 1H, Ph) ; 8,00 (dd, 1H, Ph) ; 2,51 (m, 2H, CH2) ; 2,37 (m, 2H, CH₂) ; 1,94 (m, 1H, CH_{A}) ; 1,73 (m, 1H, CH_{B}).

### 34.3) 7-[4-nitro-3-(tritluoromethyl)phenyl)-5-{9-[(4,4,5,5,5-pentafluoropentyl)sulfanyl]nonyl}-5,7-diazaspiro[3.4]octane-6,8-dione

Sous argon, on ajoute NaH (à 60 %) (44 mg, 1,1 mmole) à une solution de 7-[4-nitro-3-(trifluoromethyl)phenyl]-5,7-diazaspiro[3.4]octane-6,8-dione (329 mg, 1 mmole) dans du DMF anhydre (9 ml). Un dégagement gazeux accompagne le changement de coloration du milieu réactionnel qui devient orange. L'agitation est maintenue 1 heure à 23° C avant d'ajouter le 9-[(4,4,5,5,5-pentafluoropentyl)-thio]nonyl méthanesulfonate (préparé selon un protocole expérimental décrit dans WO 2005077968) (332 mg, 0,8 mmole). Après 15 heures de réaction, le milieu réactionnel est versé dans de l'eau (25 ml) et extrait par AcOEt (2 x 25 ml). Les phases organiques sont rassemblées et lavées successivement par de l'eau (25 ml) et de la saumure (25 ml). Après séchage sur Na₂SO₄, la solution organique est filtrée et concentrée sous vide. Le résidu d'évaporation est purifié sur une colonne de silice RP18 (éluant : ACN/H₂O : 8/2 jusqu'à 100). Le composé attendu est obtenu sous forme d'une huile incolore avec un rendement de 33 % (214 mg).
¹H NMR 400MHz (DMSO-*d₆*) δ : 8,31 (d, 1H, Ph) ; 8,18 (d, 1H, Ph) ; 8,05 (dd, 1H, Ph) ; 3,40 (m, 2H, NCH₂) ; 2,55 (m, 4H, SCH₂, CH₂) ; 2,45 (m, 2H, SCH₂) ; 2, 30 (m, 2H, CH₂); 2,03 (m, 1H, CH_{A}) ; 1,81 (m, 2H, CH₂) ; 1,74 (m, 3H, CH₂, CH_{B}) ; 1,62 (m, 2H, CH₂) ; 1,49 (s, 2H, CH₂) ; 1,30 (m, 10H, 5 x CH₂).

### 34.4) 7-[4-nitro-3-(trifluoromethyl)phenyl]-5-{9-[(4,4,5,5,5-pentafluoropentyl)sulfinyl]nonyl}-5,7-diazaspiro[3.4]octane-6,8-dione

Le composé 34.4 décrit ci-dessous a été synthétisé selon une méthode analogue à celle décrite à l'exemple 2, l'intermédiaire 34.3 remplaçant le 5,5-diméthyl-3-[4-nitro-3-(trifluorométhyl)phényl]-1-{9-[(4,4,5,5,5-pentafluoropentyl)thio]nonyl} imidazolidine-2,4-dione de l'exemple 1. Le composé attendu est obtenu sous forme d'une huile jaune pâle avec un rendement de 79 % (170 mg).
¹H NMR 400MHz (DMSO-*d₆*) δ : 8,31 (d, 1H, Ph) ; 8,18 (d, 1H, Ph) ; 8,06 (dd, 1H, Ph) ; 3,44 (m, 2H, NCH₂) ; 2,61 (m, 6H, CH₂S(=O)CH₂, CH₂) ; 2,43 (m, 4H, 2CH₂) ; 2,03 (m, 1H, CH_{A}) ; 1,87 (m, 3H, CH₂, CH_{B}) ; 1,62 (m, 4H, 2CH₂) ; 1,31 (m, 10H, 5 x CH₂).

### Exemple 35 : 5,5-dimethyl-3-[4-nitro-2-(trifluoromethyl)phenyl]-1-{9-[(4,4,5,5,5-pentafluoropentyl)sulfinyl]nonyl}imidazolidine-2,4-dione

### 35.1) 5,5-dimethyl-3-[4-nitro-2-(trifluoromethyl)phenyl]imidazolidine-2,4-dione

Le protocole expérimental utilisé est le même que celui décrit pour la synthèse du composé de l'Exemple 19.1, le 1-fluoro-4-nitro-2-(trifluoromethyl)benzene remplaçant le 4-fluoro-2-(trifluorométhyl)benzonitrile. Le composé attendu est obtenu sous forme d'une poudre blanche avec un rendement de 77 %.
Point de fusion : 201-203°C
¹H NMR 400MHz (DMSO-*d₆*) δ : 8,76 (s, 1H, NH) ; 8,67 (dd, 1H, Ph) ; 8,58 (d, 1H, Ph) ; 8,04 (d, 1H, Ph) ; 1,44 (s, 3H, CH₃) ; 1,38 (s, 3H, CH₃) .

### 35.2) 5,5-dimethyl-3-[4-nitro-2-(trifluoromethyl)phenyl]-1-{9-[(4,4,5,5,5-pentafluoropentyl)sulfanyl]nonyl}imidazolidine-2,4-dione

Le protocole expérimental utilisé est le même que celui décrit pour la synthèse de l' exemple 1, l'intermédiaire 35.1 remplaçant le 5,5-dimethyl-3-[4-nitro-3-(trifluoromethyl)phenyl]imidazolidine-2,4-dione. Le composé attendu est obtenu sous forme d'une huile incolore avec un rendement de 50 %.
¹H NMR 400MHz (DMSO-*d₆*) δ : 8,69 (dd, 1H, Ph) ; 8,57 (d, 1H, Ph) ; 8,05 (d, 1H, Ph) ; 3,27 (m, 2H, NCH₂) ; 2,57 (m, 2H, SCH₂) ; 2,48 (m, 2H, SCH₂); 2,29 (m, 2H, CH₂) ; 1,76 (m, 2H, CH₂) ; 1,59 (m, 2H, CH₂) ; 1,40 (s, 8H, 2 x CH₃ + CH₂) ; 1,28 (m, 10H, 5 x CH₂).

### 35.3) 5,5-dimethyl-3-[4-nitro-2-(trifluoromethyl)phenyl]-1-{9-[(4,4,5,5,5-pentafluoropentyl)sulfinyl]nonyl}imidazolidine-2,4-dione

Le protocole expérimental utilisé est le même que celui décrit pour la synthèse du composé de l'Exemple 2, l'intermédiaire 35.2 remplaçant le 5,5-diméthyl-3-[4-nitro-3-(trifluorométhyl)phényl]-1-{9-[(4,4,5,5,5-pentafluoropentyl)thio]nonyl}imidazolidine-2,4-dione de l'exemple 1. Le composé attendu est obtenu sous forme d'une huile incolore avec un rendement de 85 %.
¹H NMR 400MHz (DMSO-*d₆*) δ: 8,69 (dd, 1H, Ph) ; 8,57 (d, 1H, Ph) ; 8,05 (d, 1H, Ph) ; 3,30 (m, 2H, NCH₂) ; 2,82 (m, 2H, S(=O)CH₂) ; 2,66 (m, 2H, S(=O)CH₂) ; 2,39 (m, 2H, CH₂) ; 1,90 (m, 2H, CH₂) ; 1,60 (m, 4H, 2CH₂) ; 1,50 (s, 3H, CH₃) ; 1,41 (s, 3H, CH₃) ; 1,30 (m, 10H, 5 x CH₂).

### Exemple 36 : 5,5-dimethyl-3-[4-nitro-3-(trifluoromethyl)phenyl]-1-[2-(2-{2-[(4,4,5,5,5-pentafluoropentyl)sulfanyl]ethoxy}ethoxy)ethyl]imidazolidine-2,4-dione

### 36.1)1-{2-[2-(2-iodoethoxy)ethoxy]ethyl}-5,5-dimethyl-3-[4-nitro-3-(trifluoromethyl)phenyl]imidazolidine-2,4-dione

Le protocole expérimental utilisé est le même que celui décrit pour la synthèse de l'intermédiaire 4.1, le 1,2bis-(2-iodoethoxy)ethane remplaçant le 1,5-diiodopentane. Le composé attendu est obtenu sous forme d'une huile jaune pâle avec un rendement de 59 % (666 mg).
¹H NMR 400MHz (DMSO-*d*₆). δ :. 8,32 (d, 1H, Ph) ; 8,20 (d, 1H, Ph) ; 8,08 (m, 1H, Ph) ; 3,64 (m, 4H, 2 x OCH₂); 3,57 (s, 4H, 2 x OCH₂); 3,51 (m, 2H, NCH₂); 3,29 (m, 2H, CH₂I); 1,47 (s, 6H, 2 x CH₃).

### 36.2) 5,5-dimethyl-3-[4-nitro-3-(trifluoromethyl)phenyl]-1-[2-(2-{2-[(4,4,5,5,5-pentafluoropentyl)sulfanyl]ethoxy}ethoxy)ethyl]imidazolidine-2,4-dione

Le protocole expérimental utilisé est le même que celui décrit pour la synthèse de 1' exemple 4, l'intermédiaire 36.1 remplaçant le 1-(5-iodopentyl)-5,5-diméthyl-3-[4-nitro-3-(trifluorométhyl)phényl]imidazolidine-2,4-dione. Le composé attendu est obtenu sous forme d'une huile incolore avec un rendement de 73 %.
¹H NMR 400MHz (DMSO-*d*₆). δ :. 8,32 (d, 1H, Ph) ; 8,20 (d, 1H, Ph) ; 8,08 (m, 1H, Ph) ; 3,52 (m, 10H, 5 x CH₂) ; 2,63 (s, 4H, 2 x CH₂) ; 2,28 (m, 2H, CH₂); 1,75 (m, 2H, CH₂); 1,47 (s, 6H, 2 x CH₃).

### Exemple 37: 5,5-dimethyl-3-[4-nitro-3-(trifluoromethyl)phenyl]-1-[2-(2-{2-[(4,4,5,5,5-pentafluoropentyl)sulfinyl]ethoxy}ethoxy)ethyl]imidazolidine-2,4-dione

Le composé 37 décrit ci-dessous a été synthétisé selon une méthode analogue à celle décrite à l'exemple 2 en utilisant comme réactif de départ le composé 36 remplaçant le 5,5-diméthyl-3-[4-nitro-3-(trifluorométhyl)phényl]-1-{9-[(4,4,5,5,5-pentafluoropentyl)thio]nonyl}imidazolidine-2,4-dione. Le composé attendu est obtenu sous forme d'une huile jaune pâle avec un rendement de 88 % .
¹H NMR 400MHz (DMSO-*d*₆) δ :. 8,32 (d, 1H, Ph) ; 8,20 (d, 1H, Ph) ; 8,07 (m, 1H, Ph) ; 3,76 (m, 2H, CH₂) ; 3,55 (m, 8H, 8 x CH₂) ; 3,00 (m, 1H, CH) ; 2,85 (m, 3H, 2 x CH₂ + CH) ; 2,35 (m, 2H, CH₂) 1,89 (m, 2H, CH₂) ; 1,48 (s, 6H, 2CH₃).

### Exemple 38: N-[4-(4,4-dimethyl-2,5-dioxo-3-[2-(2-{2-[(4,4,5,5,5-pentafluoropentyl)sulfanyl]ethoxy}ethoxy)ethyl]imidazolidin-1-yl}-2-(trifluoromethyl)phenyl]acetamide

### 38.1) 3-[4-amino-3-(trifluoromethyl)phenyl]-5,5-dimethyl-1-[2-(2-{2-[(4,4,5,5,5-pentafluoropentyl)sulfanyl]ethoxy}ethoxy)ethyl]imidazolidine-2,4-dione

Le composé 38 décrit ci-dessous a été synthétisé selon une méthode analogue à celle décrite à l'exemple 16 en utilisant comme réactif de départ le composé 36 remplaçant le 5,5-diméthyl-3-[4-nitro-3-(trifluorométhyl)phényl]-1-{9-[(4,4,5,5,5-pentafluoropentyl)thio]nonyl}imidazolidine-2,4-dione. Le composé attendu est obtenu sous forme d'une huile jaune pâle avec un rendement de 88 % .
MH+ expérimental = 596,1 ; M théorique = 595,2

### 38.2) N-[4-{4,4-dimethyl-2,5-dioxo-3-[2-(2-{2-[(4,4,5,5,5-pentafluoropentyl)sulfanyl]ethoxy}ethoxy)ethyl]imidazolidin-1-yl}-2-(trifluoromethyl)phenyl]acetamide

Le composé 38 décrit ci-dessous a été synthétisé selon une méthode analogue à celle décrite à l'exemple 17 en utilisant comme réactif de départ le composé 38.1 remplaçant le 3-[4-amino-3-(trifluorométhyl)phényl]-5,5-diméthyl-1-{9-[(4,4,5,5,5-pentafluoropentyl)thio]nonyl}imidazolidine-2,4-dione. Le composé attendu est obtenu sous forme d'une huile jaune pâle avec un rendement de 88 %.
¹H NMR 400MHz (DMSO-*d*₆) δ: 9.64 (s, 1H, Ph) ;. 7,80 (d, 1H, Ph) ; 7,68 (m, 1H, Ph) ; 7,59 (m, 1H, Ph) ; 3,52 (m, 10H, 5 x CH₂); 2,63 (m, 4H, 2 x CH₂); 2,35 (m, 2H, CH₂); 2.06 (s, 3H, CH₃); 1,75 (m, 2H, CH₂); 1,45 (s, 6H, 2CH₃).

### Exempte 39 : N-[4-{4,4-dimethyl-2,5-dioxo-3-[2-(2-{2-[(4,4,5,5,5-pentafluoropentyl)sulfinyl]ethoxy}ethoxy)ethyl]imidazolidin-1-yl}-2-(trifluoromethyl)phenyl]acetamide

Le composé 39 décrit ci-dessous a été synthétisé selon une méthode analogue à celle décrite à l'exemple 2 en utilisant comme réactif de départ le composé de l'exemple 38 remplaçant le composé de l'exemple 1. Le composé attendu est obtenu sous forme d'une huile jaune pâle avec un rendement de 75 %.
¹H NMR 400MHz (DMSO-*d*₆) δ : 9.64 (s, 1H, Ph);. 7,81 (s, 1H, Ph) ; 7,68 (m, 1H, Ph) ; 7,59 (m, 1H, Ph) ; 3,59 (m, 10H, 5 x CH₂); 2,98 (m, 1H, CH); 2,86 (m, 3H, CH₂ + CH) ; 2.38 (m, 2H, CH₂); 2.06 (s, 3H, CH₃); 1,91 (m, 2H, CH₂); 1,45 (s, 6H, 2CH₃).

### Exemple 40 : N-[4-(4,4-dimethyl-2,5-dioxo-3-{9-[(4,4,5,5,5-pentafluoropentyl)sulfinyl]nonyl}imidazolidin-1-yl)-2-(tritluoromethyl)phenyl]-N-methylacetamide

Sous argon, on ajoute NaH (à 60 %) (6 mg, 0,16 mmole) à une solution de N-[4-(4,4-diméthyl-2,5-dioxo-3-{9-[(4,4,5,5,5-pentafluoropentyl)sulfinyl]-nonyl}imidazolidin-1-yl)-2 (trifluorométhyl)phényl]acetamide (100 mg, 0.15 mmole) (préparé selon l'exemple 18) dans du DMF anhydre (2 ml). Un dégagement gazeux accompagne le changement de coloration du milieu réactionnel. L'agitation est maintenue 1 heure à 23° C avant d'ajouter le l'iodure de méthyle (10 µl, 0,16 mmole). Après 1 heure de réaction on rajoute une même quantité d'hydrure de sodium et l'iodure de méthyl que précedemment puis on agite encore 3 heures à 23°C. Le milieu réactionnel est versé dans de l'eau (25 ml) et extrait par AcOEt (2 x 25 ml). Les phases organiques sont rassemblées et lavées successivement par de l'eau (25 ml) et de la saumure (25 ml). Après séchage sur Na₂SO₄, la solution organique est filtrée et concentrée sous vide. Le résidu d'évaporation est purifié sur une colonne de silice (éluant : CH₂Cl₂/MeOH : 95/5 jusqu'à 90/10). Le composé attendu est obtenu sous forme d'une huile incolore avec un rendement de 72 % (72 mg).
¹H NMR 400MHz (DMSO-*d*₆) δ : 7,98 (d, 1H, Ph) ; 7,86 (m, 1H, Ph) ; 7,73 (d, 1H, Ph); 3,28 (m, 2H, NCH₂); 3 ,08 (s, 3H, CH₃); 2,85 (m, 4H, 2CH₂); 2,38 (m, 2H, CH₂); 1,90 (m, 2H, CH₂); 1,66 (s, 5H, CH₃-CO, CH₂); 1,44 (s, 6H, 2 x CH₃); 1,30 (m, 4H, 2 x CH₂); 1,30 (m, 8H, 4 x CH₂).

### Exemple 41 : N-[4-(4,4-dimethyl-2,5-dioxo-3-{9-[(4,4,5,5,5-pentafluoropentyl) sulfinyl]nonyl}imidazotidin-1-yl)-2-(trifluoromethyl)phenyl]methanesulfonamide

### 41.1)N-[4-(4,4-dimethyl-2,5-dioxo-3-{9-[(4,4,5,5,5-pentafluoropentyl) sulfinyl]nonyl}imidazolidin-1-yl)-2-(trifluoromethyl)phenyl]-N-(methylsulfonyl)methanesulfonamide

A une solution de l'exemple 16 (151 mg, 0,25 mmole) dans CH₂Cl₂ anhydre (5 ml) on ajoute goutte-à-goutte du chlorure de sulfonyle (193 µl, 10 éq.) à 23° C. L'agitation est maintenue 15 heures à 23° C puis on rajoute du chlorure de sulfonyle (0.58 ml, 30 éq.) et de la diisopropyléthylamine (1.75 ml, 40 éq.). L'agitation est maintenue 3 heures à 23°C puis le mélange réactionnel est concentré à sec sous vide. Le résidu d'évaporation est dilué un mélange de solvant tetrahydrofurane /methanol/eau 1/1/1 (3 ml) et on ajoute de l'hydroxyde de litium (25 mg, 1 mmol). L'agitation est maintenue 15 heures à 23° C puis de l'hydroxyde de litium est ajouté (50 mg, 2 mmol.) dans un mélange de solvant tetrahydrofurane/methanol/eau 2/2/2 (6 ml). Après 2 heures à 23°C le milieu réactionnel est versé dans de l'eau (25 ml) et extrait par AcOEt (2 x 25 ml). Les phases organiques sont rassemblées et lavées successivement par de l'eau (25 ml) et de la saumure (25 ml). Après séchage sur Na₂SO₄, la solution organique est filtrée et concentrée sous vide. Le résidu d'évaporation est purifié sur une colonne de silice (éluant : Heptane/AcOEt : 7/3 jusqu'à 5/5). Après collection et concentration des fractions pures, le composé attendu est obtenu sous forme d'une huile incolore qui cristallise lentement avec un rendement de 47 % (81 mg).
MH+ expérimental = 684,1 ; M théorique = 683,2

### 41.2) N-[4-(4,4-dimethyl-2,5-dioxo-3-{9-[(4,4,5,5,5-pentafluoropentyl)sulfinyl]nonyl}imidazolidin-1-yl)-2-(trifluoromethyl)phenyl]methanesulfonamide

Le composé 41 décrit ci-dessous a été synthétisé selon une méthode analogue à celle décrite à l'exemple 2 en utilisant comme réactif de départ le composé de l'exemple 41.1 remplaçant le composé de l'exemple 1. Le composé attendu est obtenu sous forme d'une huile jaune pâle avec un rendement de 71 %.
¹H NMR 400MHz (DMSO-*d*₆) δ : 9.54 (s, 1H, Ph);. 7,84 (d, 1H, Ph) ; 7,72 (m, 2H, Ph) ; 3,28 (m, 2H, CH₂); 3,13 (s, 3H, CH₃); 2,75 (m, 4H, 2CH₂); 2.38 (m, 2H, CH₂); 1.90 (m, 2H, CH₂); 1,61 (m, 4H, 2CH₂); 1,45 (s, 6H, 2CH₃); 1,30 (m, 10H, 5CH₂).

### Exemple 42: N-[4-(4,4-dimethyl-2,5-dioxo-3-{9-[(4,4,5,5,5-pentafluoropentyl)sulfanyl]nonyl}imidazolidin-1-yl)-2-(trifluoromethyl)phenyl]-N²,N²-dimethylglycinamide

Le composé 41 décrit ci-dessous a été synthétisé selon une méthode analogue à celle décrite à l'exemple 17 en utilisant comme réactif de départ le chlorure de diméthyl amino acétyle sous sa forme chlorhydrate remplaçant le chlorure d'acétyle. Le composé attendu est obtenu sous forme d'une huile jaune pâle avec un rendement de 83 % .
¹H NMR 400MHz (DMSO-*d*₆) δ: 9.85 (s, 1H, Ph) ; 8,19 (d, 1 H, Ph) ; 7,81 (d, 1H, Ph) ; 7,57 (m, 1H, Ph) ; 3,27 (s, 2H, CH₂) ; 3,12 (m, 2H, CH₂) ; 2,57 (m, 2H, CH₂) ; 2.46 (m, 2H, CH₂) ; 2.30 (m, 8H, 2CH₃, CH₂) ; 1.75 (m, 2H, CH₂) ; 1,60 (m, 2H, CH₂) ; 1,45 (s, 8H, 2CH₃, CH₂); 1,30 (m, 10H, 5CH₂).

### Exemple 43a : N-[4-(4,4-dimethyl-2,5-dioxo-3-{9-[(4,4,5,5,5-pentafluoropentyl)sulfinyl]nonyl}imidazolidin-1-yl)-2-(trifluoromethyl)phenyl]-N²,N²-dimethylglycinamide

Le composé 4 3a décrit ci-dessous a été synthétisé selon une méthode analogue à celle décrite à l'exemple 2 en utilisant comme réactif de départ le composé de l'exemple 42 remplaçant le composé de l'exemple 1. Le composé attendu est obtenu sous forme d'une huile jaune pâle avec un rendement de 65%.
¹H NMR 400MHz (DMSO-*d*₆) δ : 9.85 (s, 1H, Ph) ; 8,19 (d, 1H, Ph) ; 7,81 (d, 1H, Ph) ; 7,70 (m, 1H, Ph) ; 3,19 (s, 2H, CH₂) ; 2,75 (m, 4H, 2CH₂) ; 2.30 (m, 8H, 2CH₃, CH₂) ; 1.90 (m, 2H, CH₂); 1,60 (m, 4H, 2CH₂); 1,45 (s, 8H, 2CH₃, CH₂); 1,30 (m, 10H, 5CH₂).

### Exemple 43b : Chlorhydrate de N-[4-(4,4-dimethyl-2,5-dioxo-3-{9-[(4,4,5,5,5-pentafluoropentyl)sulfinyl]nonyl}imidazolidin-1-yl)-2-(trifluoromethyl)phenyl]-N²,N²-dimethylglycinamide

A une solution de l'exemple 43a (639 mg, 0,904 mmole) dans de l'éther anhydre (35 ml) on ajoute goutte-à-goutte une solution d'acide chlorhydrique dans l'éther 2N (904 µl, 2 éq.) à 23° C. L'agitation est maintenue 15 heures à 23° C puis le mélange réactionnel est concentré à sec sous vide en ayant été repris deux fois avec de l'éther (2 x 20 ml) puis deux fois à l'isopentane (2 x 20 ml). Le résidu d'évaporation est séché sous vide à 55°C. Le composé attendu est obtenu sous forme d'une poudre de couleur beige avec un rendement de 93 % (624 mg).
¹H NMR 400MHz (DMSO-*d*₆) δ : 10.52 (s, 1H, Ph) ; 10.06 (s, 1H, Ph) ;. 7,89 (s, 1H, Ph) ; 7,78 (m, 1H, Ph) ; 7,67 (m, 1H, Ph) ; 4,14 (m, 2H, CH₂) ; 2,84 (s, 6H, 2CH₃) ; 2,70 (m, 4H, 2CH₂) ; 2,36 (m, 2H, CH₂) ; 1,94 (m, 2H, CH₂) ; 1,60 (m, 4H, 2CH₂) ; 1,45 (s, 8H, 2CH₃, CH₂) ; 1,30 (m, 10H, 5CH₂).

### Exemple 44 : N-[(1Z)-(9-{5,5-dimethyl-3-[4-nitro-3-(trifluoromethyl)phenyl]-2,4-dioxoimidazolidin-1-yl}nonyl)(4,4,5,5,5-pentafluoropentyl)-λ⁴-sulfanylidene]-2,2,2-trifluoroacetamide

A une solution de l'exemple 1 (300 mg, 0,48 mmole) dans CH₂Cl₂ anhydre (5 ml) on ajoute du 2,2,2-trifluoroacétamide (106 mg, 0.94 mmole), de l'oxyde de magnésium (76 mg, 4 éq.), de l'acétate de rhodium sous forme dimère (5,2 mg, 0.025 éq.) et du diacétate iodobenzène (228 mg, 1.5 éq.) à 23° C. L'agitation est maintenue 6 heures à 23° C puis le mélange réactionnel est filtré sous vide. Le filtrat est versé dans de l'eau (25 ml) et extrait par AcOEt (2 x 25 ml). Les phases organiques sont rassemblées et lavées successivement par de l'eau (25 ml) et de la saumure (25 ml). Après séchage sur Na₂SO₄, la solution organique est filtrée et concentrée sous vide. Le résidu d'évaporation est purifié sur une colonne de silice (éluant : Heptane/AcOEt : 5/5 jusqu'à 3/7). Après collection et concentration des fractions pures, le composé attendu est obtenu sous forme d'une huile incolore avec un rendement de 34 % (77 mg).
¹H NMR 400MHz (DMSO-*d*₆) δ :. 8,32 (d, 1H, Ph) ; 8,20 (d, 1H, Ph) ; 8,06 (dd, 1H, Ph) ; 3,30 (m, 2H, NCH₂) ; 3,10 (m, 4H, CH₂S(=NCOCF₃)CH₂); 2,40 (m, 2H, CH₂) ; 1,92 (m, 2H, CH₂) ; 1,65 (m, 4H, 2 x CH₂) ; 1,48 (s, 6H, 2 x CH₃) ; 1,42 (m, 2H, CH₂) ; 1,34 (m, 8H, 4 x CH₂).

### Exemple 45 : N-[(9-{5,5-dimethyl-3-[4-nitro-3-(trifluoromethyl)phenyl]-2,4-dioxoimidazolidin-1-yl}nonyl)(oxido)(4,4,5,5,5-pentafluoropentyl)-λ⁴-sulfanylidene]-2,2,2-trifluoroacetamide

A une solution de l'exemple 2 (109 mg, 0,17 mmole) dans CH₂Cl₂ anhydre (5 ml) on ajoute du 2,2,2-trifluoroacétamide (38 mg, 0.33 mmole), de l'oxyde de magnésium (27 mg, 4 éq.), de l'acétate de rhodium sous forme dimère (1,8 mg, 0.025 éq.) et du diacétate d'iodobenzène (81 mg, 1.5 éq.) à 23° C. L'agitation est maintenue 6 heures à 23° C puis le mélange réactionnel est filtré sous vide. Le filtrat est versé dans de l'eau (25 ml) et extrait par AcOEt (2 x 25 ml). Les phases organiques sont rassemblées et lavées successivement par de l'eau (25 ml) et de la saumure (25 ml). Après séchage sur Na₂SO₄, la solution organique est filtrée et concentrée sous vide. Le résidu d'évaporation est purifié sur une colonne de silice (éluant : Heptane/AcOEt : 5/5 jusqu'à 2/8). Après collection et concentration des fractions pures, le composé attendu est obtenu sous forme d'une huile incolore avec un rendement de 88 % (114 mg).
¹H NMR 400MHz (DMSO-*d*₆) δ :. 8,32 (d, 1H, Ph) ; 8,20 (d, 1H, Ph) ; 8,06 (dd, 1H, Ph) ; 3,76 (m, 4H, CH₂S(=O)(=NCOCF₃)CH₂); 3,30 (m, 2H, NCH₂); 2,48 (m, 2H, CH₂) ; 2,02 (m, 2H, CH₂) ; 1,70 (m, 4H, 2 x CH₂) ; 1,46 (s, 6H, 2 x CH₃) ; 1,42 (m, 2H, CH₂) ; 1,34 (m, 8H, 4 x CH₂).

### Exemple 46 : 5,5-dimethyl-3-[4-nitro-3-(trifluoromethyl)phenyl]-1-{9-[S-(4,4,5,5,5-pentafluoropentyl)sulfonimidoyl]nonyl}imidazolidine-2,4-dione

A une solution de l'exemple 45 (38 mg, 0,05 mmole) dans MeOH anhydre (0.5 ml) on ajoute du carbonate de potassium (35 mg, 0.25 mmole) à 23° C. L'agitation est maintenue 15 heures à 23° C puis le mélange réactionnel est filtré sous vide. Le filtrat est versé dans de l'eau (25 ml) et extrait par AcOEt (2 x 25 ml). Le filtrat est concentré sous vide. Le résidu d'évaporation est purifié sur une colonne de silice (éluant 2 x 10 ml avec AcOEt /CH₂Cl₂ : 5/5 puis 10 ml AcOEt /Heptane avec 7.5/2.5). Après collection et concentration des fractions pures, le composé attendu est obtenu sous forme d'une huile incolore avec un rendement de 67 % (22 mg).
¹H NMR 400MHz (DMSO-*d*₆) δ :. 8,28 (d, 1H, Ph) ; 8,17 (d, 1H, Ph) ; 8,03 (dd, 1H, Ph) ; 3,65 (s, 1H, NH) ; 3,30 (m, 2H, NCH₂) ; 3,10 (m, 4H, CH₂S(=NH)CH₂) ; 2,31 (m, 2H, CH₂) ; 1,92 (m, 2H, CH₂) ; 1,65 (m, 4H, 2 x CH₂) ; 1,43 (s, 6H, 2 x CH₃) ; 1,42 (m, 2H, CH₂) ; 1,34 (m, 8H, 4 x CH₂).

### Etude pharmacologique des composés selon l'invention

### Mesures des activités anti-prolifératives :

### 1. Activité anti-proliférative sur LNCaP en milieu complet

L'activité anti-proliférative des composés de la présente invention est déterminée sur LNCaP en milieu complet en appliquant la procédure expérimentale suivante :

Le type cellulaire LNCaP (ATCC, 1740) est issu d'un carcinome de prostate exprimant le récepteur aux androgènes, cette lignée est hormono-dépendante.

L'entretien de la lignée LNCaP est réalisé en milieu de culture complet : RPMI, 10 % de sérum de veau foetal, 2 mM glutamine, 100 U/ml pénicilline, 0,1 mg/ml de streptomycine et HEPES 0,01 M, pyruvate de sodium 1mM, 40 % de D-glucose.
- Ensemencement des plaques :
   La lignée LNCaP est ensemencée à 20000 cellules/puits dans 90 µl de milieu complet en plaques 96 puits coatées poly-D-lysine (Biocoat , Costar) .
- Traitement des cellules : 24h après l'ensemencement, les cellules sont traitées avec 10 µl/puits de composé dilué dans le milieu de culture. Des expériences d'effet dose du composé sont effectuées sur une échelle de 1 nM à 100 µM. Les concentrations utilisées sont les suivantes : 1nM/10/30/100/300/1000/3000/10000/100000 nM. La Testostérone (SIGMA T1500) est utilisé comme référence et testée aux mêmes concentrations. Les cellules sont incubées 144 h à 37° C, 5 % CO₂.
- Lecture : A J6, 10 µL du réactif "cell proliferation WST-1" (Roche ref 1644807) est ajouté dans chaque puits. Après une incubation de 2 heures à 37° C, 5 % CO₂, l'absorbance à 450 nm est mesuré par spectrophotomètrie (Envision, Perkin Elmer).
- Résultats : Les expériences sont réalisées en duplicat et les meilleurs composés sont testés deux fois. Une valeur de concentration inhibant de 50 % la prolifération cellulaire (CI₅₀) est calculée.

Les composés des exemples suivants ont des CI₅₀ inférieures à 2000 nM sur les cellules LNCaP en culture : 1, 2, 3, 4, 5, 7, 9, 11, 12, 13, 15, 17, 18, 20, 22, 24, 28, 30, 31, 32, 34, 35, 36, 37, 38, 40, 41, 42, 43, 44, 45 et 46.

Parmi eux, les composés des exemples suivants ont des CI₅₀ inférieures à 1000 nM sur les cellules LNCaP en culture : 2, 3, 9, 12, 13, 15, 17, 18, 20, 22, 24, 32, 34, 35, 36, 38, 41, 43, 44, 45 et 46.

Les composés des exemples suivants ont des CI₅₀ inférieures à 500 nM sur les cellules LNCaP en culture : 2, 9, 12, 13, 18, 35, 36, 38, 43, 44 et 45.

### 2. Activité antiproliférative sur LNCaP en milieu sans stéroïde :

L'activité anti-proliférative des composés de la présente invention est déterminée sur LNCaP en milieu sans stéroïde.

L'entretien de la lignée LNCaP est réalisé dans les conditions habituelles en RPMI, 10 % de sérum de veau foetal, 2 mM de glutamine, 100 U/ml de pénicilline, 0,1 mg/ml de streptomycine et HEPES 0,01M, pyruvate de sodium 1 mM, 40 % de D-glucose.

Pour l'étude en conditions sans stéroïde, 24 heures avant l'ensemencement le milieu de culture des cellules est éliminé. Les cellules sont lavées avec du PBS, puis incubées en présence de milieu RPMI sans rouge de phénol, 10 % de sérum de veau foetal sans stéroïde (traitement au charbon), 2 mM de glutamine, 100 U/ml de pénicilline, 0,1 mg/ml de streptomycine et HEPES 0,01M, pyruvate de sodium 1 mM, 40 % de D-glucose.
- Ensemencement des plaques :
   La lignée LNCaP est ensemencée à 20000 cellules/puits dans 90 µl de milieu RPMI avec 10% de sérum de veau foetal sans stéroide en plaques 96 puits coatées poly-D-lysine (Biocoat, Costar).
- Traitement des cellules : 24h après l'ensemencement, les cellules sont traitées avec 10 µl/puits de composé dilué dans le milieu de culture. Des expériences d'effet dose du composé sont effectuées sur une échelle de 1 nM à 100 µM. Les concentrations utilisées sont les suivantes : 1nM/10/30/100/300/1000/3000/10000/100000 nM. La Testosterone (SIGMA T1500) est utilisée comme référence et testée aux mêmes concentrations. Les cellules sont incubées 144 h à 37° C, 5 % CO₂.
- Lecture : A J6, 10 µL de réactif "cell prolifération WST-1" (Roche ref 1644807) est ajouté dans chaque puits. Après une incubation de 2 à 4 heures à 37° C, 5 % CO₂, l'absorbance à 450 nm est mesuré par spectrophotomètrie (Envision, Perkin Elmer).
- Résultats : Les expériences sont réalisées en duplicat et les meilleurs composés sont testés deux fois. Une valeur de concentration inhibant de 50 % la prolifération cellulaire (CI₅₀) est calculée.

Les produits des exemples 1 à 46 ne présentent pas d'effet agoniste sur LNCaP en milieu sans stéroïde.

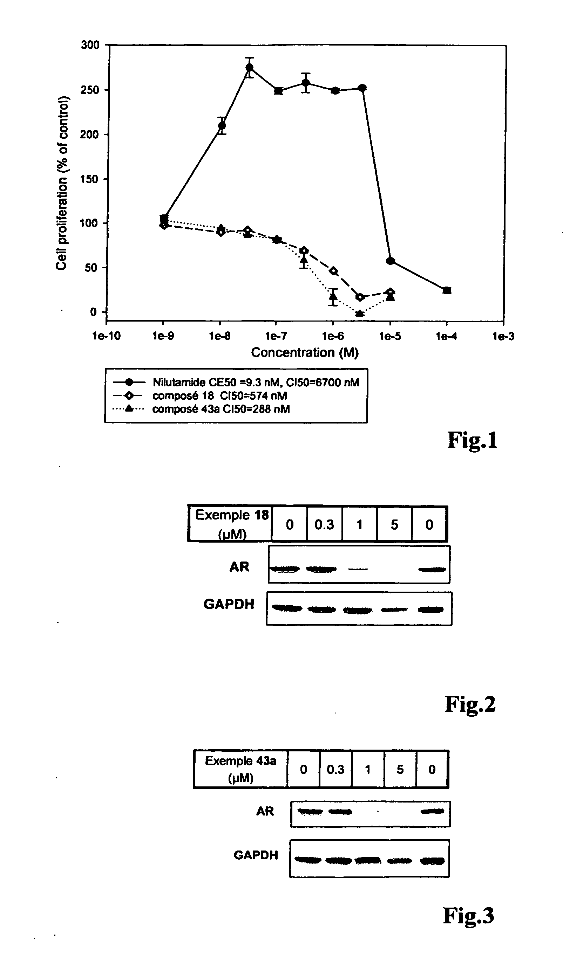

La figure 1 montre l'Effet des composés des exemples 18 et 43a sur la prolifération cellulaire des LNCaP cultivées en milieu sans stéroïde.

Les composés 18 et 43a montrent de façon surprenante une activité anti-proliférative sur les cellules LNCaP sans effet agoniste. Au contraire, le nilutamide présente un profil biphasique avec une activité agoniste à faible concentration suivie par une activité inhibitrice à forte concentration.

### 3. Activité antiproliférative sur DU-145 en milieu complet :

L'activité anti-proliférative des composés de la présente invention est déterminée sur DU-145 en milieu complet.

L'entretien de la lignée DU-145 est réalisée en DMEM, 10 % de sérum de veau foetal, 2 mM de glutamine, 100 U/ml de pénicilline, 0,1 mg/ml de streptomycine.
- Ensemencement des plaques :
   La lignée DU145 est ensemencée à 400 ou 800 cellules/puits dans 90 µl de milieu DMEM complet en plaques 96 puits (TPP).
- Traitement des cellules : 24h après l'ensemencement, les cellules sont traitées avec 10 µl/puits de composé dilué dans le milieu de culture. Des expériences d'effet dose du composé sont effectuées sur une échelle de 1 nM à 100 µM. Les concentrations utilisées sont les suivantes : 1nM/10/30/100/300/1000/3000/10000/100000 nM. Les cellules sont incubées 144 h à 37° C, 5 % CO₂.
- Lecture : A J6, 10 µL de réactif "cell prolifération WST-1" (Roche ref 1644807) est ajouté dans chaque puits. Après une incubation de 2 à 4 heures à 37° C, 5 % CO₂, l'absorbance à 450 nm est mesuré par spectrophotomètrie (Envision, Perkin Elmer).
- Résultats : Les expériences sont réalisées en duplicat et les meilleurs composés sont testés deux fois. Une valeur de concentration inhibant de 50 % la prolifération cellulaire (CI₅₀) est calculée.
- Les composés des exemples suivants ont des CI₅₀ supérieures à 10000 nM sur les cellules DU145 en culture : 1, 2, 3, 4, 5, 7, 8, 9, 11, 14, 15, 16, 17, 18, 20, 22, 24, 26, 28, 30, 31, 32, 34, 35, 36, 37, 38, 39, 40, 41, 42, 43, 44 et 45.

### 4. Mesure de la disparition du récepteur aux androgènes

Il s'agit de montrer que les composés diminuent le niveau d'expression protéique du récepteur aux androgènes.

Les cellules de la lignée LNCaP sont ensemencées à raison de 2,5 millions de cellules en boite de Pétri 10cm en RPMI, 10 % de sérum de veau foetal, 2 mM de glutamine, 100 U/ml de pénicilline, 0,1 mg/ml de streptomycine et HEPES 0,01M, pyruvate de sodium 1 mM, 40 % de D-glucose. 4 jours après, les cellules sont traitées par le composé à tester. 72 heures après le traitement les cellules sont lysées en tampon de lyse (Tris pH7.4 50 mM, NaCl 150 mM, EDTA 1 mM, NaF 20 mM, Na₂VO₃ 100 mM, NP40 0.5%, Triton X-100 1%, EGTA 1mM, Pefabloc, protéase inhibiteur cocktail 11836170001 RocheDiagnostics, Phosphatase inhibitor cocktail set II Calbiochem). Les cellules sont ensuite grattées et le lysat transféré en tubes QIAshredder (cat -n°79656 Qiagen) pour centrifugation à 13 000 rpm pendant 15 min à 4°C. Le surnageant est transféré dans les tubes QIAshredder pour une seconde centrifugation à 13000 rpm pendant 5 min afin d'éliminer complètement les filaments d'ADN. Après congélation à -80°C, la concentration protéique est déterminée (Bio-Rad DC protein assay kit) et ajustée entre 10 et 20µg par puits. Le tampon de charge (sample loading buffer 3 X ref 7722 Cell signaling technology) additionné de béta-mercaptoéthanol à 1% et de DTT à 50 mM est ajouté aux échantillons qui sont ensuite chauffés pendant 10 min à 90°C. Les échantillons sont déposés sous un volume de 20 µl sur des gels NuPAGE 4-12% Bis-Tris gel (cat N° NP0322BOX, Invitrogen). La migration a lieu en tampon MOPS (Invitrogen) et s'effectue pendant 1 heure à 180V. Les protéines sont transférées sur une membrane de nitrocellulose (Hybond ECL RPN78D, GE Healthcare) dans des conditions semi-sèches, en présence de tampon de transfert (NP0006-1, Invitrogen) pendant 45 min à 15V. La membrane est ensuite bloquée pendant 1 heure dans du tampon de blocage (Non-fat dry milk, cat 170-6404, Biorad) à 5% dans du TBS 0.1 % Tween 20. Puis elle est incubée à 4°C toute la nuit en présence d'anticorps primaire dirigé contre l'androgen receptor (AR441, sc-7305, Santa Cruz) dilué au 1/2000^{ème} dans du tampon de blocage ainsi qu'en présence d'anticorps primaire dirigé contre la GAPDH (Cat.MAB374, Millipore) dilué au 1/20000^{ème} dans du tampon de blocage (contrôle de charge en protéines). La membrane est ensuite lavée 3 fois dans du tampon de lavage (TBS, 0.1 % Tween 20). La membrane est ensuite incubée en présence d'anticorps secondaire anti-immunoglobuline de souris couplée à l'HRP (Goat anti-mouse IgG-HRP, sc 2031, Santa Cruz) dilué au 1/5000^{éme} dans du tampon de blocage.

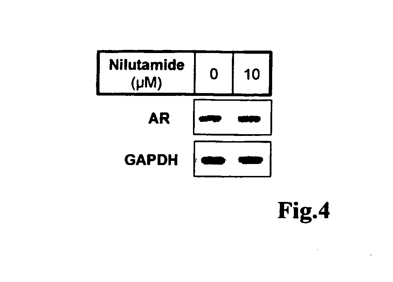

La membrane est ensuite lavée 3 fois dans du tampon de lavage. Les protéines sont révélées par électrochimioluminescence (western Blotting detection system ECL+, Amersham) qui est détectée soit à l'aide de films photographiques (Biomax light, Sigma), soit par un système d'acquisition de la chimioluminescence (G :Box, Syngene).Les figures 2 et 3 montre l'effet des composés 18 et 43a sur l'expression du récepteur aux androgènes. La figure 4 montre l'absence d'effet du nilutamide sur l'expression du récepteur aux androgènes.

## Revendications

1. Composé de formule générale (**I**) 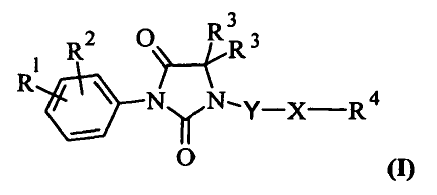 sous forme racémique, d'énantiomère ou toutes combinaisons de ces formes et dans laquelle,
R¹ et R² représentent indépendamment un atome d'halogène, ou bien un radical alkyle, haloalkyle, alkoxy, cyano, nitro, amino, alkylamino, dialkylamino, -NR⁸-CO-R⁵, -NR⁸-SO₂-R⁵, -NR⁸-CO-(CH₂)ₙ-NR⁶ R⁷, -NR⁸-SO₂-(CH₂)ₙ-NR⁶R⁷ ou -CO-NH₂ ;
n représente un entier choisi parmi 0, 1, 2, 3, 4, 5 et 6 ;
R⁵ représente un radical alkyle, aryle, ou hétéroaryle ;
R⁶ et R⁷ représentent indépendamment un atome d'hydrogène, un radical alkyle ou alkyloxycarbonyl ;
R⁸ représente un atome d'hydrogène ou un radical alkyle ;
R³ représente un radical alkyle ou un atome d'hydrogène ; ou bien les deux radicaux R³ forment ensemble avec l'atome de carbone auquel ils se rattachent un radical cycloalkyle comportant de 3 à 6 chaînons ;
R⁴ représente un radical haloalkyle de 2 à 10 atomes de carbone ;
Y représente une chaîne alkylène de 2 à 14 atomes de carbone, linéaire ou ramifiée, cette chaîne pouvant être saturée ou insaturée, et pouvant contenir un ou plusieurs chaînons -O-supplémentaires ;
X représente -S-, -SO-, -SO₂-, -S=N(R⁹)- ou -S(O)=N(R⁹)- ;
R⁹ représente un atome d'hydrogène ou un radical haloalkylcarbonyle,
ou un sel pharmaceutiquement acceptable de ce dernier.

2. Composé selon la revendication 1, **caractérisé en ce que** R¹ représente un atome d'halogène, ou bien un radical alkyle, alkoxy, cyano, nitro, amino, alkylamino, dialkylamino, -NR⁸-CO-R⁵, -NR⁸-SO₂-R⁵, -NR⁸-CO-(CH₂)ₙ-NR⁶R⁷, -NR⁸-SO₂-(CH₂)ₙ-NR⁶ R⁷, ou -CO-NH₂; R² représente un atome d'halogène, un radical alkyle ou haloalkyle ou un sel pharmaceutiquement acceptable de ce dernier.

3. Composé selon l'une des revendications 1 ou 2, **caractérisé en ce que** R⁵ représente un radical alkyle.

4. Composé selon l'une des revendications 1 à 3, **caractérisé en ce que** R³ représente un radical alkyle.

5. Composé selon l'une des revendications 1 à 4, **caractérisé en ce que** R⁴ représente un radical haloalkyle comportant de 4 à 6 atomes de carbone et 3 à 9 atomes de fluor ; et Y représente une chaîne alkylène de 5 à 10 atomes de carbone.

6. Composé selon l'une des revendications 1 à 5, **caractérisé en ce que** R¹ est en position para.

7. Composé selon l'une des revendications 1 à 6, **caractérisé en ce que** R² est en position meta.

8. Composé selon l'une des revendications 1 à 7, **caractérisé en ce que** :
R¹ représente un radical cyano, nitro, amino, -NR⁸-CO-R⁵, -NR⁸-SO₂-R⁵, -NR⁸-CO-(CH₂)ₙ-NR⁶R⁷, -NR⁸-SO₂-(CH₂)ₙ-NR⁶ R⁷, ou -CO-NH₂ ;
R² représente un radical alkyle ou haloalkyle ;
R⁵ représente un radical alkyle ;
R⁶ et R⁷ représentent indépendamment un atome d'hydrogène, un radical alkyle ou alkyloxycarbonyle ;
R³ représente un radical alkyle ou bien les deux radicaux R³ forment ensemble avec l'atome de carbone auquel ils se rattachent un radical cycloalkyle comportant de 3 à 6 chaînons ;
R⁴ représente un radical haloalkyle comportant de 4 à 6 atomes de carbone et 3 à 9 atomes de fluor ;
n vaut 0 ou 1
R⁹ représente un atome d'hydrogène ou -COCF₃.

9. Composé selon l'une des revendications 1 à 8, **caractérisé en ce que** R¹ représente un radical cyano, nitro, -NR⁸-CO-R⁵, -NR⁸-SO₂-R⁵, -NR⁸-CO-(CH₂)ₙNR⁶ R⁷, -NR⁸-SO₂-(CH₂)ₙ-NR⁶ R⁷, ou -CO-NH₂ ; n vaut 0 ou 1 ; R⁵ représente un radical alkyle, R⁶ et R⁷ représentent indépendamment un atome d'hydrogène ou un radical alkyle, et R² représente un radical alkyle ou haloalkyle.

10. Composé selon l'une des revendications précédentes **caractérisé en ce que** R¹ représente un radical nitro ou -NR⁸-CO-R⁵ dans lequel R⁵ représente un radical alkyle.

11. Composé selon l'une des revendications 1 à 10, **caractérisé en ce que** le radical alkyle représente un groupement méthyle et/ou le radical haloalkyle représente un groupement triflurométhyle, ou un radical de formule brute C₅H₆F₅, C₅H₄F₇, C₆H₈F₅, C₆F₆F₇ ou C₆H₄F₉.

12. Composé selon l'une des revendications précédentes **caractérisé en ce que** Y représente une chaîne alkylène de 9 à 10 atomes de carbones.

13. Composé de formule générale (I) **caractérisé en ce qu'**il s'agit d'un composé choisi parmi
- 5,5-diméthyl-3-[4-nitro-3-(trifluorométhyl)phényl]-1-{9-[(4,4,5,5,5-pentafluoropentyl)sulfinyl]nonyl} imidazolidine-2,4-dione
- 5,5-diméthyl-3-[4-nitro-3-(trifluorométhyl)phényl]-1-{10-[(4,4,5,5,5-pentafluoropentyl)sulfinyl]décyl}imidazolidine-2,4-dione
- 5,5-diméthyl-3-[4-nitro-3-(trifluorométhyl)phényl]-1-{9-[(4,4,4-trifluorobutyl)thio]nonyl}imidazolidine-2,4-dione
- 5,5-diméthyl-3-[4-nitro-3-(trifluorométhyl)phényl]-1-{9-[(4,4,4-trifluorobutyl)sulfinyl]nonyl}imidazolidine-2,4-dione
- N-[4-(4,4-diméthyl-2,5-dioxo-3-{9-[(4,4,5,5,5-pentafluoropentyl)sulfinyl]-nonyl} imidazolidin-1-yl)-2-(trifluorométhyl)phényl]acetamide
- N-[4-(4,4-dimethyl-2,5-dioxo-3-{9-[(4,4,5,5,5-pentafluoropentyl)sulfinyl]nonyl} imidazolidin-1-yl)-2-(trifluoromethyl)phenyl]-N2,N2-dimethylglycinamide
- Chlorhydrate de N-[4-(4,4-dimethyl-2,5-dioxo-3-{9-[(4,4,5,5,5-pentafluoropentyl)sulfinyl]nonyl}imidazolidin-1-yl)-2-(trifluoromethyl)phenyl]-N2,N2-dimethylglycinamide
ou un sel pharmaceutiquement acceptable de ce dernier.

14. Composé de formule générale (**I**) **caractérisé en ce qu'**il s'agit du N-[4-(4,4-diméthyl-2,5-dioxo-3-{9-[(4,4,5,5,5-pentafluoropentyl)sulfinyl]-nonyl}imidazolidin-1-yl)-2-(trifluorométhyl)phényl]acetamide ou un sel pharmaceutiquement acceptable de ce dernier.

15. Procédé de préparation d'un composé de formule (**I**) tel que défini à l'une des revendications 1 à 14, **caractérisé en ce qu'**il comprend l'obtention de composés de formule générale (**I.1**) (composé de formule générale (**I**) dans laquelle X représente l'atome de soufre), 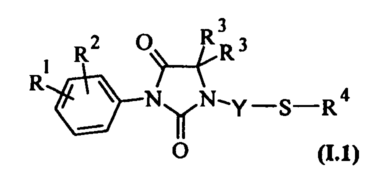 dans laquelle R¹, R², R³, R⁴ et Y sont tels que définis à l'une des revendications 1 à 14,
- soit par condensation des dérivés d'hydantoine de formule générale (**II**) 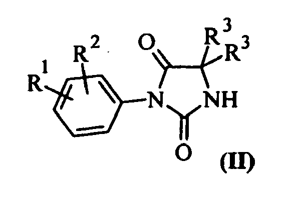 dans laquelle R¹, R², et R³ sont tels que définis à l'une des revendications 1 à 14, en présence d'une base forte à une température comprise entre 25 et 60° C, dans un solvant polaire anhydre, sur les dérivés mésylates de formule générale (**III**), 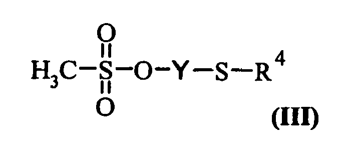 dans laquelle R⁴ et Y sont tels que définis à l'une des revendications 1 à 12,
- soit par le traitement de dérivés thiobenzoyles de formule générale (**V**), 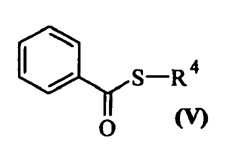
dans laquelle R⁴ est tel que défini à l'une des revendications 1 à 14, par un alcoolate dans un solvant protique polaire suivi de
- l'addition du dérivé halogéné de formule générale (**IV**), 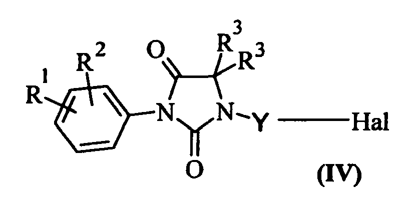
dans laquelle R¹, R², R³ et Y sont tels que définis à l'une des revendications 1 à 14, en solution dans un solvant polaire.

16. Procédé de préparation d'un composé de formule (**I**) selon la revendication 16, comprenant en outre une étape de :
oxydation des composés de formule générale (**I.1**) telle que définie à la revendication 15 en sulfoxyde de formule générale (**I.2**) (composé de formule générale (**I**) dans laquelle X représente le radical -SO-), 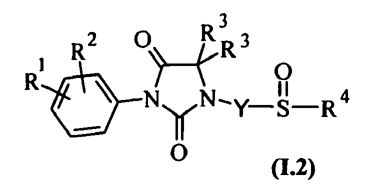
dans laquelle R¹, R², R³, R⁴ et Y sont tels que définis à l'une des revendications 1 à 14,

17. Procédé de préparation d'un composé de formule (I) selon la revendication 16, comprenant en outre une étape de :
oxydation des dérivés sulfoxydes de formule générale **(I.2)** en sulfones de formule générale **(I.3)** (composé de formule générale **(I)** dans laquelle X représente le groupe -SO₂-), 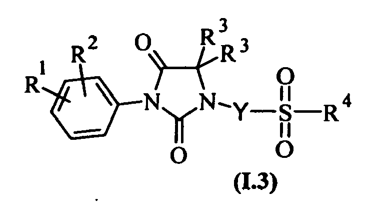
dans laquelle R¹, R², R³, R⁴ et Y sont tels que définis à l'une des revendications 1 à 14.

18. A titre d'intermédiaire, l'un des composés suivants :
- 1-(5-iodopentyl)-5,5-dimethyl-3-[4-nitro-3-(trifluoromethyl)phenyl]imidazolidine-2,4-dione
- 1-(8-iodooctyl)-5,5-dimethyl-3-[4-nitro-3-(trifluoromethyl)phenyl]imidazolidine-2,4-dione
- 1-(9-bromononyl)-5,5-dimethyl-3-[4-nitro-3-(trifluoromethyl)phenyl]imidazolidine-2,4-dione
- 4-[3-(9-bromononyl)-4,4-dimethyl-2,5-dioxoimidazolidin-1-yl]-2-(trifluoromethyl)benzonitrile
- 5,5-dimethyl-3-(3-methyl-4-nitrophenyl)imidazolidine-2,4-dione
- 1-(9-bromononyl)-5,5-dimethyl-3-(3-methyl-4-nitrophenyl)imidazolidine-2,4-dione.

19. En tant que médicament, un composé selon l'une des revendications 1 à 14.

20. Compositions pharmaceutiques contenant, à titre de principe actif, au moins un composé de formule **(I)** tel que défini à l'une des revendications 1 à 14, en association avec un support pharmaceutiquement acceptable.

21. Utilisation d'un composé de formule **(I)** selon l'une des revendications 1 à 14, pour la préparation d'un médicament destiné à traiter les cancers.

22. Utilisation selon la revendication 21 **caractérisé en ce que** le médicament est destiné à traiter un cancer hormono-dépendant.

23. Utilisation selon la revendication 21 **caractérisé en ce que** le médicament est destiné à traiter un cancer exprimant les récepteurs aux androgènes.

24. Utilisation selon l'une des revendications 22 ou 23 **caractérisé en ce que** le médicament est destiné à traiter un cancer du sein ou de la prostate.

## Claims

1. Compound of general formula (**I**) 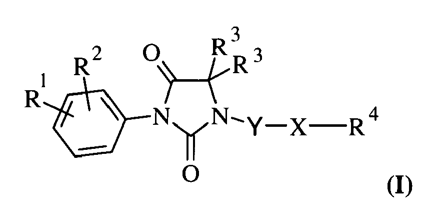 in racemic or enantiomeric form or any combinations of these forms and in which
R¹ and R² represent independently a halogen atom, or an alkyl, haloalkyl, alkoxy, cyano, nitro, amino, alkylamino, dialkylamino, -NR⁸-CO-R⁵, -NR⁸-SO₂-R⁵, -NR⁸-CO-(CH₂)ₙ-NR⁶ R⁷, -NR⁸-SO₂-(CH₂)ₙ-NR⁶R⁷ or -CO-NH₂ radical;
n represents an integer chosen from 0, 1, 2, 3, 4, 5 and 6;
R⁵ represents an alkyl, aryl, or heteroaryl radical;
R⁶ and R⁷ represent independently a hydrogen atom, an alkyl or alkyloxycarbonyl radical;
R⁸ represents a hydrogen atom or an alkyl radical;
R³ represents an alkyl radical or a hydrogen atom; or the two R³ radicals form together with the carbon atom to which they are attached a cycloalkyl radical comprising 3 to 6 members;
R⁴ represents a haloalkyl radical with 2 to 10 carbon atoms;
Y represents a linear or branched alkylene chain with 2 to 14 carbon atoms, this chain being able to be saturated or unsaturated, and being able to contain one or more additional -O- members;
X represents -S-, -SO-, -SO₂-, -S=N(R⁹)- or -S(O)=N(R⁹)-;
R⁹ represents a hydrogen atom or a haloalkylcarbonyl radical,
or a pharmaceutically acceptable salt thereof.

2. Compound according to claim 1, **characterized in that** R¹ represents a halogen atom, or an alkyl, alkoxy, cyano, nitro, amino, alkylamino, dialkylamino, -NR⁸-CO-R⁵, -NR⁸-SO₂-R⁵, -NR⁸-CO-(CH₂)ₙ-NR⁶R⁷, -NR⁸-SO₂-(CH₂)ₙ-NR⁶R⁷, or -CO-NH₂ radical; R² represents a halogen atom, an alkyl or haloalkyl radical or a pharmaceutically acceptable salt thereof.

3. Compound according to one of claims 1 or 2, **characterized in that** R⁵ represents an alkyl radical.

4. Compound according to one of claims 1 to 3, **characterized in that** R³ represents an alkyl radical.

5. Compound according to one of claims 1 to 4, **characterized in that** R⁴ represents a haloalkyl radical comprising 4 to 6 carbon atoms and 3 to 9 fluorine atoms; and Y represents an alkylene chain with 5 to 10 carbon atoms.

6. Compound according to one of claims 1 to 5, **characterized in that** R¹ is in para position.

7. Compound according to one of claims 1 to 6, **characterized in that** R² is in meta position.

8. Compound according to one of claims 1 to 7, **characterized in that**:
R¹ represents a cyano, nitro, amino, -NR⁸-CO-R⁵, -NR⁸-SO₂-R⁵, -NR⁸-CO-(CH₂)ₙ-NR⁶R⁷, -NR⁸-SO₂-(CH₂)ₙ-NR⁶R⁷, or -CO-NH₂ radical;
R² represents an alkyl or haloalkyl radical;
R⁵ represents an alkyl radical;
R⁶ and R⁷ represent independently a hydrogen atom, an alkyl or alkyloxycarbonyl radical;
R³ represents an alkyl radical or the two R³ radicals form together with the carbon atom to which they are attached a cycloalkyl radical comprising 3 to 6 members;
R⁴ represents a haloalkyl radical comprising 4 to 6 carbon atoms and 3 to 9 fluorine atoms;
n is equal to 0 or 1
R⁹ represents a hydrogen atom or -COCF₃.

9. Compound according to one of claims 1 to 8, **characterized in that** R¹ represents a cyano, nitro, -NR⁸-CO-R⁵, -NR⁸-SO₂-R⁵, -NR⁸-CO-(CH₂)ₙNR⁶R⁷, -NR⁸-SO₂-(CH₂)ₙ-NR⁶R⁷, or -CO-NH₂ radical; n is equal to 0 or 1; R⁵ represents an alkyl radical, R⁶ and R⁷ represent independently a hydrogen atom or an alkyl radical, and R² represents an alkyl or haloalkyl radical.

10. Compound according to one of the previous claims **characterized in that** R¹ represents a nitro or -NR⁸-CO-R⁵ radical in which R⁵ represents an alkyl radical.

11. Compound according to one of claims 1 to 10, **characterized in that** the alkyl radical represents a methyl group and/or the haloalkyl radical represents a trifluromethyl group, or a radical of molecular formula C₅H₆F₅, C₅H₄F₇, C₆H₈F₅, C₆H₆F₇ or C₆H₄F₉.

12. Compound according to one of the previous claims **characterized in that** Y represents an alkylene chain with 9 to 10 carbon atoms.

13. Compound of general formula (I) **characterized in that** it is a compound chosen from
- 5,5-dimethyl-3-[4-nitro-3-(trifluoromethyl)phenyl]-1-{9-[(4,4,5,5,5-pentafluoropentyl)sulphinyl]nonyl}imidazolidine-2,4-dione
- 5,5-dimethyl-3-[4-nitro-3-(trifluoromethyl)phenyl]-1-{10-[(4,4,5,5,5-pentafluoropentyl)sulphinyl]decyl imidazolidine-2,4-dione
- 5,5-dimethyl-3-[4-nitro-3-(trifluoromethyl)phenyl]-1-{9-[(4,4,4-trifluorobutyl)thio]nonyl}imidazolidine-2,4-dione
- 5,5-dimethyl-3-[4-nitro-3-(trifluoromethyl)phenyl]-1-{9-[(4,4,4-trifluorobutyl)sulphinyl]nonylimidazolidine-2,4-dione
- N-[4-(4,4-dimethyl-2,5-dioxo-3-{9-[(4,4,5,5,5-pentafluoropentyl)sulphinyl]-nonyl}imidazolidin-1-yl)-2-(trifluoromethyl)phenyl]acetamide
- N-[4-(4,4-dimethyl-2,5-dioxo-3-{9-[(4,4,5,5,5-pentafluoropentyl)sulphinyl]nonyl}imidazolidin-1-yl)-2-(trifluoromethyl)phenyl]-N2,N2-dimethylglycinamide
- N-[4-(4,4-dimethyl-2,5-dioxo-3-{9-[(4,4,5,5,5-pentafluoropentyl)sulphinyl]nonyl}imidazolidin-1-yl)-2-(trifluoromethyl)phenyl]-N2,N2-dimethylglycinamide hydrochloride
or a pharmaceutically acceptable salt thereof.

14. Compound of general formula (I) **characterized in that** it is N-[4-(4,4-dimethyl-2,5-dioxo-3-{9-[(4,4,5,5,5-pentafluoropentyl)sulphinyl]-nonyl}imidazolidin-1-yl)-2-(trifluoromethyl)phenyl]acetamide or a pharmaceutically acceptable salt thereof.

15. Process for the preparation of a compound of formula (**I**) as defined in one of claims 1 to 14, **characterized in that** it comprises obtaining compounds of general formula (**I.1**) (compound of general formula (**I**) in which X represents the sulphur atom), 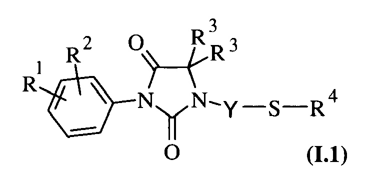 in which R¹, R², R³, R⁴ and Y are as defined in one of claims 1 to 14,
- either by condensation of the hydantoin derivatives of general formula (**II**) 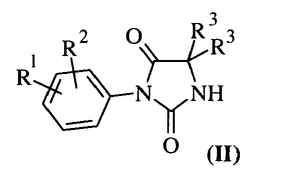 in which R¹, R², and R³ are as defined in one of claims 1 to 14, in the presence of a strong base at a temperature comprised between 25 and 60°C, in an anhydrous polar solvent, on the mesylate derivatives of general formula (**III**), 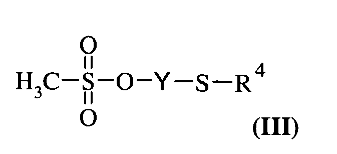 in which R⁴ and Y are as defined in one of claims 1 to 12,
- or by the treatment of thiobenzoyl derivatives of general formula (**V**), 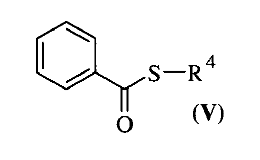 in which R⁴ is as defined in one of claims 1 to 14, with an alcoholate in a polar protic solvent followed by
- addition of the halogenated derivative of general formula (**IV**), 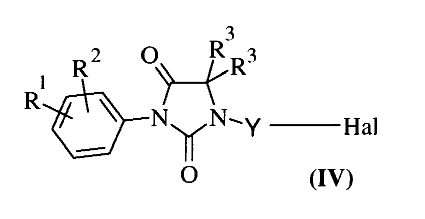
in which R¹, R², R³ and Y are as defined in one of claims 1 to 14, in solution in a polar solvent.

16. Process for the preparation of a compound of formula (**I**) according to claim 16, comprising moreover a stage of:
oxidation of the compounds of general formula (**I.1**) as defined in claim 15 to the sulphoxide of general formula (**I.2**) (compound of general formula (**I**) in which X represents the -SO- radical), 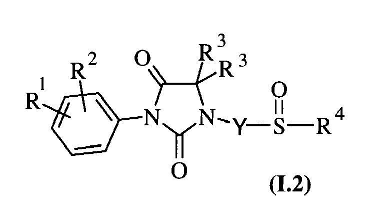
in which R¹, R², R³, R⁴ and Y are as defined in one of claims 1 to 14,

17. Process for the preparation of a compound of formula (I) according to claim 16, comprising moreover a stage of:
oxidation of the sulphoxide derivatives of general formula (**I.2**) to sulphones of general formula (**I.3**) (compound of general formula (**I**) in which X represents the -SO₂-group), 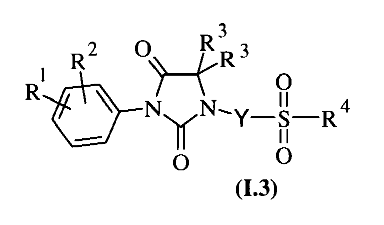
in which R', R², R³, R⁴ and Y are as defined in one of claims 1 to 14.

18. As an intermediate, one of the following compounds:
- 1-(5-iodopentyl)-5,5-dimethyl-3-[4-nitro-3-(trifluoromethyl)phenyl]imidazolidine-2,4-dione
- 1-(8-iodooctyl)-5,5-dimethyl-3-[4-nitro-3-(trifluoromethyl)phenyl]imidazolidine-2,4-dione
- 1-(9-bromononyl)-5,5-dimethyl-3-[4-nitro-3-(trifluoromethyl)phenyl]imidazolidine-2,4-dione
- 4-[3-(9-bromononyl)-4,4-dimethyl-2,5-dioxoimidazolidin-1-yl]-2-(trifluoromethyl)benzonitrile
- 5,5-dimethyl-3-(3-methyl-4-nitrophenyl)imidazolidine-2,4-dione
- 1-(9-bromononyl)-5,5-dimethyl-3-(3-methyl-4-nitrophenyl)imidazolidine-2,4-dione.

19. As medicament, a compound according to one of claims 1 to 14.

20. Pharmaceutical compositions containing, as active ingredient, at least one compound of formula (**I**) as defined in one of claims 1 to 14, in combination with a pharmaceutically acceptable support.

21. Use of a compound of formula (**I**) according to one of claims 1 to 14, for the preparation of a medicament intended to treat cancers.

22. Use according to claim 21 **characterized in that** the medicament is intended to treat a hormone-dependent cancer.

23. Use according to claim 21 **characterized in that** the medicament is intended to treat a cancer expressing the androgen receptors.

24. Use according to one of claims 22 or 23 **characterized in that** the medicament is intended to treat a breast or prostate cancer.

## Patentansprüche

1. Verbindung der allgemeinen Formel (**I**) 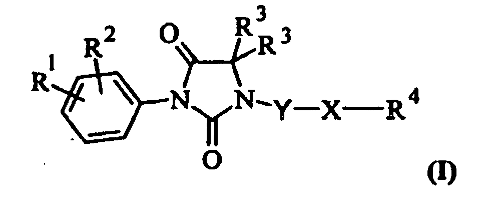 in racemischer Form, in enantiomerer Form oder in allen Kombinationen dieser Formen, in der
R¹ und R² unabhängig ein Halogenatom oder auch einen Rest Alkyl, Halogenalkyl, Alkoxy, Cyano, Nitro, Amino, Alkylamino, Dialkylamino, -NR⁸-CO-R⁵, -NR⁸-SO₂-R⁵, -NR⁸-CO-(CH₂)ₙ-NR⁶R⁷, -NR⁸-SO₂-(CH₂)ₙ-NR⁶R⁷ oder -CO-NH₂ darstellen;
n eine ganze Zahl, ausgewählt aus 0, 1, 2, 3, 4, 5 und 6, darstellt;
R⁵ einen Alkyl-, Aryl- oder Heteroaryl-Rest darstellt;
R⁶ und R⁷ unabhängig ein Wasserstoffatom, einen Alkyl- oder Alkyloxycarbonyl-Rest darstellen;
R⁸ ein Wasserstoffatom oder einen Alkyl-Rest darstellt;
R³ einen Alkyl-Rest oder ein Wasserstoffatom darstellt, oder die zwei Reste R³ zusammen mit dem Kohlenstoffatom, an das sie gebunden sind, einen Cycloalkyl-Rest bilden, der 3 bis 6 Kettenglieder umfasst;
R⁴ einen Halogenalkyl-Rest mit 2 bis 10 Kohlenstoffatomen darstellt;
Y eine lineare oder verzweigte Alkylenkette mit 2 bis 14 Kohlenstoffatomen darstellt, wobei diese Kette gesättigt oder ungesättigt sein kann und ein oder mehrere zusätzliche Kettenglieder -O- enthalten kann;
X -S-, -SO-, -SO₂-, -S=N(R⁹)- oder -S(O)=N(R⁹)- darstellt;
R⁹ ein Wasserstoffatom oder einen Halogenalkylcarbonyl-Rest darstellt,
oder ein pharmazeutisch annehmbares Salz davon.

2. Verbindung nach Anspruch 1, **dadurch gekennzeichnet, dass** R¹ ein Halogenatom oder auch einen Rest Alkyl, Alkoxy, Cyano, Nitro, Amino, Alkylamino, Dialkylamino, -NR⁸-CO-R⁵, -NR⁸-SO₂-R⁵, -NR⁸-CO-(CH₂)ₙ-NR⁶R⁷, -NR⁸-SO₂-(CH₂)ₙ-NR⁶R⁷ oder -CO-NH₂ darstellt; R² ein Halogenatom, einen Alkyl- oder Halogenalkyl-Rest darstellt, oder ein pharmazeutisch annehmbares Salz davon.

3. Verbindung nach einem der Ansprüche 1 oder 2, **dadurch gekennzeichnet, dass** R⁵ einen Alkyl-Rest darstellt.

4. Verbindung nach einem der Ansprüche 1 bis 3, **dadurch gekennzeichnet, dass** R³ einen Alkyl-Rest darstellt.

5. Verbindung nach einem der Ansprüche 1 bis 4, **dadurch gekennzeichnet, dass** R⁴ einen Halogenalkyl-Rest darstellt, der 4 bis 6 Kohlenstoffatome und 3 bis 9 Fluoratome umfasst, und Y eine Alkylenkette mit 5 bis 10 Kohlenstoffatomen darstellt.

6. Verbindung nach einem der Ansprüche 1 bis 5, **dadurch gekennzeichnet, dass** R¹ in para-Position ist.

7. Verbindung nach einem der Ansprüche 1 bis 6, **dadurch gekennzeichnet, dass** R² in meta-Position ist.

8. Verbindung nach einem der Ansprüche 1 bis 7, **dadurch gekennzeichnet, dass**:
R¹ einen Rest Cyano, Nitro, Amino, -NR⁸-CO-R⁵, -NR⁸-SO₂-R⁵, -NR⁸-CO-(CH₂)ₙ-NR⁶R⁷, -NR⁸-SO₂-(CH₂)ₙ-NR⁶R⁷ oder -CO-NH₂ darstellt;
R² einen Alkyl- oder Halogenalkyl-Rest darstellt;
R⁵ einen Alkyl-Rest darstellt;
R⁶ und R⁷ unabhängig ein Wasserstoffatom, einen Alkyl- oder Alklyoxycarbonyl-Rest darstellen;
R³ einen Alkyl-Rest darstellt oder auch die zwei Reste R³ zusammen mit dem Kohlenstoffatom, an das sie gebunden sind, einen Cycloalkyl-Rest bilden, der 3 bis 6 Kettenglieder umfasst;
R⁴ einen Halogenalkyl-Rest darstellt, der 4 bis 6 Kohlenstoffatome und 3 bis 9 Fluoratome umfasst;
n 0 oder 1 ist;
R⁹ ein Wasserstoffatom oder -COCF₃ darstellt.

9. Verbindung nach einem der Ansprüche 1 bis 8, **dadurch gekennzeichnet, dass** R¹ einen Rest Cyano, Nitro, -NR⁸-CO-R⁵, -NR⁸-SO₂-R⁵, -NR⁸-CO-(CH₂)ₙ-NR⁶R⁷, -NR⁸-SO₂-(CH₂)ₙ-NR⁶R⁷ oder -CO-NH₂ darstellt; n 0 oder 1 ist; R⁵ einen Alkyl-Rest darstellt, R⁶ und R⁷ unabhängig ein Wasserstoffatom oder einen Alkyl-Rest darstellen, und R² einen Alkyl- oder Halogenalkyl-Rest darstellt.

10. Verbindung nach einem der vorangehenden Ansprüche, **dadurch gekennzeichnet, dass** R¹ einen Rest Nitro oder -NR⁸-CO-R⁵, worin R⁵ einen Alkyl-Rest darstellt, darstellt.

11. Verbindung nach einem der Ansprüche 1 bis 10, **dadurch gekennzeichnet, dass** der Alkyl-Rest eine Methyl-Gruppierung darstellt und/oder der Halogenalkyl-Rest eine Trifluormethyl-Gruppierung oder einen Rest der Summenformel C₅H₆F₅, C₅H₄F₇, C₆H₈F₅, C₆H₆F₇ oder C₆H₄F₉ darstellt.

12. Verbindung nach einem der vorangehenden Ansprüche, **dadurch gekennzeichnet, dass** Y eine Alkylenkette mit 9 bis 10 Kohlenstoffatomen darstellt.

13. Verbindung der allgemeinen Formel (I), **dadurch gekennzeichnet, dass** es sich um eine Verbindung handelt, die ausgewählt ist aus:
- 5,5-Dimethyl-3-[4-nitro-3-(trifluormethyl)phenyl]-1-{9-[(4,4,5,5,5-pentafluorpentyl)sulfinyl]nonyl}imidazolidin-2,4-dion
- 5,5-Dimethyl-3-[4-nitro-3-(trifluormethyl)phenyl]-1-{10-[(4,4,5,5,5-pentafluorpentyl)sulfinyl]decyl}imidazolidin-2,4-dion
- 5,5-Dimethyl-3-[4-nitro-3-(trifluormethyl)phenyl]-1-{9-[(4,4,4-trifluorbutyl)-thio]nonyl}imidazolidin-2,4-dion
- 5,5-Dimethyl-3-[4-nitro-3-(trifluormethyl)phenyl]-1-{9-[(4,4,4-trifluorbutyl)-sulfinyl]-nonyl}imidazolidin-2,4-dion
- N-[4-(4,4-Dimethyl-2,5-dioxo-3-{9-[(4,4,5,5,5-pentafluorpentyl)sulfinyl]-nonyl}-imidazolidin-1-yl)-2-(trifluormethyl)phenyl]acetamid
- N-[4-(4,4-Dimethyl-2,5-dioxo-3-{9-[(4,4,5,5,5-pentafluorpentyl)sulfinyl]-nonyl}-imidazolidin-1-yl)-2-(trifluormethyl)phenyl]-N2,N2-dimethylglycinamid
- N-[4-(4,4-Dimethyl-2,5-dioxo-3-{9-[(4,4,5,5,5-pentafluorpentyl)sulfinyl]-nonyl}-imidazolidin-1-yl)-2-(trifluormethyl)phenyl]-N2,N2-dimethylglycinamid-Hydrochlorid
oder ein pharmazeutisch annehmbares Salz davon.

14. Verbindung der allgemeinen Formel (**I**), **dadurch gekennzeichnet, dass** es sich um N-[4-(4,4-Dimethyl-2,5-dioxo-3-{9-[(4,4,5,5,5-pentafluorpentyl)sulfinyl]-nonyl}imidazolidin-1-yl)-2-(trifluormethyl)phenyl]acetamid oder ein pharmazeutisch annehmbares Salz davon handelt.

15. Verfahren zur Herstellung einer Verbindung der Formel (**I**), wie sie in einem der Ansprüche 1 bis 14 definiert ist, **dadurch gekennzeichnet, dass** es den Erhalt von Verbindungen der allgemeinen Formel (**I.1**) (Verbindung der allgemeinen Formel (**I**), in der X ein Schwefelatom darstellt) 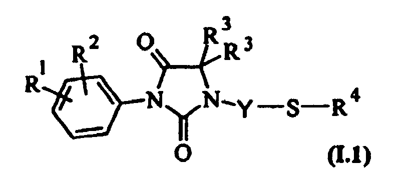 in der R¹, R², R³, R⁴ und Y wie in einem der Ansprüche 1 bis 14 definiert sind,
- entweder durch Kondensation von Hydantoin-Derivaten der allgemeinen Formel (**II**) 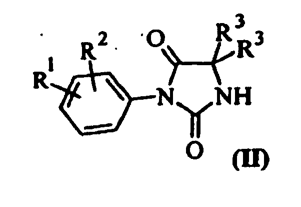 in der R¹, R² und R³ wie in einem der Ansprüche 1 bis 14 definiert sind, in Gegenwart einer starken Base bei einer Temperatur zwischen 25 und 60 °C in einem wasserfreien polaren Lösungsmittel mit Mesylat-Derivaten der allgemeinen Formel (**III**) 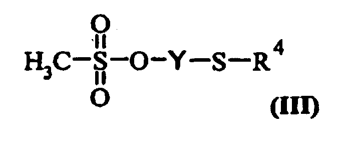 in der R⁴ und Y wie in einem der Ansprüche1 bis 12 definiert sind,
- oder durch Behandlung von Thiobenzoyl-Derivaten der allgemeinen Formel (**V**) 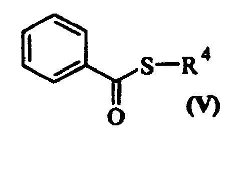 in der R⁴ wie in einem der Ansprüche 1 bis 14 definiert ist, mit einem Alkoholat in einem polaren protischen Lösungsmittel, gefolgt von
- Addition des halogenierten Derivats der allgemeinen Formel (**IV**) 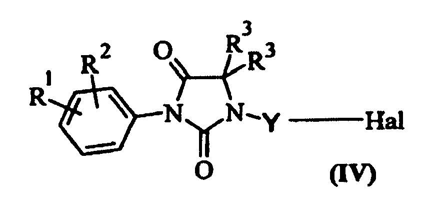
in der R¹, R², R³ und Y wie in einem der Ansprüche 1 bis 14 definiert sind, in Lösung in einem polaren Lösungsmittel
umfasst.

16. Verfahren zur Herstellung einer Verbindung der Formel (**I**) nach Anspruch 16, das außerdem eine Stufe der:
Oxidation der Verbindungen der allgemeinen Formel (**I.1**), wie sie in Anspruch 15 definiert ist, zu einem Sulfoxid der allgemeinen Formel (**I.2**) (Verbindung der allgemeinen Formel (**I**), in der X den Rest -SO- darstellt), 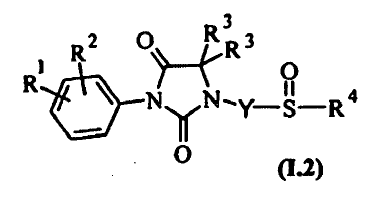
in der R¹, R², R³, R⁴ und Y wie in einem der Ansprüche 1 bis 14 definiert sind, umfasst.

17. Verfahren zur Herstellung einer Verbindung der Formel (**I**) gemäß Anspruch 16, das außerdem eine Stufe der:
Oxidation der Sulfoxid-Derivate der allgemeinen Formel (**I.2**) zu Sulfonen der allgemeinen Formel (**I.3**) (Verbindung der allgemeinen Formel (**I**), in der X die Gruppe -SO₂- darstellt) 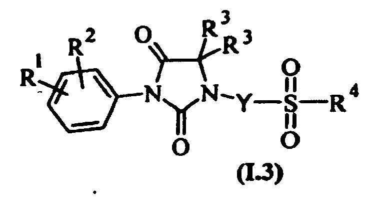
in der R¹, R², R³, R⁴ und Y wie in einem der Ansprüche 1 bis 14 definiert sind, umfasst.

18. Eine der folgenden Verbindungen:
- 1-(5-lodpentyl)-5,5-dimethyl-3-[4-nitro-3-(trifluormethyl)phenyl]imidazolidin-2,4-dion
- 1-(8-lodoctyl)-5,5-dimethyl-3-[4-nitro-3-(trifluormethyl)phenyl]imidazolidin-2,4-dion
- 1-(9-Bromnonyl)-5,5-dimethyl-3-[4-nitro-3-(trifluormethyl)phenyl]imidazolidin-2,4-dion
- 4-[3-(9-Bromnonyl)-4,4-dimethyl-2,5-dioxoimidazolidin-1-yl]-2-(trifluormethyl)benzonitril
- 5,5-Dimethyl-3-(3-methyl-4-nitrophenyl)imidazolidin-2,4-dion
- 1-(9-Bromnonyl)-5,5-dimethyl-3-(3-methyl-4-nitrophenyl)imidazolidin-2,4-dion
als Zwischenprodukt.

19. Verbindung gemäß einem der Ansprüche 1 bis 14 als Medikament.

20. Pharmazeutische Zusammensetzungen, die als Wirkstoff wenigstens eine Verbindung der Formel (**I**), wie sie in einem der Ansprüche1 bis 14 definiert ist, in Verbindung mit einem pharmazeutisch annehmbaren Träger enthalten.

21. Verwendung einer Verbindung der Formel (**I**) gemäß einem der Ansprüche 1 bis 14 zur Herstellung eines Medikaments, das dazu bestimmt ist, Krebs zu behandeln.

22. Verwendung gemäß Anspruch 21, **dadurch gekennzeichnet, dass** das Medikament dazu bestimmt ist, einen hormonabhängigen Krebs zu behandeln.

23. Verwendung gemäß Anspruch 21, **dadurch gekennzeichnet, dass** das Medikament dazu bestimmt ist, einen Krebs zu behandeln, der Rezeptoren für Androgene exprimiert.

24. Verwendung gemäß einem der Ansprüche 22 oder 23, **dadurch gekennzeichnet, dass** das Medikament dazu bestimmt ist, Brustkrebs oder Prostatakrebs zu behandeln.
